# EUROPEAN PATENT SPECIFICATION

(11) **EP 3 484 483 B1**
(45) Date of publication and mention of the grant of the patent: **08.03.2023**
(21) Application number: 17746236.3
(22) Date of filing: 13.07.2017
(51) Int. Cl.: A61K 31/715, A61K 9/00, A61P 43/00

(54) **GLYCAN COMPOSITIONS AND METHODS OF USE**
GLYCANZUSAMMENSETZUNGEN UND VERFAHREN ZUR VERWENDUNG
COMPOSITIONS DE GLYCANE ET LEURS PROCÉDÉS D'UTILISATION

(30) Priority: 13.07.2016 US 201662361998 P
(43) Date of publication of application: 22.05.2019
(73) Proprietor: Kaleido Biosciences, Inc., Lexington, MA 02421 (US)
(72) Inventor: VON MALTZAHN, Geoffrey, A., Somerville MA 02145 (US); RUBENS, Jacob, Rosenblum, Cambridge MA 02139 (US)
(74) Representative: Maiwald GmbH
(86) International application number: PCT/US2017/042022
(87) International publication number: WO 2018/013871

(56) References cited:
- WO-A2-2007/010084
- WO-A2-2013/032744
- US-A- 5 308 838

## Description

### BACKROUND OF THE INVENTION

Maintaining or restoring human health faces a large number of challenges many of which result from the lack of effective treatment options. WO 2007/010084 relates to a method of inhibiting a Th2-type immune response comprising administration of a mannan polysaccharide. US 5308838 relates to a method for enhancing the therapeutic effect in an animal of a known pharmaceutic agent by administration of an acetylated mannan derivative. WO 2013/032744 relates to formulations comprising carbohydrate, glycan, and polyphenol compounds for augmenting the efficacy of metformin.There is a continued need for novel therapies and treatment regimens.

### SUMMARY OF THE INVENTION

The subject matter of the invention is as defined by the appended claims.

In one aspect, the invention provides a glycan composition for use in increasing drug activity in a human subject comprising:
a) administering a glycan composition in an amount effective and for a time sufficient to increase the drug activity in the subject;
b) administering a glycan composition in an amount effective and for a time sufficient to increase drug activity in the subject, and wherein at the time of administration of the glycan composition, the subject comprises a level of the drug that, in the presence of the administered glycan composition, provides a therapeutic effect;
c) administering the drug, wherein at the time of administration of the drug, the subject has already been administered the glycan composition in an amount effective and for a time sufficient to increase the drug activity in the subject;
d) administering the drug in an amount effective and for a time sufficient to increase the drug activity in the subject, wherein subject has been determined to be in need of the glycan composition; or
e) administering the drug and the glycan composition to the subject, in amounts effective and for times sufficient to increase the drug activity in the subject, wherein administration of the drug and the glycan composition overlap;

wherein the glycan composition comprises any combination of two, three, four, five, six, seven, eight, or nine of:
   i) the glycan composition comprises glycan polymers that comprise glucose, galactose, arabinose, mannose, fructose, xylose, fucose, or rhamnose glycan units;
   ii) the average degree of branching (DB) of the glycan polymers in the glycan preparation is between 0.01 and 0.6;
   iii) at least 50% of the glycan polymers in the glycan composition have a degree of polymerization (DP) of at least 3 and less than 30 glycan units;
   iv) the average DP or mean DP of the glycan composition is between 5-15;
   v) the ratio of alpha- to beta-glycosidic bonds present in the glycan polymers of the glycan preparation between 0.8:1 to 5:1;
   vi) the glycan composition comprises between 15 mol % and 75 mol % 1,6 glycosidic bonds;
   vii) the glycan composition comprises between 1 mol % and 40 mol % of each at least one of 1,2; 1,3; and 1,4 glycosidic bonds;
   viii) the glycan composition has a final solubility limit in water of at least 50 Brix at 23 °C: or
   ix) the glycan composition has a dietary fiber content of at least 50%;
wherein the drug comprises 4-aminosalicylic acid, 5-aminosalicylic acid, balsalazide, olsalazine, or sulfasalazine.

The invention is defined by the claims. Any subject-matter falling outside the scope of the claims is provided for information purposes only and is mentioned "to be disclosed" or "described" or "as provided".

The processing of exogenous substances, such as, e.g., a drug, a drug metabolite, a drug additive, a food, a food additive, an allergen, a toxin or toxicant can be carried out in a subject by mammalian machinery and/or microbial machinery. In some instances, the processing of an exogenous substance can be mediated by the microbial constituents of a subject, such as, e.g., microbes in the gut of the subject. By modulating the processing, e.g., the microbe-mediated processing, of the exogenous substance the effect of the exogenous substance, or its processed forms, on a subject can be altered.

Methods, compositions, kits and the like, described herein are based at least in part on the discovery that a glycan composition can alter the way microbes (e.g., gut microbes) mediate the processing of an exogenous substrate in a subject, e.g., a human subject. In an embodiment, the glycan composition modulates the microbe-mediated processing by increasing or decreasing the number or prevalence of a microbe, e.g., bacterial taxa. In an embodiment, the glycan composition modulates the microbe-mediated processing by increasing or decreasing the activity or level of a constituent or product of the microbe, e.g., an enzyme or a metabolite made by a microbe. In an embodiment, the glycan composition increases or decreases the transcription of an enzyme or other microbial proteins (e.g., one or a plurality of protein constituents of a pathway, such as, e.g., a metabolic pathway) that result in altered activity of the microbe.

Further disclosed is a method for increasing drug activity in a subject comprising:
a) administering a glycan composition in an amount effective and for a time sufficient to increase the drug activity in the subject;
b) administering a glycan composition in an amount effective and for a time sufficient to increase drug activity in the subject, and wherein at the time of administration of the glycan composition, the subject comprises a level of the drug that, in the presence of the administered glycan composition, provides a therapeutic effect;
c) administering the drug, wherein at the time of administration of the drug, the subject has already been administered the glycan composition in an amount effective and for a time sufficient to increase the drug activity in the subject;
d) administering the drug in an amount effective and for a time sufficient to increase the drug activity in the subject, wherein subject has been determined to be in need of the glycan composition, e.g., to increase the activity of the drug; or
e) administering the drug and the glycan composition to the subject, in amounts effective and for times sufficient to increase the drug activity in the subject, wherein administration of the drug and the glycan composition overlap;
wherein
i) the glycan preparation comprises glycan polymers that comprise glucose, galactose, arabinose, mannose, fructose, xylose, fucose, or rhamnose glycan units;
ii) the average degree of branching (DB) of the glycan polymers in the glycan preparation is 0, between 0.01 and 0.6, between 0.05 and 0.5, between 0.1 and 0.4, or between 0.15 and 0.4;
iii) at least 50% (at least 60%, 65%, 70%, 75%, 80%, or 85%, or less than 50%) of the glycan polymers in the glycan preparation have a degree of polymerization (DP) of at least 3 and less than 30 glycan units, at least 3 and less than 10 glycan units, at least 5 and less than 25 glycan units, or at least 10 and less than 35 glycan units;
iv) the average DP (mean DP) of the glycan preparation is between about 5 and 8, between about 8 and 13, between about 13 and 25, between about 5 and 15, between about 5 and 20, or between about 5-15;
v) the ratio of alpha- to beta-glycosidic bonds present in the glycan polymers of the glycan preparation is 0, or between about 0.8:1 to about 5:1, between about 1:1 to about 5:1, between about 1:1 to about 3:1, between about 3:2 to about 2:1, or between about 3:2 to about 3:1,
vi) the glycan preparation comprises between 15 mol % and 75 mol % (between 20 mol % and 60 mol %, between 25 mol % and 50 mol %, or between 30 mol % and 45 mol %) 1,6 glycosidic bonds;
vii) the glycan preparation comprises between 1 mol % and 40 mol % (between 1 mol % and 30 mol %, between 5 mol % and 25 mol %, between 10 mol % and 20 mol %) of each at least one, two, or three of 1,2; 1,3; and 1,4 glycosidic bonds;
viii) the glycan preparation has a final solubility limit in water of at least about 50 (at least about 60, 70, at least about 75, or less than 50) Brix at 23 °C; or
ix) the glycan preparation has a dietary fiber content of at least 50% (at least 60%, 70%, 80%, or at least 90%, or less than 50%),
x) any combination of two, three, four, five, six, seven, eight, or nine of i), ii), iii), iv), v), vi), vii), viii), and ix), and
   wherein the drug comprises a:
   i) cardiac glycoside;
   ii) sulfonamide;
   iii) nucleoside analogue; or
   iv) amino salicylate; or
      wherein the drug is:
      - a nonsteroidal anti-inflammatory (NSAID) drug;
      - a chemotherapeutic drug, or generally a drug that is anti proliferative effect on target cells, e.g., cancer cells;
      - an antibiotic or antibacterial;
      - an antifungal;
      - an anti-parasitic agent, e.g., an anti-nematodal;
      - a hormone;
      - a sedative;
      - a heart medication;
      - a high blood pressure medication;
      - a colony-stimulating factor;
      - a dopamine;
      - an opioid receptor agonist;
      - a statin;
      - a CNS stimulant;
      - a sensitizer/radio-therapy agent;
      - a narcotic pain reliever;
      - a hypnotic drug;
      - an antiacid;
      - an analgesic;
      - an uricase inhibitors,
      - an antipsychotic;
      - a laxative; or
      - a neurotropic agent, e.g., an anticonvulsant.

Further disclosed is a method for increasing an activity of an ingested substance, e.g., a substance in a food, food supplement, or medical food, e.g., phytoestrogen or polyphenol activity, in a subject, e.g., a human subject, comprising:
a) administering a glycan composition in an amount effective and for a time sufficient to increase an activity of the ingested substance, e.g., phytoestrogen or polyphenol, in the subject;
b) administering a glycan composition in an amount effective and for a time sufficient to increase an activity of the ingested substance, e.g., phytoestrogen or polyphenol, in the subject, and wherein at the time of administration of the glycan composition, the subject comprises a level of the ingested substance, e.g., phytoestrogen or polyphenol, that, in the presence of the administered glycan composition, will provide an increase, e.g., a beneficial increase in the ingested substance, e.g., phytoestrogen or polyphenol activity;
c) administering the ingested substance, e.g., phytoestrogen or polyphenol, wherein at the time of administration of the ingested substance, e.g., phytoestrogen or polyphenol, the subject has already been administered the glycan composition in an amount effective and for a time sufficient to increase the activity of the ingested substance, e.g., phytoestrogen or polyphenol, in the subject;
d) administering the ingested substance, e.g., phytoestrogen or polyphenol, wherein subject that has been determined to be in need of the glycan composition; or
e) administering the ingested substance, e.g., phytoestrogen or polyphenol, and the glycan composition to the subject, in amounts effective and for times sufficient to increase the drug activity in the subject, wherein administration of the drug and the glycan composition overlap; wherein:
   i) the glycan preparation comprises glycan polymers that comprise glucose, galactose, arabinose, mannose, fructose, xylose, fucose, or rhamnose glycan units;
   ii) the average degree of branching (DB) of the glycan polymers in the glycan preparation is 0, between 0.01 and 0.6, between 0.05 and 0.5, between 0.1 and 0.4, or between 0.15 and 0.4;
   iii) at least 50% (at least 60%, 65%, 70%, 75%, 80%, or 85%, or less than 50%) of the glycan polymers in the glycan preparation have a degree of polymerization (DP) of at least 3 and less than 30 glycan units, at least 3 and less than 10 glycan units, at least 5 and less than 25 glycan units, or at least 10 and less than 35 glycan units;
   iv) the average DP (mean DP) of the glycan preparation is between about 5 and 8, between about 8 and 13, between about 13 and 25, between about 5 and 15, between about 5 and 20, or between about 5-15;
   v) the ratio of alpha- to beta-glycosidic bonds present in the glycan polymers of the glycan preparation is 0, or between about 0.8:1 to about 5:1, between about 1:1 to about 5:1, between about 1:1 to about 3:1, between about 3:2 to about 2:1, or between about 3:2 to about 3:1,
   vi) the glycan preparation comprises between 15 mol % and 75 mol % (between 20 mol % and 60 mol %, between 25 mol % and 50 mol %, or between 30 mol % and 45 mol %) 1,6 glycosidic bonds;
   vii) the glycan preparation comprises between 1 mol % and 40 mol % (between 1 mol % and 30 mol %, between 5 mol % and 25 mol %, between 10 mol % and 20 mol %) of each at least one, two, or three of 1,2; 1,3; and 1,4 glycosidic bonds;
   viii) the glycan preparation has a final solubility limit in water of at least about 50 (at least about 60, 70, at least about 75, or less than 50) Brix at 23 °C; or
   ix) the glycan preparation has a dietary fiber content of at least 50% (at least 60%, 70%, 80%, or at least 90%, or less than 50%),
   x) any combination of two, three, four, five, six, seven, eight, or nine of i), ii), iii), iv), v), vi), vii), viii), and ix).

Further disclosed is a method for decreasing a toxic activity of an ingested substance, e.g., a substance in a food, food supplement, or medical food, e.g., a heterocyclic amine (HCA) or a polycyclic aromatic hydrocarbon (PAH), in a subject, e.g., a human subject, comprising:
a) administering a glycan composition in an amount effective and for a time sufficient to decrease a toxic activity of the ingested substance, e.g., a HCA or PAH, in the subject;
b) administering a glycan composition in an amount effective and for a time sufficient to decrease a toxic activity of the ingested substance, e.g., a HCA or PAH, in the subject, and wherein at the time of administration of the glycan composition, the subject comprises a level of the ingested substance, e.g., a HCA or PAH, that, in the presence of the administered glycan composition, will provide a decrease, e.g., a beneficial decrease, in a toxic activity of the ingested substance, e.g., a HCA or PAH;
c) administering the ingested substance, e.g., a HCA or PAH, wherein at the time of administration of the ingested substance, e.g., A HCA or PAH, the subject has already been administered the glycan composition in an amount effective and for a time sufficient to decrease a toxic activity of the ingested substance, e.g., a HCA or PAH, in the subject;
d) administering the ingested substance, e.g., a HCA or PAH, wherein subject that has been determined to be in need of the glycan composition; or
e) administering the ingested substance, e.g., a HCA or PAH, and the glycan composition to the subject, in amounts effective and for times sufficient to decrease a toxic activity of the ingested substance, e.g., a HCA or PAH, in the subject, wherein administration of the drug and the ingested substance, e.g., a HCA or PAH composition overlap;
wherein:
i) the glycan preparation comprises glycan polymers that comprise glucose, galactose, arabinose, mannose, fructose, xylose, fucose, or rhamnose glycan units;
ii) the average degree of branching (DB) of the glycan polymers in the glycan preparation is 0, between 0.01 and 0.6, between 0.05 and 0.5, between 0.1 and 0.4, or between 0.15 and 0.4;
iii) at least 50% (at least 60%, 65%, 70%, 75%, 80%, or 85%, or less than 50%) of the glycan polymers in the glycan preparation have a degree of polymerization (DP) of at least 3 and less than 30 glycan units, at least 3 and less than 10 glycan units, at least 5 and less than 25 glycan units, or at least 10 and less than 35 glycan units;
iv) the average DP (mean DP) of the glycan preparation is between about 5 and 8, between about 8 and 13, between about 13 and 25, between about 5 and 15, between about 5 and 20, or between about 5-15;
v) the ratio of alpha- to beta-glycosidic bonds present in the glycan polymers of the glycan preparation is 0, or between about 0.8:1 to about 5:1, between about 1:1 to about 5:1, between about 1:1 to about 3:1, between about 3:2 to about 2:1, or between about 3:2 to about 3:1,
vi) the glycan preparation comprises between 15 mol % and 75 mol % (between 20 mol % and 60 mol %, between 25 mol % and 50 mol %, or between 30 mol % and 45 mol %) 1,6 glycosidic bonds;
vii) the glycan preparation comprises between 1 mol % and 40 mol % (between 1 mol % and 30 mol %, between 5 mol % and 25 mol %, between 10 mol % and 20 mol %) of each at least one, two, or three of 1,2; 1,3; and 1,4 glycosidic bonds;
viii) the glycan preparation has a final solubility limit in water of at least about 50 (at least about 60, 70, at least about 75, or less than 50) Brix at 23 °C; or
ix) the glycan preparation has a dietary fiber content of at least 50% (at least 60%, 70%, 80%, or at least 90%, or less than 50%),
x) any combination of two, three, four, five, six, seven, eight, or nine of i), ii), iii), iv), v), vi), vii), viii), and ix).

In another aspect, the invention features a method of:
(i) modulating the processing of an exogenous substance (modulating processing) in, or
(ii) modulating an enzyme activity in the gastrointestinal tract (modulating activity) of, a subject comprising:
   a) administering a glycan composition in an amount effective and for a time sufficient to modulate processing or modulate activity in the subject;
   b) administering a glycan composition in an amount effective and for a time sufficient to to modulate processing or modulate activity in the subject, and wherein at the time of administration of the glycan composition, the subject comprises the exogenous substance or enzyme;
   c) administering the exogenous substance, wherein at the time of administration of the exogenous substance, the subject has already been administered the glycan composition in an amount effective and for a time sufficient to modulate the processing of the exogenous substance in the subject;
   d) administering the exogenous substance, wherein subject that has been determined to be in need of the glycan composition; or
   e) administering the exogenous substance and the glycan composition to the subject, in amounts effective and for times sufficient to increase the modulate processing or modulate activity in the subject, wherein administration of the exogenous substance and the glycan composition overlap;
wherein:
i) the glycan preparation comprises glycan polymers that comprise glucose, galactose, arabinose, mannose, fructose, xylose, fucose, or rhamnose glycan units;
ii) the average degree of branching (DB) of the glycan polymers in the glycan preparation is 0, between 0.01 and 0.6, between 0.05 and 0.5, between 0.1 and 0.4, or between 0.15 and 0.4;
iii) at least 50% (at least 60%, 65%, 70%, 75%, 80%, or 85%, or less than 50%) of the glycan polymers in the glycan preparation have a degree of polymerization (DP) of at least 3 and less than 30 glycan units, at least 3 and less than 10 glycan units, at least 5 and less than 25 glycan units, or at least 10 and less than 35 glycan units;
iv) the average DP (mean DP) of the glycan preparation is between about 5 and 8, between about 8 and 13, between about 13 and 25, between about 5 and 15, between about 5 and 20, or between about 5-15;
v) the ratio of alpha- to beta-glycosidic bonds present in the glycan polymers of the glycan preparation is 0, or between about 0.8:1 to about 5:1, between about 1:1 to about 5:1, between about 1:1 to about 3:1, between about 3:2 to about 2:1, or between about 3:2 to about 3:1,
vi) the glycan preparation comprises between 15 mol % and 75 mol % (between 20 mol % and 60 mol %, between 25 mol % and 50 mol %, or between 30 mol % and 45 mol %) 1,6 glycosidic bonds;
vii) the glycan preparation comprises between 1 mol % and 40 mol % (between 1 mol % and 30 mol %, between 5 mol % and 25 mol %, between 10 mol % and 20 mol %) of each at least one, two, or three of 1,2; 1,3; and 1,4 glycosidic bonds;
viii) the glycan preparation has a final solubility limit in water of at least about 50 (at least about 60, 70, at least about 75, or less than 50) Brix at 23 °C; or
ix) the glycan preparation has a dietary fiber content of at least 50% (at least 60%, 70%, 80%, or at least 90%, or less than 50%),
x) any combination of two, three, four, five, six, seven, eight, or nine of i), ii), iii), iv), v), vi), vii), viii), and ix).

In another aspect, the invention features a glycan composition disclosed herein.

In an embodiment, a glycan composition, alters the way microbes (e.g., gut microbes) mediate host processing, e.g., mediate the production, level, structure, distribution, effect, or the activity, of a microbial entity, e.g., an enzyme, that acts on an exogenous substance.

In an embodiment, a glycan composition, alters the way microbes (e.g., gut microbes) mediate the processing by increasing or decreasing the number or prevalence of a microbe, e.g., in the gut of the subject. In an embodiment, the increase or decrease in number or prevalence of a microbe, e.g., a bacterial taxa is associated with an increase or decrease in the processing activity, e.g., of an enzyme, that acts on the exogenous substance. In an embodiment, a microbial entity, e.g., an enzyme, alters the processing of an exogenous compound, e.g., a drug, a drug metabolite, a drug additive, a food, a food additive, an allergen, a toxin or toxicant.

In an embodiment, a microbe mediates a change in the production, level, structure, distribution, or activity, of a constituent of the host, e.g., an enzyme (such as a mammalian enzyme), made by the subject. In an embodiment, the host entity, e.g., an enzyme, alters the processing of an exogenous compound, e.g., a drug, a drug metabolite, a drug additive, a food, a food additive, an allergen, a toxin or toxicant.

Methods described herein provide administering, in combination with an exogenous substance, a glycan composition, having a preselected property, e.g., the ability to alter i) the number or prevalence (relative abundance) of a microbe, e.g., a gut microbe, that alters the exogenous substance (e.g., by increasing or decreasing growth of the microbe), ii) the ability of the microbe, e.g., a gut microbe, to provide a processing activity (e.g., in form of a microbial enzyme) that alters the exogenous substance (e.g., by altering the transcription levels/expression level of the microbial enzyme in a microbe), or iii) the ability of the microbe, e.g., a gut microbe, to modulate a host subject response (e.g., increase or decrease a host processing activity (e.g., in form of a host enzyme) that alters the exogenous substance (e.g., by altering metabolite or signaling output by the microbe).

In some embodiments, an enzyme provided by a microbe modifies (e.g., modifies the production, level, structure, distribution, effect, and/or the activity) an exogenous compound, e.g., directly. In some embodiments, an enzyme provided by a microbe modifies (e.g., modifies the production, level, structure, distribution, effect, and/or the activity) an exogenous compound, e.g., indirectly, e.g., the enzyme generates a metabolite that modifies an exogenous compound or mediates host processing in such a way as to modify an exogenous compound. For example, an enzyme provided by a microbe that indirectly modifies an exogenous compound may generate a metabolite that competes with a host enzyme, altering the way the host enzyme interacts with an exogenous compound, thus modifying the exogenous compound. In another example, an enzyme provided by a microbe that indirectly modifies an exogenous compound may activate or inhibit a host enzyme which processes (e.g., metabolizes) the exogenous compound. In embodiments, the increase or decrease in the number or prevalence (e.g., relative abundance) of the microbe, or its ability to provide an enzyme activity, is associated with an increase or decrease in the activity of an enzyme that interacts with the exogenous substance. In an embodiment, the interaction of the enzyme with the exogenous substance, e.g., a drug, can optimize the effect of the drug, e.g., by increasing levels of an active (activated) form or its bioavailability, or decreasing the levels of an inactive (or inactivated) form or thelevels of toxic intermediate (e.g., produced through drug metabolization, e.g., by host or microbial enzymes), thus modulating the subsequent effects of the drug on the host (e.g., treatment effects). In an embodiment, the interaction of the enzyme with the exogenous substance, e.g., a toxin or toxicant, can reduce the harmful effects on the subject, e.g., by increasing the processing to nontoxic or less forms or intermediates that are more rapidly excreted from the subject's body (e.g., more soluble, less reactive, etc.).

In an embodiment, a glycan composition that promotes the growth of a Bacteroides sp., Enterococcus faecalis, and/or a Lactobacillus sp, is administered in combination with sulfasalazine to a subject, e.g., a subject with rheumatoid arthritis. Methods are provided (e.g., methods of treatment of rheumatoid arthritis) comprising the administration of a glycan composition described herein to a subject receiving (or about to receive) sulfasalazine in an amount effective to increase conversion of the prodrug sulfasalazine to 5-aminosalicylic acid, e.g., by increasing the level or activity of microbial azoreductase, thereby resulting in increased levels of 5-aminosalicylic acid. See, e.g., Table 1, row 3.

In an embodiment, a glycan composition that decreases growth of a gut microbes (e.g. aerobic enterobacteria or anaerobes such as Clostridium perfringens) or decreases the production of an enzyme that generates p-cresol, e.g., from tyrosine is administered in combination with acetaminophen/paracetamol. Methods are provided comprising the administration of a glycan composition described herein to a subject receiving (or about to receive) acetaminophen/paracetamol in an amount effective to decrease drug-induced toxicity by acetaminophen/paracetamol and or increase activity of acetaminophen/paracetamol, e.g., by decreasing the levels of p-cresol which competes with acetaminophen as a substrate of SILT1A1, thereby decreasing interference of p-cresol with host metabolism of acetaminophen/paracetamol. In an embodiment, a glycan composition that inhibits the growth of pathogenic Firmicutes (e.g., Clostridium difficile), Bacteroidetes, Actinobacteria, and/or Fusobacteria is administered in combination with tyrosine and/or phenylalanine, to a subject, e.g., a subject with pain, fever, or drug-induced toxicity (e.g., from acetaminophen). Methods are provided comprising the administration of a glycan composition described herein to a subject in an amount effective to decrease levels of p-cresol, e.g., by decreasing the level or activity of a microbial enzyme that metabolizes a substrate to p-cresol. See, e.g., Table 1, row 8.

In an embodiment, a glycan composition that promotes the growth of a gut microbe (e.g., Proteobacteria, Firmicutes, or Actinobacteria), is administered in combination with irinotecan to a subject, e.g., a subject with cancer, e.g., colorectal cancer. Methods are provided (e.g., methods of treatment of cancer) comprising the administration of a glycan composition described herein to a subject receiving (or about to receive) irinotecan in an amount effective to decrease the levels of toxic intermediates of irinotecan (such as, e.g., SN-38 glucoronide), e.g., by decreasing the level or activity of microbial beta-glucuronidase. In embodiments, methods are provided to treat side effects associated with irinotecan treatment, e.g. myelosuppresion, diarrhea, and neutropenia. See, e.g., Table 1, row 5.

In an embodiment, a glycan composition that promotes the growth of Eggerthella lenta, e.g., strain DSM2243, is administered in combination with digoxin to a subject, e.g., a subject having a cardiac disease or disorder, e.g., cardiac arrhythmia, or heart failure. Methods are provided (e.g., methods of treatment of cardiac disease or disorder) comprising the administration of a glycan composition described herein to a subject receiving (or about to receive) digoxin in an amount effective to increase drug activity, e.g., by decreasing the level or activity of microbial bacterial reductase. See, e.g., Table 1, row 7.

In an embodiment, a glycan composition that promotes the growth Enterococcus faecium, Lactobacillus mucosae, a Bifidobacterium sp., ora Eggerthella sp is administered in combination with phytoestrogen (e.g., isoflavone or lignan), e.g., a glycosidic isoflavone such as daidzin to a subject, e.g., a subject having or at risk for breast cancer. Methods are provided (e.g., methods of treatment of breast cancer) comprising the administration of a glycan composition described herein to a subject receiving (or about to receive) daidzin in an amount effective to increase levels of equol, e.g., by increasing the level or activity of microbial bacterial reductase that catalyzes the glycosidic cleavage and reduction of an α, β-unsaturated ketone. See, e.g., Table 1, row 14.

In an embodiment, a glycan composition that promotes the growth of Actinobacteria, Bacteroidetes, and/or Firmicutes is administered in combination with phytoestrogen (e.g., isoflavone or lignan), e.g., a glycosidic isoflavone such as daidzin to a subject, e.g., a subject having or at risk for breast cancer. Methods are provided (e.g., methods of treatment of breast cancer) comprising the administration of a glycan composition described herein to a subject receiving (or about to receive) a phytoestrogen in an amount effective to increase levels or enhance activity of a microbial enzyme which boosts metabolism of phytoestrogen to molecules that bind estrogen receptors. See, e.g., Table 1, row 13.

In an embodiment, a glycan composition that inhibits or reduces the growth of bacteria that carry the *uidA* gene, e.g., Escherichia coli, is administered in combination with a heterocyclic amine (e.g., 2-amino-3-methylimidazo[4,5-f]quinolone (IQ), 2-amino-1-methyl-6-phenylimidazo[4,5-b]pyridine (PhIP), 2-amino-3,8-dimethylimidazo[4,5-f]-quinoxaline (MeIQx)) (e.g., formed during the burning of meat), to a subject, e.g., a subject at risk for a cancer. Methods are provided comprising the administration of a glycan composition described herein to a subject receiving (or about to receive), e.g., eating or about to eat, heterocyclic amine (e.g., as a constituent of burned meat) in an amount effective to decrease levels of a toxic compound, e.g., a carcinogen, e.g., by decreasing the level or activity of microbial beta-glucuronidase. See, e.g., Table 1, row 16.

In an embodiment, a glycan composition that inhibits or reduces the growth of a microbe in the colon, e.g., Firmicutes, Proteobacteria, Actinobacteria (e.g., not including Bacteroides), and/or bacteria carrying the choline utilization (*cut*) gene cluster, is administered in combination with a choline containing compound, e.g., L-carnitine, to a subject, e.g., a subject having or at risk for high cholesterol or a cardiac condition. Methods are provided comprising the administration of a glycan composition described herein to a subject receiving (or about to receive) L-carnitine in an amount effective to decrease levels of a trimethylamine (TMA), e.g., by decreasing the level or activity of microbial glycyl radical enzyme. See, e.g., Table 1, row 17.

In an embodiment, a glycan composition that inhibits the growth of Firmicutes (e.g., Lactobacillus) is administered in combination with taurine-conjugated bile acid (e.g., tauro-betamuricholic acid), to a subject, e.g., a subject with obesity (e.g., diet-induced obesity). Methods are provided comprising the administration of a glycan composition described herein to a subject in an amount effective to decrease levels of free bile acids and/or increase the levels of taurine-conjugated bile acids (which, e.g., emulsify fats and oils), e.g., by decreasing the level or activity of a microbial bile salt hydrolase. See, e.g., Table 1, row 10.

In an embodiment, a glycan composition that inhibits the growth of Actinobacteria, e.g., Gordonibacter, is administered in combination with ellagitannin to a subject. Methods are provided comprising the administration of a glycan composition described herein to a subject in an amount effective to decrease levels of ellagitannin and/or increase the levels of ellagic acid, e.g., by increasing the level or activity of a microbial enzyme that hydrolyzes ellagitannin to ellagic acid. See, e.g., Table 1, rows 11 and 12.

In an embodiment, a glycan composition that promotes the growth of E. faecalis, E. lenta, Blautia product, Eubacterium limosum, Clostridium scindens, Lactonifactor longoviformis, Clostridium saccharogumia, and/or P. producta is administered in combination with lignan (from plants), e.g., pinoresinol, secoisolariciresinol, to a subject, e.g., a subject having or at risk for breast cancer. Methods are provided (e.g. methods of treating breast cancer) comprising the administration of a glycan composition described herein to a subject in an amount effective to increase levels of enterodiol and/or enterolactone, e.g., by increasing the level or activity of a microbial enzyme which metabolizes pinoresinol and/or secoisolariciresinol to enterodiol and/or enterolactone. See, e.g., Table 1, row 15.

In an embodiment, a glycan composition that inhibits the growth of Enterococcus, Clostridium, Corynebacterium, Campylobacter, and/or Escherichia is administered in combination with a non-caloric artificial sweetener, e.g., cyclamate, xylitol, or saccharin, to a subject. Methods are provided comprising the administration of a glycan composition described herein to a subject in an amount effective to decrease levels of toxic conversion products of sweeteners (e.g., conversion of cyclamate to cyclohexylamine, which can be toxic), e.g., by decreasing the level or activity of a microbial enzyme which metabolizes artificial sweeteners. See, e.g., Table 1, row 18.

In an embodiment, a glycan composition that inhibits or reduces the growth of a gut microbe, e.g., Klebsiella terrigena, is administered in combination with melamine, e.g., food or substance containing melamine, to a subject, e.g., a subject having or at risk for a renal condition (e.g., renal failure). Methods are provided comprising the administration of a glycan composition described herein to a subject in an amount effective to decrease toxic levels of cyanuric acid, e.g., by decreasing the level or activity of a microbial enzyme which metabolizes melamine.

In an embodiment, a glycan composition that increases the growth of a gut microbe, e.g., Bifidobacterium, Lactobacillus, Escherichia, is administered in combination with a conjugated hydroxycinnamate (e.g., found in foods, such as fruits, vegetables, cereals, and coffee), to a subject, e.g., a subject having or at risk for inflammation, e.g., an inflammatory disease. Methods are provided comprising the administration of a glycan composition described herein to a subject in an amount effective to increase levels of anti-inflammatory substances and/or anti-oxidants caffeic acid, ferulic acid, and p-coumaric acid, e.g., by increasing the level or activity of a microbial enzyme processing these substances.

In an embodiment, a glycan composition that inhibits or decreases the growth of a microbe, is administered in combination with a cycasin (e.g., found in some plants), to a subject, e.g., a subject having or at risk for cancer. Methods are provided comprising the administration of a glycan composition described herein to a subject in an amount effective to decrease toxic levels of methylazoxymethanol, a carcinogen, e.g., by decreasing the level or activity of a microbial enzyme processing this substance.

In an embodiment, a glycan composition that increases the growth of a microbe, is administered in combination with an anthocyanin, to a subject, e.g., a subject having or at risk for cancer. Methods are provided comprising the administration of a glycan composition described herein to a subject in an amount effective to increase levels and/or activity of aglycone, which has anticancer properties, e.g., by increasing the level or activity of a microbial enzyme processing this substance.

In an embodiment, a glycan composition that increases the growth of a microbe, e.g., Oxalobacter formigenes, is administered in combination with an oxalate, to a subject, e.g., a subject having or at risk for kidney stones, renal failure, hyperoxaluria, and/or cardiac conduction disorders. Methods are provided comprising the administration of a glycan composition described herein to a subject in an amount effective to decrease levels of oxalate, which is associated with renal toxicity, e.g., by increasing the level or activity of an oxalateprocessing microbial enzyme (e.g., oxalate:formate antiporter, formyl-CoA transferase, or oxalyl-CoA decarboxylase).

In an embodiment, a glycan composition that increases the growth of a microbe, e.g., a microbe that upregulates expression of host CYP450 enzyme(s), is administered in combination with a polycyclic aromatic hydrocarbon (PAH), e.g., benzo[a]pyrene, e.g., found in some plant and animal foods, e.g., meats cooked over open flame, to a subject, e.g., a subject having or at risk for cancer. Methods are provided comprising the administration of a glycan composition described herein to a subject in an amount effective to upregulate host CYP450 enzyme(s), thereby conferring protection against carcinogenic PAHs, e.g., by increasing the level or activity of a suitable microbial enzyme.

In an embodiment, a glycan composition that modulates the growth of a microbe, e.g., microbe that controls expression of host Phase I (CYPs) and Phase II drug metabolizing enzymes (e.g. UGTs, SULTs) which are implicated in the metabolism of drugs can mediate host drug response. Methods are provided comprising the administration of a glycan composition described herein to a subject in an amount effective to modulate levels or activity of a microbe thereby altering host drug metabolizing enzymes, thereby increasing the hosts ability to better respond to a drug.

In an embodiment, a glycan composition that decreases the growth of a microbe, e.g., a microbe which generates a metabolite of sorivudine, e.g., (E)-5-(2-bromovinyl)uracil (BVU), is administered in combination with sorivudine and 5- fluorouracil (5-FU), to a subject, e.g., a subject having or at risk for a viral infection, e,g., herpes zoster, e.g., a cancer patient having or at risk of having herpes zoster. Methods are provided comprising the administration of a glycan composition described herein to a subject in an amount effective to decrease toxic levels of 5-FU, e.g., by modulating (increasing or decreasing) the level or activity of a suitable microbial enzyme.

In an embodiment, a glycan composition that decreases the growth of an Enterobacteria, e.g., K. pneumoniae, is administered in combination with sorivudine, to a subject, e.g., a subject having or at risk for a viral infection, e.g., herpes zoster or varicella-zoster. Methods are provided comprising the administration of a glycan composition described herein to a subject in an amount effective to decrease inactivation of sorivudine, e.g., by decreasing the levels of or activity of a microbial phosphorylase, e.g., thymidine phosphorylase or uridine phosphorylase. See, e.g., Table 1, row 25.

In an embodiment, a glycan composition that decreases the growth of bacteria, e.g., described herein, e.g., Gram-negative bacteria, e.g., that produce lipopolysaccharide, is administered in combination with CpG-oligonucleotide immunotherapy for cancer, to a subject, e.g., a subject having or at risk of cancer. Methods are provided comprising the administration of a glycan composition described herein to a subject in an amount effective to increase efficacy of CpG-oligonucleotide immunotherapy, e.g., by modulating the levels of or activity of a suitable microbial enzyme. See, e.g., Table 3, row 2.

In an embodiment, a glycan composition that decreases the growth of Bacteroides , e.g., Bacteroides thetaiotaomicron and/or Bacteroides fragilis, is administered in combination with Cytotoxic T lymphocyte protein 4 (CTLA4) inhibitor (e.g., antibody), to a subject, e.g., a subject having or at risk of cancer. Methods are provided comprising the administration of a glycan composition described herein to a subject in an amount effective to increase efficacy of CTLA4 inhibitor, e.g., by modulating the levels of or activity of a suitable microbial enzyme. See, e.g., Table 3, row 6.

In an embodiment, a glycan composition that decreases the growth of Staphylococcus is administered in combination with anti-inflammatory drugs, e.g., to treat inflammatory bowel disease, e.g., tumor necrosis factor (TNF) inhibitors (e.g., antibodies), to a subject, e.g., a subject having or at risk of inflammation or an inflammatory disorder, e.g., inflammatory bowel disease. Methods are provided comprising the administration of a glycan composition described herein to a subject in an amount effective to increase efficacy of the anti-inflammatory drugs, e.g., by modulating the levels of or activity of a suitable microbial enzyme. See, e.g., Table 3, row 7.

In an embodiment, a glycan composition that decreases the growth of Bacteroidetes (e.g., Bacteroidales), and/or mucolytic bacteria such as Ruminococcus gnavus, is administered in combination with an emulsifying agent, e.g., carboxymethylcellulose, polysorbate-80, to a subject, e.g., a subject having or at risk of inflammation, an inflammatory disorder, or metabolic syndrome. Methods are provided comprising the administration of a glycan composition described herein to a subject in an amount effective to increase efficacy of the emulsifying agent, e.g., by modulating the levels of or activity of a suitable microbial enzyme. See, e.g., Table 3, row 8.

Examples of other exogenous substances, enzymes, microbes, and disorders can be found in Tables 1, 2, and 3.

Glycan compositions for use in the methods described herein include the administration of a glycan composition to increase the level or prevalence (relative abundance) of a microbe, or increases its ability to make an enzyme that catalyzes the converson of a drug or prodrug into an active form.

Glycan compositions for use in methods described herein include the administration of a glycan composition to decrease the level or prevalence (relative abundance) of a microbe, or decreases its ability to make an enzyme that inhibits the converson of a drug or prodrug into an active form.

Glycan compositions for use in the methods described herein include the administration of a glycan composition to decrease the level or prevalence (relative abundance) of a microbe, or decreases its ability to make an enzyme that coverts a drug or prodrug into an undesirable form, e.g., a toxic intermediate/metabolite.

Glycan compositions for use in the methods described herein include the administration of a glycan composition to increase the level or prevalence of a microbe, or increases its ability to make an enzyme that inhibits the conversion of a drug or prodrug into an undesirable form, e.g., a toxic intermediate/metabolite.

The glycan compositions can be useful to treat a variety of disorders, as described herein. In addition, the glycan compositions can be used in combination with a substance, e.g., exogenous substance, which is processed by a microbe. Accordingly, provided herein are compositions and methods for modulating the processing of an exogenous substance, modulating an enzyme activity, identifying/selecting a treatment for a subject, reducing toxicity of a substance, increasing drug efficacy, and eliciting specific chemical modifications or reactions in vivo.

### BRIEF DESCRIPTION OF THE FIGURES

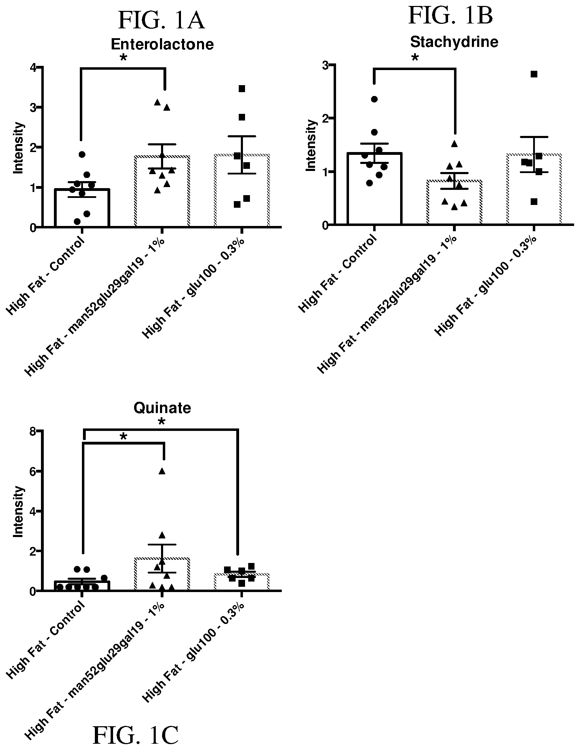

**Figures 1A, 1B, and 1C****:** A set of graphs showing modification of exogenous substances by glycan-mediated microbiota shifts. (*P<0.05, Welch two sample t-test).

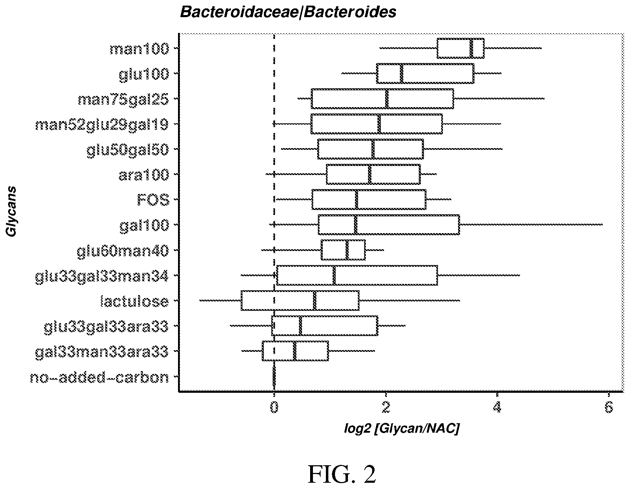

**Figure 2****:** Box and whisker plots showing the change in abundance of Bacteroidaceae/Bacteroides microbes associated, e.g., with sulfasalazine metabolism in 12 human fecal cultures (from healthy subjects) contacted with various glycan compositions described herein, commercially obtained FOS, and no added carbon control.

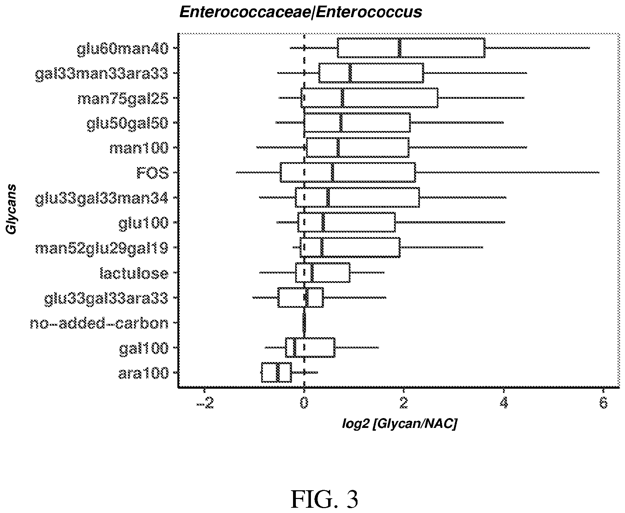

**Figure 3****:** Box and whisker plots showing the change in abundance of Enterococcaceae/Enterococcus microbes associated, e.g., with sulfasalazine metabolism, non-caloric artificial sweetener metabolism, and daidzin metabolism in 12 human fecal cultures (from healthy subjects) contacted with various glycan compositions described herein, commercially obtained FOS, and no added carbon control.

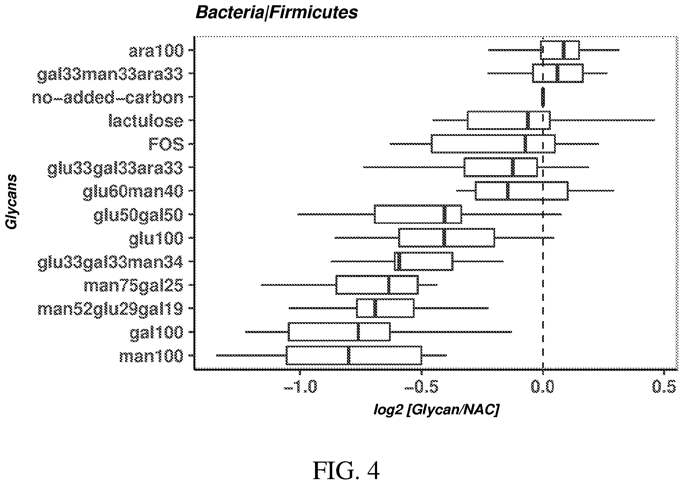

**Figure 4****:** Box and whisker plots showing the change in abundance of Bacteria/Firmicutes microbes associated, e.g., with irinotecan/SN-38 glucuronide metabolism and tyrosine and/or phenylalanine metabolism in 12 human fecal cultures (from healthy subjects) contacted with various glycan compositions described herein, commercially obtained FOS, and no added carbon control.

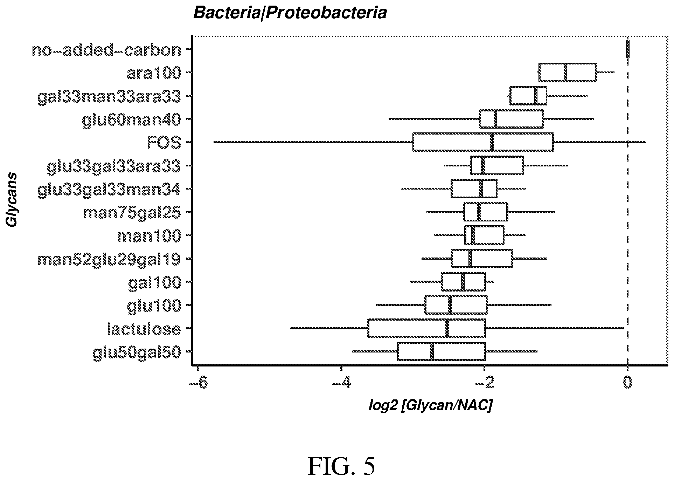

**Figure 5****:** Box and whisker plots showing the change in abundance of Bacteria/Proteobacteria microbes associated, e.g., with irinotecan/SN-38 glucuronide metabolism in 12 human fecal cultures (from healthy subjects) contacted with various glycan compositions described herein, commercially obtained FOS, and no added carbon control.

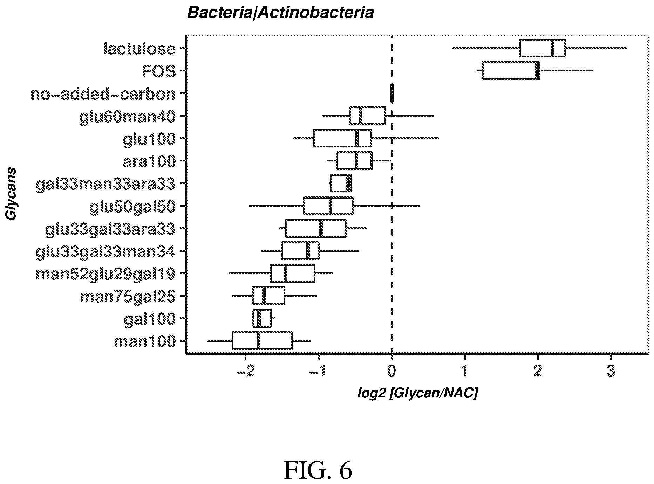

**Figure 6****:** Box and whisker plots showing the change in abundance of Bacteria/Actinobacteria microbes associated, e.g., with irinotecan/SN-38 glucuronide metabolism, tyrosine and/or phenylalanine metabolism, and ellagitannin metabolism in 12 human fecal cultures (from healthy subjects) contacted with various glycan compositions described herein, commercially obtained FOS, and no added carbon control.

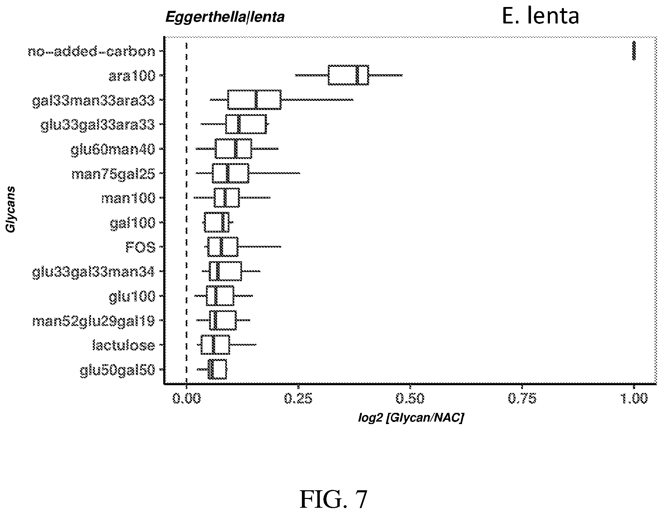

**Figure 7****:** Box and whisker plot showing the change in abundance of Eggerthella lenta microbes associated with digoxin metabolism in 12 human fecal cultures (from healthy subjects) contacted with various glycan compositions described herein, commercially obtained FOS, and no added carbon control.

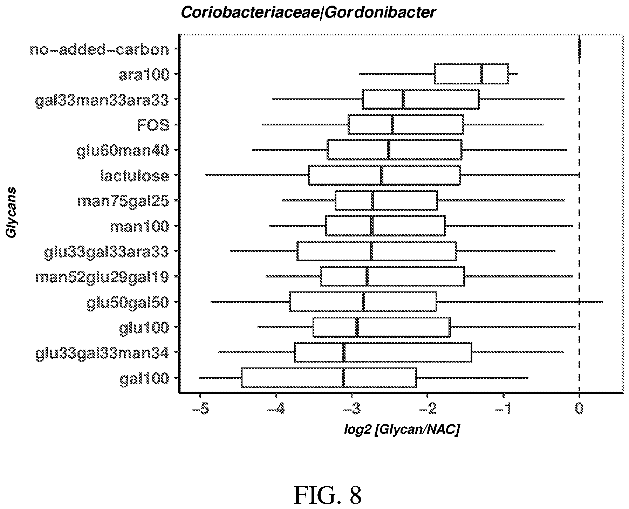

**Figure 8****:** Box and whisker plots showing the change in abundance of Coriobacteriaceae/Gordonibacter microbes associated, e.g., with ellagitannin metabolism in 12 human fecal cultures (from healthy subjects) contacted with various glycan compositions described herein, commercially obtained FOS, and no added carbon control.

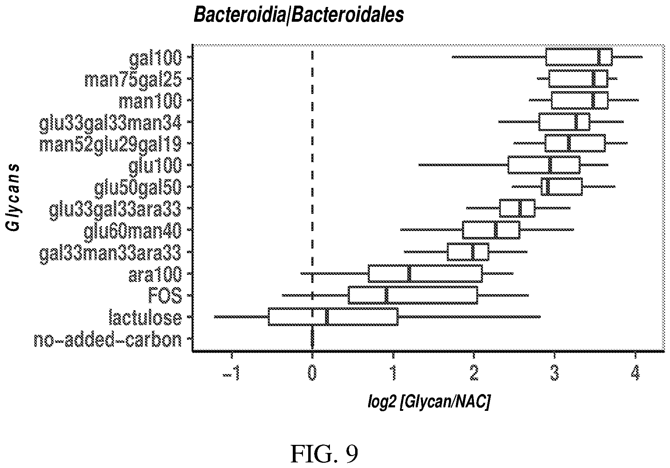

**Figure 9****:** Box and whisker plots showing the change in abundance of Bacteria/Bacteroidetes microbes associated, e.g., with phytoestrogen metabolism and CpG-oligonucleotide immunotherapy metabolism in 12 human fecal cultures (from healthy subjects) contacted with various glycan compositions described herein, commercially obtained FOS, and no added carbon control.

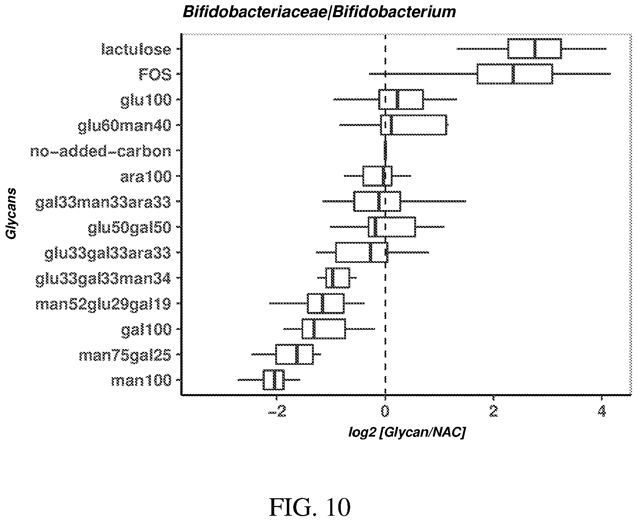

**Figure 10****:** Box and whisker plots showing the change in abundance of Bifidobacteriaceae/Bifidobacterium microbes associated, e.g., with daidzin metabolism in 12 human fecal cultures (from healthy subjects) contacted with various glycan compositions described herein, commercially obtained FOS, and no added carbon control.

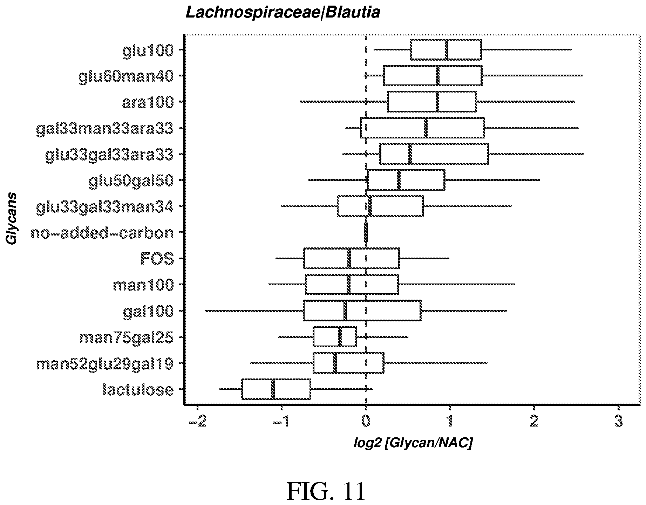

**Figure 11****:** Box and whisker plots showing the change in abundance of Lachnospiraceae/Blautia microbes associated, e.g., with lignan metabolism in 12 human fecal cultures (from healthy subjects) contacted with various glycan compositions described herein, commercially obtained FOS, and no added carbon control.

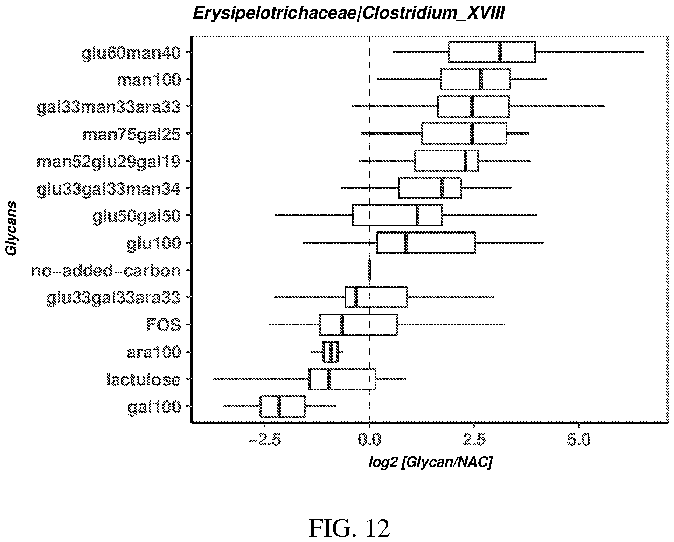

**Figure 12****:** Box and whisker plots showing the change in abundance of Erysipelotrichaceae/Clostridium_XVIII microbes associated, e.g., with lignan metabolism in 12 human fecal cultures (from healthy subjects) contacted with various glycan compositions described herein, commercially obtained FOS, and no added carbon control.

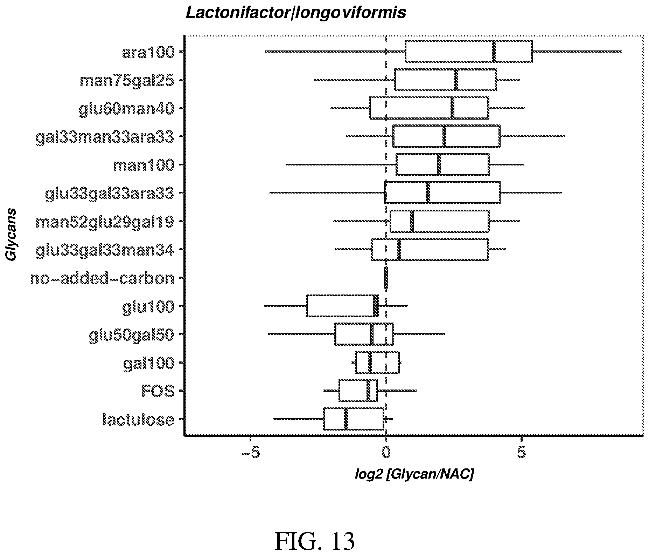

**Figure 13****:** Box and whisker plots showing the change in abundance of Lactonifactor/longoviformis microbes associated, e.g., with lignan metabolism in 12 human fecal cultures (from healthy subjects) contacted with various glycan compositions described herein, commercially obtained FOS, and no added carbon control.

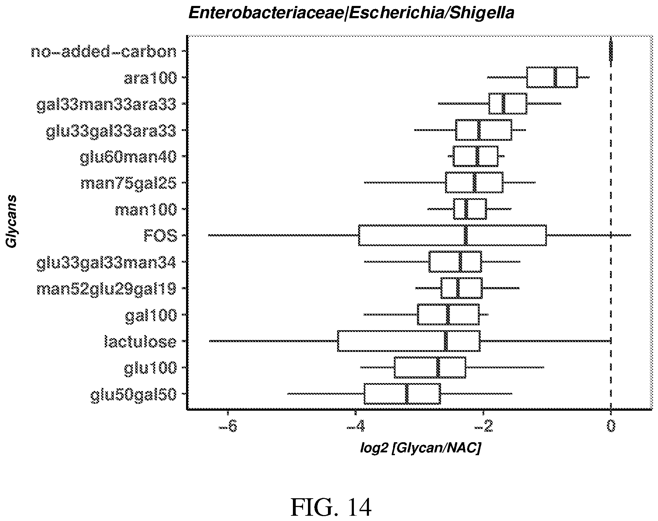

**Figure 14****:** Box and whisker plots showing the change in abundance of Enterobacteriaceae/Escherichia/Shigella microbes associated, e.g., with heterocyclic amine metabolism and non-caloric artificial sweetener metabolism in 12 human fecal cultures (from healthy subjects) contacted with various glycan compositions described herein, commercially obtained FOS, and no added carbon control.

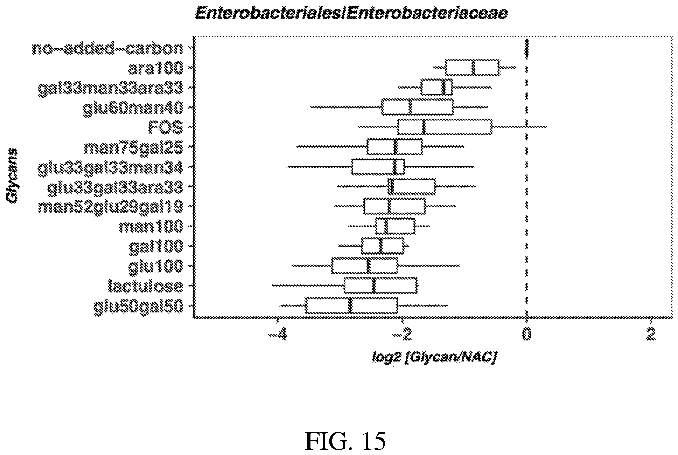

**Figure 15****:** Box and whisker plots showing the change in abundance of Enterobacteriales/Enterobacteriaceae microbes associated, e.g., with sorivudine metabolism in 12 human fecal cultures (from healthy subjects) contacted with various glycan compositions described herein, commercially obtained FOS, and no added carbon control.

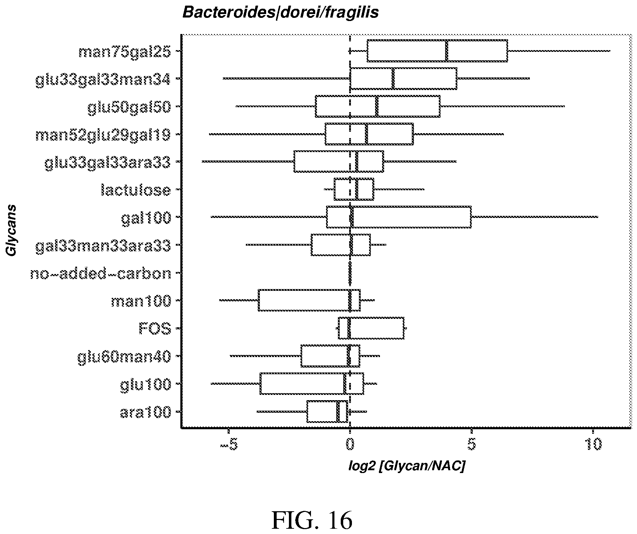

**Figure 16****:** Box and whisker plots showing the change in abundance of Bacteroides/dorei/fragilis microbes associated, e.g., with cytotoxic T lymphocyte protein 4 (CTLA4) inhibitor metabolism in 12 human fecal cultures (from healthy subjects) contacted with various glycan compositions described herein, commercially obtained FOS, and no added carbon control.

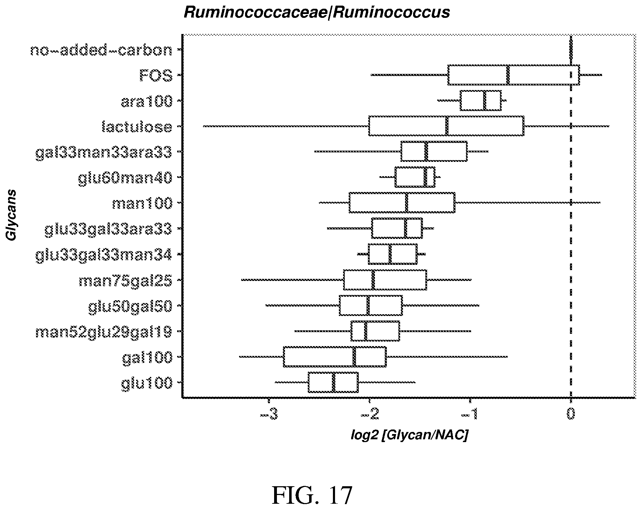

**Figure 17****:** Box and whisker plots showing the change in abundance of Ruminococcaceae/Ruminococcus microbes associated, e.g., with emulsifying agent metabolism in 12 human fecal cultures (from healthy subjects) contacted with various glycan compositions described herein, commercially obtained FOS, and no added carbon control.

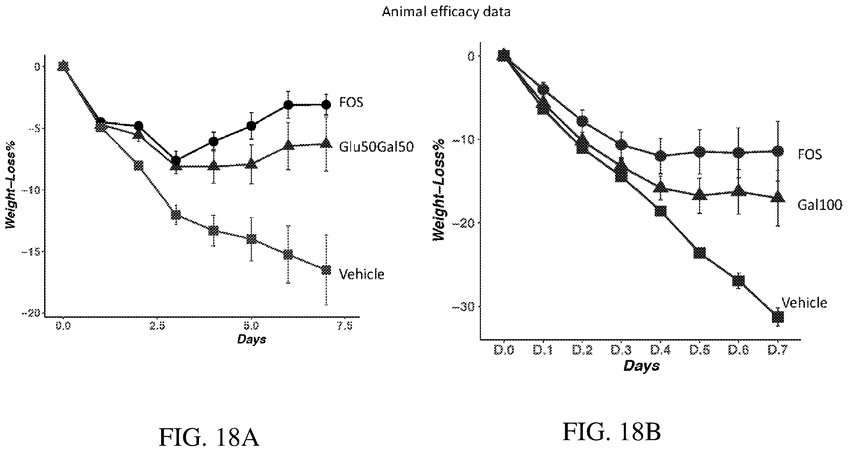

**Figures 18A and 18B****:** Graphs of weight loss over time in mice gavaged with glycans from Day -7 to Day 6. Mice were dosed with 200 mg irinotecan per kg body weight at Day 0.

### DETAILED DESCRIPTION OF THE INVENTION

Described herein are method for modulating the processing of exogenous substances, such as, e.g., a drug, a drug metabolite, a drug additive, a food, a food additive, an allergen, a toxin or toxicant. In embodiments, modulating the processing, e.g., the microbe-mediated processing of the exogenous substance alters the effect of the exogenous substance, or its processed forms, on a subject. Further described herein are glycan compositions for modulating the processing of exogenous substances. In embodiments, glycan compositions are provided as pharmaceutical compositions, medical foods, nutritional compositions, and food ingredients. Further provided are methods, which are effective to treat a number of diseases, disorders or pathological conditions.

### Definitions

As used herein, the term "abundance" or "prevalence" as it relates to a microbial taxa refers to the presence of one microbial taxa as compared to another microbial taxa in a defined microbial niche, such as the GI tract, or in the entire host organism (e.g., a human or an animal model).

"Acquire" or "acquiring" as the terms are used herein, refer to obtaining possession of a value, e.g., a numerical value, or image, or a physical entity (e.g., a sample), by "directly acquiring" or "indirectly acquiring" the value or physical entity. "Directly acquiring" means performing a process (e.g., performing a synthetic or analytical method or protocol) to obtain the value or physical entity. "Indirectly acquiring" refers to receiving the value or physical entity from another party or source (e.g., a third-party laboratory that directly acquired the physical entity or value). Directly acquiring a value or physical entity includes performing a process that includes a physical change in a physical substance or the use of a machine or device. Examples of directly acquiring a value include obtaining a sample from a human subject. Directly acquiring a value includes performing a process that uses a machine or device, e.g., an NMR spectrometer to obtain an NMR spectrum.

As used herein, "antibody" is used in the broadest sense and includes monoclonal antibodies (including full length or intact monoclonal antibodies), polyclonal antibodies, multivalent antibodies, multi-specific antibodies (e.g., bispecific antibodies), and antibody fragments so long as they exhibit the desired activity.

As used herein, the term "cancer" refers to a cell (or cells) that has an aberrant capacity for autonomous growth or replication and an abnormal state or condition (e.g. of a tissue or organ) characterized by proliferative cell growth. "Cancer" as used herein includes any solid or liquid, benign or malignant, non-invasive or invasive cancer or tumor, including hyperplasias, neoplasms, carcinoma, sarcoma, or a hematopoietic neoplastic disorder (e.g., a leukemia) and pre-cancerous or premalignant lesions.

As used herein, a "combination therapy" or "administered in combination" means that two (or more) different agents or treatments are administered to a subject as part of a defined treatment regimen for a particular disease or condition. The treatment regimen defines the doses and periodicity of administration of each agent such that the effects of the separate agents on the subject overlap. In some embodiments, the delivery of the two or more agents is simultaneous or concurrent and the agents may be co-formulated. In other embodiments, the two or more agents are not co-formulated and are administered in a sequential manner as part of a prescribed regimen. In some embodiments, administration of two or more agents or treatments in combination is such that the reduction in a symptom, or other parameter related to the disorder is greater than what would be observed with one agent or treatment delivered alone or in the absence of the other. The effect of the two treatments can be partially additive, wholly additive, or greater than additive (e.g., synergistic). Sequential or substantially simultaneous administration of each therapeutic agent can be effected by any appropriate route including oral routes, intravenous routes, intramuscular routes, and direct absorption through mucous membrane tissues. The therapeutic agents can be administered by the same route or by different routes. For example, a first therapeutic agent of the combination may be administered by intravenous injection while a second therapeutic agent of the combination may be administered orally. In some embodiments, a combination therapy means that two (or more) different agents or treatments are administered to a subject as part of a defined treatment regimen in response to a condition related to previous administration of one (or more) of the two (or more) different agents. For example, administration of a first agent may produce an undesirable condition in a subject, prompting administration of a combination therapy comprising the first agent and a second (or further) agent (taken/formulated together or separately) which addresses the undesirable condition, e.g., treats, ameliorates, or mitigates the undesirable condition.

"Distinct" as used herein, e.g. with reference to a species in a glycan polymer, is meant to denote that it is chemically and/or structurally different from another. For example, two sugars are "distinct" if they are chemically different, e.g. a fucose and a xylose, or structurally different, e.g. cyclic vs. acyclic, L- vs. D-form. Two dimers are distinct if they consist of the same two monomers but one pair contains alpha-1,4 bond and the other contains a beta-1,6 bond. Distinct entities may have any other suitable distinguishing characteristic or property that can be detected by methods known in the art and/or described herein.

As used herein, "toxin" refers to any compound, naturally occurring or made by humans, e.g., introduced into the environment by human action. Examples of toxins include toxicants, environmental pollutants (e.g., triclosan, TCDD, pesticides, and arsenic), poisons produced by mushrooms, and snake venom. As used herein, an environmental toxin is a toxin commonly encountered by humans in the environment.

As used herein, a "dosage regimen", "dosing regimen", or "treatment regimen" is a modality of drug administration that achieves a therapeutic objective. A dosage regimen includes definition of one, two, three, or four of: a route of administration, a unit dose, a frequency of dosage, or a length of treatment.

An "effective amount" and "therapeutically effective amount" as used herein refers to an amount of a pharmaceutical composition or a drug agent that is sufficient to provide a desired effect. In some embodiments, a physician or other health professional decides the appropriate amount and dosage regimen. An effective amount also refers to an amount of a pharmaceutical composition or a drug agent that prevents the development or relapse of a medical condition.

As used herein, "exogenous substance" refers to any substance introduced from or produced outside an organism, cell tissue, or system, e.g., outside a subject. In embodiments, the exogenous substance is introduced into a subject, e.g., orally, nasally, intravenously, intramuscularly. Exogenous substances can include foreign substances, e.g., that do not naturally exist in the subject. Exogenous substances can include substances that naturally exist in some humans, e.g., but may not exist in all humans, can include substance that naturally exist in some humans at some points in time but not others during their lifetime. In some embodiments, an exogenous substance includes a derivative of the exogenous substance, provided that the derivative has not been incorporated into a host macromolecule, e.g., protein, lipid, polysaccharide, or nucleic acid molecules. In some embodiments, the molecular weight of the derivative does not differ from the molecular weight of the exogenous substance by more than 5%, e.g., more than 5%, 10%, 15%, or 20%. In an embodiment, the derivative excludes products of ordinary metabolism, which incorporates atoms from a food or other energy source, vitamin, mineral, or the like. Exogenous substances are described in greater detail herein.

A "glycan unit" as used herein refers to the individual unit of a glycan disclosed herein, e.g., the building blocks from which the glycan is made.

As used herein, an "isolated" or "purified" glycan composition (or component thereof) is substantially pure and free of contaminants, e.g. pathogens or otherwise unwanted biological material, or toxic or otherwise unwanted organic or inorganic compounds. In some embodiments, pure or isolated compounds, compositions or preparations may contain traces of solvents and/or salts (such as less than 10%, 9%, 8%, 7%, 6%, 5%, 4%, 3%, 2%, 1%, less than 0.5% or 0.1% by w/w, w/v, v/v or molar %). Purified compounds are or preparations contain at least about 60% (by w/w, w/v, v/v or molar %), at least about 75%, at least about 90%, at least about 95%, at least about 97%, at least about 98%, or at least about 99% by w/w, w/v, v/v or molar % the compound(s) of interest. For example, a purified (substantially pure) or isolated glycan composition is one that is at least 80%, 85%, 90%, 91%, 92%, 93%, 94%, 95%, 98%, 99%, 99.5%, 99.8%, 99.9% or 100% of the glycan therapeutic by w/w, w/v, v/v or molar % (i.e. not including any solvent, such as e.g. water, in which the glycan composition may be dissolved) and separated from the components that accompany it, e.g. during manufacture, extraction/purification and/or processing (e.g. such that the glycan composition is substantially free from undesired compounds). Purity may be measured by any appropriate standard method, for example, by column chromatography (e.g., size-exclusion chromatography (SEC)), thin layer chromatography (TLC), gas chromatography (GC), high-performance liquid chromatography (HPLC) or nuclear magnetic resonance (NMR) spectroscopy. Purified or purity may also define a degree of sterility that is safe for administration to a human subject, e.g., lacking viable infectious or toxic agents.

As used herein, "microbiome" refers to the genetic content of the communities of microbes that live in and on a subject (e.g. a human subject), both sustainably and transiently, including eukaryotes, archaea, bacteria, and viruses (including bacterial viruses (e.g., phage)), wherein "genetic content" includes genomic DNA, RNA such as ribosomal RNA and messenger RNA, the epigenome, plasmids, and all other types of genetic information. In some embodiments, microbiome specifically refers to genetic content of the communities of microorganisms in a niche.

"Microbiota" as used herein refers to the community of microorganisms that occur (sustainably or transiently) in and on a subject (e.g. a human subject), including eukaryotes, archaea, bacteria, and viruses (including bacterial viruses, e.g. phage). In some embodiments, microbiota specifically refers to the microbial community in a niche.

"Modulate the microbiota" or "modulating the microbiota" as used herein refers to changing the state of the microbiota. Changing the state of the microbiota may include changing the structure and/or function of the microbiota. A change in the structure of the microbiota is, e.g., a change in the relative composition of a taxa, e.g., in one or more region of the GI tract such as the cecum, ascending colon, transverse colon, descending colon, sigmoid colon, and/or rectum. In an embodiment, a change in the structure of the microbiota comprises a change in the abundance of a taxa, e.g., relative to another taxa or relative to what would be observed in the absence of the modulation. Modulation of the microbiota may also, or in addition, include a change in a function of the microbiota, such as a change in microbiota gene expression, level of a gene product (e.g., RNA or protein), or metabolic output of the microbiota. Modulation of the structure or function of the microbiota may additionally induce a change in one or more functional pathway of the host (e.g., a change in gene expression, level of a gene product, and/or metabolic output of a host cell or host process) as a result of a change in the microbiota or its function.

As used herein, the term "oligosaccharide" refers to a molecule consisting of multiple (i.e., two or more) individual glycan units linked covalently. Each glycan unit may be linked through a glycosidic bond (e.g., a 1->2 glycosidic bond, a 1->3 glycosidic bond, a 1->4 glycosidic bond, a 1->5 glycosidic bond or a 1->6 glycosidic bond) present in either the alpha or beta configuration.

As used herein, a "pharmaceutical composition" or "pharmaceutical preparation" is a composition or preparation having pharmacological activity or other direct effect in the mitigation, treatment, or prevention of disease, and/or a finished dosage form or formulation thereof and is for human use. A pharmaceutical composition or pharmaceutical preparation is typically produced under good manufacturing practices (GMP) conditions. Pharmaceutical compositions or preparations may be sterile or non-sterile. If non-sterile, such pharmaceutical compositions meet the microbiological specifications and criteria for non-sterile pharmaceutical products as described in the U.S. Pharmacopeia (USP) or European Pharmacopoeia (EP). Pharmaceutical compositions may further comprise or may be co-administered with additional active agents, such as, e.g. additional therapeutic agents. Pharmaceutical compositions may also comprise pharmaceutically acceptable excipients, solvents, carriers, fillers, or any combination thereof.

As used herein, the term "polysaccharide" refers to a polymeric molecule consisting of multiple individual glycan units linked covalently. In some embodiments, a polysaccharide comprises at least 10 or more glycan units (e.g., at least 10, at least 15, at least 20, at least 25, or at least 50, at least 100, at least 250, at least 500, or at least 1000 glycan units). Each glycan unit may be linked through a glycosidic bond (e.g., a 1->2 glycosidic bond, a 1->3 glycosidic bond, a 1->4 glycosidic bond, a 1->5 glycosidic bond and a 1->6 glycosidic bond) present in either the alpha or beta confirguation. In some embodiments, a polysaccharide is a homogenous polymer comprising identical repeating units. In other embodiments, a polysaccharide is a heterogenous polymer comprised of varied repeating units. Polysaccharides may further be characterized by a degree of branching (DB, branching points per residue) or a degree of polymerization (DP).

As used herein, the term "subject" or "patient" generally refers to any human subject. The term does not denote a particular age or gender. Subjects may include pregnant women. Subjects may include a newborn (a preterm newborn, a full-term newborn), an infant up to one year of age, young children (e.g., 1 yr to 12 yrs), teenagers, (e.g., 13-19 yrs), adults (e.g., 20-64 yrs), and elderly adults (65 yrs and older). In general, a subject comprises a host and its corresponding microbiota.
A "substantial decrease" as used herein is a decrease of 5%, 10%, 20%, 30%, 40%, 50%, 60%, 70%, 80%, 90%, 95%, 97%, 98%, 99%, 99.9%, or 100%.

A "substantial increase" as used herein is an increase of 10%, 20%, 30%, 40%, 50%, 60%, 70%, 80%, 90%, 100%, 150%, 200%, 250%, 300%, 350%, 400%, 450%, 500%, 550%, 600%, 650%, 700%, 750%, 800%, 850%, 900%, 950%, 1000%, or more than 1000%.

"Synthetic" as used herein refers to a man-made compound or preparation, such as a glycan composition, that is not naturally occurring. In one embodiment, the polymeric catalyst described herein is used to synthesize the glycans of the preparation under suitable reaction conditions, e.g. by a polymerization reaction that creates oligomers and polymers from individual glycan units that are added to the reaction. In some embodiments, the polymeric catalyst acts as a hydrolysis agent and can break glycosidic bonds. In other embodiments, the polymer catalyst can form glycosidic bonds.

The terms "treating" and "treatment" as used herein refer to the administration of an agent or composition to a subject (e.g., a symptomatic subject afflicted with an adverse condition, disorder, or disease) so as to affect a reduction in severity and/or frequency of a symptom, eliminate a symptom and/or its underlying cause, and/or facilitate improvement or remediation of damage, and/or preventing an adverse condition, disorder, or disease in an asymptomatic subject who is susceptible to a particular adverse condition, disorder, or disease, or who is suspected of developing or at risk of developing the condition, disorder, or disease.

The term "antigen" refers to a substance capable of eliciting an immune response and ordinarily this is also the substance used for detection of the corresponding antibodies by one of the many in vitro and in vivo immunological procedures available for the demonstration of antigen-antibody interactions. Similarly, the term allergen is used to denote an antigen having the capacity to induce and combine with antibodies; however, this definition does not exclude the possibility that allergens may also induce antibodies of classes other than IgE.

As used herein, "derivative" refers to the product of a processed exogenous substance. A derivative can include a metabolite and/or a product of any enzymatic reaction described herein.

As used herein, administered "in combination", means that two (or more) different treatments, e.g., treatments described herein, are delivered to a subject, e.g., during the course of the subject's affliction with a disorder/condition. For example, the two or more treatments are delivered after the subject has been diagnosed with the disorder/condition and before the disorder/condition has been cured or eliminated or treatment has terminated for other reasons. In embodiments, the delivery of one treatment is still occurring when the delivery of the second commences, i.e., there is overlap in terms of administration. This is sometimes referred to herein as "simultaneous" or "concurrent delivery". In other embodiments, the delivery of one treatment stops before the delivery of the other treatment starts. In some embodiments of either case, the treatment is more effective because of the combined administration. For example, the second treatment is more effective, e.g., an equivalent effect is seen with less of (e.g., a lower dosage of) the second treatment, or the second treatment reduces symptoms to a greater extent, than would be seen if the second treatment were administered in the absence of the first treatment, or the analogous situation is seen with the first treatment. In some embodiments, delivery of the treatments is such that the reduction in a symptom, or other parameter related to the disorder/condition is greater than what would be observed with one treatment delivered in the absence of the other. In embodiments, the effect of the two treatments can be partially additive, wholly additive, or greater than additive. In embodiments, the delivery can be such that an effect of the first delivered treatment is still detectable when the second treatment is delivered.

"Fructooligosaccharide" or "FOS", as the terms are used herein, refer to a fructose polymer, optionally comprising terminal glucose, of the following sequence: (Fru)n-Glc consisting of one or more of: beta 2,1, beta 2,6, alpha 1,2 and beta-1,2 glycosidic bonds, wherein n typically is 3-10. Variants include Inulin type β-1,2 and Levan type β-2,6 linkages between fructosyl units in the main chain. In an embodiment, FOS is made from an enzyme from B. macerans, Z. mobilis, L. reutri, A. niger, A. japonicas, A. foetidus, A. sydowi,bA. Pullans, C. purpurea, F. oxysporum P. citrinum, P. frequentans, P. spinulosum, P. rigulosum, P. parasitica S. brevicaulis, S. cerevisiae, or K. marxianus. In embodiments FOS is produced by enzymatic action of a Fructosyltransferase, β-fructofuranosidase (EC 3.2.1.26), inulosuscrase (EC 2.4.1.9) levansucrase (EC 2.4.1.10), or endoinulinase.

As used herein, a "glycan polymer preparation" (also referred to as a "preparation of glycan polymers", "glycan preparation" or "glycan polymer") is a preparation comprising glycan polymers that exhibits a desired effect (e.g., a therapeutic effect or a modulating effect, e.g., with regard to an exogenous substance, or a beneficial effect, e.g., with regard to a subject's health). In some embodiments, preparations of glycan polymers do not contain one or more naturally occurring oligosaccharide, including: glucooligosaccharide, mannanoligosaccharide, inulin, lychnose, maltotretraose, nigerotetraose, nystose, sesemose, stachyose, isomaltotriose, nigerotriose, maltotriose, melezitose, maltotriulose, raffinose, kestose, fructooligosaccharide, 2'-fucosyllactose, galactooligosaccharide, glycosyl, idraparinux, isomaltooligosaccharide, maltodextrin, xylooligosaccharide, agar, agarose, alginic acid, alguronic acid, alpha glucan, amylopectin, amylose, arabioxylan, beta-glucan, callose, capsulan, carrageenan, cellodextrin, cellulin, cellulose, chitin, chitin nanofibril, chitin-glucan complex, chitosan, chrysolaminarin, curdlan, cyclodextrin, alpha-cylcodextrin, dextran, dextrin, dialdehyde starch, ficoll, fructan, fucoidan, galactoglucomannan, galactomannan, galactosamineogalactan, gellan gum, glucan, glucomannan, glucoronoxyland, glycocalyx, glycogen, hemicellulose, hypromellose, icodextrin, kefiran, laminarin, lentinan, levan polysaccharide, lichenin, mannan, mucilage, natural gum, paramylon, pectic acid, pectin, pentastarch, phytoglycogen, pleuran, poligeenan, polydextrose, porphyran, pullulan, schizophyllan, sepharose, sinistrin, sizofiran, sugammadex, welan gum, xantham gum, xylan, xyloglucan, zymosan, and the like. In some embodiments, a glycan polymer exists as a salt, e.g., a pharmaceutically acceptable salt. In some embodiments, glycan preparations do not contain sorbitol. In some embodiments, glycan preparations do not contain citric acid. In some embodiments, glycan preparations do not contain cyclic glycans.

"Increasing drug activity" as that term is used herein, refers to one or more of any of: a) increasing a therapeutic or other beneficial effect, of a drug on a weight, molar, number of molecules, or dosage unit, basis. By way of example, the administration of X mgs of a drug, when the drug activity is increased, has greater activity than the administration of X mgs in the absence of the increase. While not wishing to be bound by mechanism, an increase in drug activity can comprise an increase in conversion from inactive form to active form, e.g., an increase in processing of a drug to a prodrug, a decrease in the conversion of an active form of the drug to an inactive from, a decrease in the elimination of an active form of a drug (e.g., by excretion), etc.; b) increasing the amount of drug that can be administered relative to the amount that can be administered before reaching a non-therapeutic event. Exemplary non-therapeutic events comprise: toxicity, e.g., toxicity of the drug, or of a species arising from metabolism of the drug or off-target activity; c) increasing drug efficacy, wherein drug efficacy refers to the ability of a drug to produce an effect, e.g., a desired effect, e.g., increasing GI motility or lowering cholesterol levels. In embodiments, drug efficacy includes a drug's intrinsic activity, which can be expressed as the amount of a biological effect produced per unit of drug-receptor complex formed. A drug's bioavailability refers to the proportion of the drug that enters the circulation of a subject after administration and is thus able to elicit an effect. Drug potency refers to the measure of drug activity expressed in terms of the amount of drug required to produce an effect with a predetermined intensity. Drug efficacy can be measured qualitatively and/or quantitatively. In embodiments, drug efficacy can be measured by detecting an improvement in (e.g., lack of) one or more symptoms of the disease/disorder that the drug was intended to treat. In embodiments, drug efficacy can be measured in vitro, e.g., in a cell or tissue sample, e.g., cell culture, by determining a functional readout (e.g., protein, e.g., enzyme level or activity, production of a molecule such as a second messenger, posttranslational modification such as phosphorylation of a protein, or changes in gene expression) after incubation of the cell or tissue sample with the drug. In other embodiments, drug efficacy can be measured ex vivo or in vivo, e.g., by determining a functional readout after administration of the drug or incubation ex vivo with a sample from the subject; d) increasing drug potency, wherein drug potency can be measured by determining the drug's EC50 (half maximal effective concentration), which is the concentration of the drug at which the effect is 50% of Emax (the maximum possible effect for the drug). The lower the EC50, the higher the potency of the drug. EC50 of a drug can be determined by standard methods, e.g., measuring a functional readout in a cell or tissue sample, e.g., cell culture, after administration of increasing doses of the drug; or e) increasing drug (bio)availability, wherein drug bioavailability can be measured by determining the area under the plasma concentration-time curve (AUC), which is directly proportional to the total amount of drug (e.g., unmodified drug) that reaches the systemic circulation of a subject.

### Exogenous substances

Methods described herein modulate the processing of an exogenous substance. In some embodiments, methods described herein modulate the ability of the microbiome to mediate the processing of an exogenous substance. Exogenous substances can include a variety of agents, e.g., pharmaceutical agents, environmental toxins or toxicants, dietary components, food additives, drug additives, and/or allergens.

In one embodiment, the glycan composition of the invention is for use in increasing drug activity in a human subject, wherein the drug comprises 4-aminosalicylic acid, 5-aminosalicylic acid, balsalazide, olsalazine, or sulfasalazine.

Pharmaceutical agents can be, e.g., a protein/peptide/polypeptide (e.g., antibody molecule or fragment thereof), a nucleic acid (e.g., DNA, RNA, and/or inhibitory nucleic acid, e.g., siRNA, RNAi, miRNA), or modified forms thereof, or a small molecule.

Pharmaceutical agents include an enzyme, a receptor, an antibody, or an adaptor protein. In embodiments, a pharmaceutical agent is an FDA approved agent, e.g., approved for preventing or treating a disorder, disease, or condition described herein.

In some embodiments, the pharmaceutical agent is a prodrug, e.g., that requires bioactivation by the subject (e.g., by a microorganism in the subject, e.g., gut microorganism in the subject) to become an active drug form. Examples of prodrugs are irinotecan, protonsil, or sulfasalazine. For example, a prodrug comprises an azo bond, such as, e.g., protonsil and sulfasalazine.

Exemplary classes of pharmaceutical agents include but are not limited to an anti-inflammatory agent, a non-steroidal anti-inflammatory drug (NSAID), a statin, an antioxidant, an antimicrobial (e.g., antibiotic or antifungal), a cancer therapy, an immunotherapy (e.g., for cancer), an antibody therapy, a protein or peptide therapy, a cell based therapy, a nucleic acid therapy, or a macrolide.

Exemplary pharmaceutical agents (and some exemplary processed/metabolized forms thereof) include 5-aminosalicylic acid, 5-fluorouracil, balsalazide, benzylpenicillin, BILR 355, calcitonin, chloramphenicol, clonazepam, deleobuvir, diclofenac (glucuronide), digoxin, eltrombopag, flucytosine, glyceryl trinitrate, glycyrrhizin, indocine (n-oxide), indomethacin glucuronide, insulin, isosorbide dinitrate, ketoprofen (glucuronide), levamisole, levodopa, loperamide (N-oxide), lovastatin, methamphetamine, methotrexate, metronidazole, misonidazole, morphine (6-glucuronide), neo-prontosil, nitrazepam, nizatidine, olsalazine, omeprazole, phenacetin, potassium oxonate, prontosil, ranitidine, risperidone, sennosides, irinotecan (SN-38G), sodium picosulfate, sorivudine, succinylsulfathiazole, sulfapyridine, sulfasalazine, sulfinpyrazone, sulindac, and/or zonisamide. Additional exemplary pharmaceutical agents are described herein.

Exemplary pharmaceutical agents (and some exemplary processed/metabolized forms thereof) include:
i) Nonsteroidal anti-inflammatory (NSAID) drug, such as, e.g., 5-aminosalicylic acid and derivatives, e.g., balsalazide, olsalazine, sulfasalazine (aminosalicylate anti-inflammatory drug), diclofenac (=> glucuronide) (for pain, migraines, and arthritis), indocine (=> N-oxide), indomethacin (=> glucuronide), ketoprofen (=> glucuronide) (propionic acid class), sulindac,
ii) Chemotherapeutic drug, such as, e.g., 5-fluorouracil and methotrexate (antimetabolite antineoplastic agents and immunosuppressants for cancer), irinotecan (SN-38G) (colon and rectum cancer)
iii) Antibiotics/Antibacterials, such as, e.g., benzylpenicillin (a penicillin-class antibacterial), chloramphenicol, metronidazole, prontosil, neo-prontosil, sulfapyridine
iv) Antivirals, such as, e.g., BILR 355 and sorivudine (a nucleoside analog/reverse transcriptase inhibitor, e.g., for HIV), deleobuvir (a non-nucleoside polymerase inhibitor for hepatitis C virus)
v) Antifungals, such as, e.g., flucytosine (5-FC) (a Pyrimidine analogue),
vi) Antinematodals (e.g., anti-worm drugs), such as, e.g., levamisole (an immunomodulatory agent for hookworm infections),
vii) Hormones, such as, e.g., calcitonin (a thyroid gland hormone, e.g., for osteoporosis, cancer-related bone pain), insulin (glucose levels)
viii) Sedatives, such as, e.g., clonazepam (a Benzodiazepine for seizures, panic disorder, and anxiety)
ix) Heart medications/high blood pressure medication, such as, e.g., cardiac glycosides, such as, e.g. digoxin (an antiarrhythmic agent for heart failure), glyceryl trinitrate (for heart failure and high blood pressure), isosorbide dinitrate (nitrate) (for chest pain (angina)),
x) Colony-stimulating factors, such as, e.g., eltrombopag (a bone marrow stimulant for thrombocytopenia and aplastic anemia),
xi) Emulsifiers/ gel-forming agents, such as, e.g., glycyrrhizin (a saponin, e.g., for foodstuff and cosmetics)
xii) Dopamines, such as, levodopa (a dopamine precursor for Parkinson's disease and Parkinson's-like symptoms)
xiii) Opioid receptor agonists, such as, e.g., loperamide (=> N-oxide) (for diarrhea),
xiv) Statins, such as, e.g., lovastatin (for high cholesterol and triglyceride levels),
xv) CNS stimulants, such as, e.g. methamphetamine (for ADHD and recreational drug use)
xvi) Sensitizers/radio-therapy agents, such as, e.g., misonidazole (a nitroimidazole acting as a radiosensitizer in radiation therapy)
xvii) Narcotic pain relievers, such as, e.g., morphine (=> 6-glucuronide),
xviii) Hypnotic drugs, such as, e.g., nitrazepam (benzodiazepine class for anxiety, insomnia, amnestic, anticonvulsant, and skeletal muscle relaxant)
xix) Antiacids/ proton-pump inhibitor, such as, e.g., nizatidine, ranitidine and omeprazole (H2 antagonist) (for ulcers, gastroesophageal reflux disease (GERD)),
xx) Analgesics, such as, e.g., phenacetin (pain relief),
xxi) Uricase inhibitors, such as, e.g., potassium oxonate (for inhibition of 5-fluorouracil-induced gastrointestinal toxicity),
xxii) Antipsychotics, such as, e..g., risperidone (for schizophrenia, bipolar disorder, and irritability caused by autism),
xxiii) Laxatives, such as, e.g., sennosides (Senna glycoside) and sodium picosulfate (for constipation)
xxiv) Sulfonamides, such as, e.g., succinylsulfathiazole, sulfapyridine, sulfasalazine,
xxv) Anticonvulsant, such as, e.g., zonisamide (for seizures, epilepsy)
xxvi) Immunetherapy agents, such as, e.g., cytotoxic T-lymphocyte associated antigen 4 (CTLA-4) and CpG oligonucleotides (for cancer)

In some disclosures, dietary components can include substances that are diet-derived and that can have bioactivity, e.g., can affect health and/or disease of a subject.

In some disclosures, the dietary component comprises caffeic acid, chlorogenic acid, choline, cycasin, ellagic acid, geniposide, 2-amino-3-methylimidazo[4,5-f]quinoline (IQ), quinic acid, an ellagitannin (e.g., punicalagin, pedunculagin), a flavone (e.g., baicalin, catechin/epicatechin, hesperidine, quercetin-3-glucoside), isoflavones (e.g., daidzein, genistein, glycitein), and/or a lignan (e.g., pinoresinol, secoisolariciresinol).

In some disclosures, the dietary component comprises a polyphenol, e.g., a polyphenol described herein, e.g., anthocyanin or proanthocyanidin. In embodiments, the dietary component comprises a phytoestrogen, e.g., a phytoestrogen described herein, e.g., isoflavone or lignan. In some disclosures, the dietary component comprises a heterocyclic amine. In embodiments, the dietary component comprises a choline-containing compound, e.g., L-carnitine or phosphatidylcholine.

Food additives and/or drug additives can include chemicals that are added to foods and/or drugs, e.g., to enhance their shelf life and/or flavor. Such food additives and drug additives can interact with gut microbes. Examples of food additives include artificial sweeteners (e.g., cyclamate, xylitol, saccharin), emulsifiers (e.g., carboxymethylcellulose or polysorbate-80), and/or contaminants (e.g., melamine). Some contaminants in foods can be toxic, e.g., melamine and processed intermediates thereof. Examles of food and drug additives include acedoben, cyclamate, lactitol, lactulose, melamine, rebaudioside a, and/or stevioside.

Environmental toxins and toxicants can include chemicals that may affect gut microbes, e.g., affect their growth and/or metabolism. Exemplary environmental toxins can include bisphenol A, oxybenzone, fluoride, parabens, phthalates, butylated hydroxyanisole, perfluorooctanoic acid, perchlorate, decabromodiphenyl ether, and asbestos.

Allergens are a type of antigen that produces an abnormal, vigorous immune response to an antigen, which should normally be perceived by the body as harmless. Exemplary classes of allergens include animal components, drugs, foods, insect components, mold spores, chemicals, and plant components. Exemplary animal component allergens include Fel d1 (from cats), animal fur, animal dander, cockroach calyx, wool, and dust mite excretions. Exemlpary drug allergens include penicillin, sulfonamides, and salicylates. Exemplary food allergens include celery, celeriac, corn, maize, eggs (e.g., egg albumin), fruit, legume (e.g., beans, peas, peanuts, soybeans), milk, seafood, sesame, soy, tree nut (e.g., pecan, almond), and wheat. Exemplary insect component allergens include bee sting venom, wasp sting venom, and mosquito stings. Exemlary chemical allergens include nickel sulfate, Balsam of Peru, fragances, quaternium-15, neomycin, latex, and metal. Exemplary plant component allergens include wood, grass (e.g., ryegrass or timothy grass), weed (e.g., ragweek, plantago, nettle, Chenopodium album, sorrel, and Artemisia vulgaris), and trees (e.g., birch, alder, hazel, hornbeam, Aesculus, willow, Platanus, Tilia, Olea, poplar, Ashe juniper, and Alstonia scholaris).

### Processing of exogenous substances

### Chemical reactions

In embodiments, the processing of the exogenous substance comprises modulation of the level of derivatization and/or degradation of the exogenous substance. In an embodiment, the glycan composition modulates the ability of a microbe, e.g., of the gut, to produce an entity, e.g., an enzyme, that processes, e.g., derivatizes and/or degrades, the exogenous substance, e.g., a drug or a drug metabolite or intermediate. In embodiments, the processing of the exogenous substance comprises metabolizing (e.g., generating of one or more metabolites or intermediates, e.g., from the exogenous substance as a starting material).

In embodiments, the processing of the exogenous substance comprises a reaction such as hydrolysis, oxidation, reduction, aromatization, alkylation, acylation, phosphorylation, glycosylation, sulfation, and/or nitrosylation. In an embodiment, the glycan composition modulates the ability of a microbe, e.g., the gut, to produce an enzyme that catalyzes the hydrolysis, oxidation, reduction, aromatization, alkylation, acylation, phosphorylation, glycosylation, sulfation, and/or nitrosylation, the exogenous substance. In embodiments, the exogenous substance is a drug, a drug metabolite, a drug additive, a food, a food additive, an allergen, a toxin or toxicant.

In embodiments, the processing occurs in vivo, e.g., in a host, e.g., subject described herein.

Provided herein are methods of (i) hydroxylating, (ii) methylating, (iii) sulfonating, (iv) hydrolyzing, (v) oxidizing, (vi) reducing, (vii) aromatizing, (viii) alkylating, (ix) acylating, (x) phosphorylating, (xi) glycosylating, (xii) sulfating, and/or (xiii) nitrosylating, an exogenous substance in vivo in a subject, comprising administering a glycan composition to the subject. In an embodiment, the glycan composition modulates the ability of a microbe of the microbiome, e.g., of the gut, to produce an enzyme that i) hydroxylates, (ii) methylates, (iii) sulfonates, (iv) hydrolyzes, (v) oxidizes, (vi) reduces, (vii) aromatizes, (viii) alkylates, (ix) acylates, (x) phosphorylates, (xi) glycosylates, (xii) sulfates, and/or (xiii) nitrosylates, the exogenous substance. In embodiments, the exogenous substance is a drug, a drug metabolite, a drug additive, a food, a food additive, an allergen, a toxin or toxicant.

In some embodiments, modification of the exogenous substance can be detected and/or pharmacokinetic parameters can be determined using mass spectrometry analsysis, e.g., mass spectrometry analsys of blood, fecal, or urine samples taken from a subject. In some embodiments, modification of the exogenous substance can be detected and/or pharmacokinetic parameters can be determined using in vitro tests, e.g., using isolated exogenous substances as substrates and isolated biological samples (e.g., fecal samples, such as fecal slurries), isolated microbes (e.g., isolated bacterial taxa), and/or isolated enzymes or purified enzyme extracts (e.g., microbial enzymes) to modify the exogenous substances in vitro, e.g., in a test vessel, optionally using appropriate solvents, buffers, energy sources, and other suitable reaction conditions and assays that are suitable to detect the modification.

In embodiments, any one of the (i)-(xiii) is performed by a microbe, e.g., a bacterial taxa. In embodiments, any one of the (i)-(xiii) is performed by an enzyme, e.g., microbial enzyme. In embodiments, any one of the (i)-(xiii) is not performed by a host enzyme (e.g., a non-microbial, human or mammalian enzyme). In embodiments, any one of the (i)-(xiii) is performed in the gastrointestinal tract, e.g., the stomach, small intestine, and/or large intestine. In embodimetns, any one of the (i)-(xiii) is performed in a region of the small intestine (e.g., the duodenum, jejunum, or ileum). In embodiments, any one of the (i)-(xiii) is performed in a region of the large intestine (e.g., cecum, colon, or rectum). In embodiments, any one of the (i)-(xiii) is substantially performed in the colon.

### Processing enzymes

In embodiments, the processing of the exogenous substance is performed by an enzyme, e.g., a microbial (e.g., bacterial) enzyme or a host enzyme (e.g., eukaryotic, e.g., mammalian, e.g., human enzyme). In embodiments, the processing includes derivatization and/or degradation. The processing, e.g., the derivatization and/or degradation, can be carried out by an enzyme described herein. Exemplary enzymes include: (i) oxidoreductase (EC 1) (e.g., dehydrogenases, oxidases, catalases), (ii) transferase (EC 2) (e.g., aminotransferases, peptidyltransferases, glycosyltransferases), (iii) hydrolase (EC 3) (e.g., reductases (e.g. metal reductases), aromatase/cyclases, phosphorylases, glycosidases, cellulases, amylases, ureases, lipases, proteases, peptidases, mannanases, pullulanases, xylanases), (iv) lyase (EC 4) (e.g., pectate lyases), (v) isomerase (EC 5) (e.g., epimerases, mutases);(vi) ligase (EC 6) (e.g., synthases);(vii) azoreductase (e.g., arylamine N-acetyltransferase);(viii) beta-glucuronidase (e.g., uridine diphosphate (UDP)-glucuronosyltransferase); and/or (ix) carboxylesterase.

In embodiments, the enzyme acts on one or more of the following types of bonds: (i) ester bonds;(ii) ether bonds;(iii) peptide bonds;(iv) carbon-nitrogen bonds, e.g., other than peptide bonds;(v) acid anhydrides;(vi) carbon-carbon bonds;(vii) halide bonds;(viii) phosphorusnitrogen bonds;(ix) sulfur-nitrogen bonds;(x) carbon-phosphorus bonds;(xi) sulfur-sulfur bonds; and/or(xii) carbon-sulfur bonds.

In embodiments, the enzyme comprises a reductase, e.g., nitrate/nitrite/nitric oxide reductase, arsenate reductase, an iron/ferric reductase, a chlorate reductase, a fumarate reductase, an aldehyde reductase, a peroxide reductase, a CO₂-reductase, a morphinone reductase, a TMAO reductase, a sulfite reductase, a DMSO reductase, a ribonucleotide reductase, a fatty acid reductase, a xylose reductase, a thioredoxin reductase, a chromium reductase, a perchlorate reductase, or a dihydrofolate reductase.

In embodiments, the enzyme comprises a hydrolase, e.g., a carboxylic-ester hydrolase, a thioester hydrolase, a phosphoric-mono(di-) (tri)ester hydrolase, a sulfuric-ester hydrolase, a diphosphoric-monoester hydrolase, a phosphoric-triester hydrolase, an exodeoxyribonuclease, an exonuclease, an endodeoxyribonuclease, an endoribonuclease, a glycosylase, a glycosidase (O, N, or S glycosidase), a trialkylsulfonium hydrolase, an ether hydrolase, or a peptidase.

In embodiments, the peptidase comprises an α-amino-acyl-peptide hydrolase, a peptidylamino-acid hydrolase, a dipeptide hydrolase, a peptidyl peptide hydrolase, an aminopeptidase, a peptidylamino-acid hydrolase, an acylamino-acid hydrolase, a dipeptidase, a dipeptidylpeptidase, a tripeptidyl-peptidase, a peptidyl-dipeptidase, a serine-type carboxypeptidase, a metallocarboxypeptidase, a cysteine-type carboxypeptidase, an omega peptidase, a serine endopeptidase, a cysteine endopeptidase, an aspartic endopeptidase, a metalloendopeptidase, a threonine endopeptidase, or an endopeptidase.

Enzymes can be produced by any of the exemplary bacterial taxa described herein, e.g., in Tables 1-6.

In embodiments, the enzyme comprises an activity, e.g., action on a substrate, described herein, e.g., in Tables 1-3.

Methods described herein include modifying an enzyme activity, e.g., activity or level of a microbial or mammalian enzyme , using a glycan composition described herein. In an embodiment, the modification of enzyme activity is or includes the increase of activity by an increase in the number or prevalence (relative abundance) of a microbe (e.g., a microbe comprising or capable of producing the enzyme or producing or capable of producing an entity that increases enzyme activity). In an embodiment, the modification of enzyme activity is or includes the decrease of activity by a decrease in the number or prevalence (relative abundance) of a microbe (e.g., a microbe comprising or capable of producing the enzyme or producing or capable of producing an entity that decreases enzyme activity).

Enzyme activity (or enzymatic activity) can include the level of (e.g., expression level of) the enzyme, activity (e.g., specific activity) of the enzyme, and/or availability/bioavailability of the enzyme, e.g, in a host. In some cases, an increase in enzyme activity may be desired, e.g., where the enzyme generates an active drug form from a prodrug, or where the enzyme detoxifies a substrate. In embodiments, the method comprises increasing the enzyme activity, e.g., by at least 5% (e.g., at least 5%, 10%, 15%, 20%, 25%, 30%, 40%, 50%, 60%, 70%, 80%, 90%, 95%, 97%, 98%, 99%, or at least 100%), or at least 1.5-fold, 2-fold, 3-fold, 4-fold, 5-fold, 10-fold, 15-fold, 20-fold, 25-fold, 50-fold, 100-fold, 250-fold, 500-fold, at least 1000-fold, or more), e.g., relative to a reference level (e.g., level of processing that occurs in the subject prior to administration of the glycan composition).

In some cases, a decrease in enzyme activity may be desired, e.g., where the enzyme creates a toxic product, e.g., metabolite or intermediate, or where the enzyme modifies an active drug form such that it is eliminated more rapidly (e.g., excreted), or otherwise decreased in its bioavailability. In embodiments, the method comprises decreasing the processing (e.g., amount of substance processed and/or rate at which the substance is processed), e.g., by at least least 5% (e.g., at least 5%, 10%, 15%, 20%, 25%, 30%, 40%, 50%, 60%, 70%, 80%, 90%, 95%, 97%, 98%, or at least 99%), or at least 1.5-fold, 2-fold, 3-fold, 4-fold, 5-fold, 10-fold, 15-fold, 20-fold, 25-fold, 50-fold, 100-fold, 250-fold, 500-fold, at least 1000-fold, or more), e.g., relative to a reference level (e.g., level of processing that occurs in the subject prior to administration of the glycan composition).

### Bacterial taxa that process the exogenous substance

In embodiments, the exogenous substance is processed by a bacterium, e.g., a bacterial taxa, e.g., an enzyme produced by a bacterial taxa. In embodiments, the exogenous substance is metabolized by the bacterial taxa. In embodiments, the processing comprises decreasing the amount of an exogenous substance or derivative/metabolite/intermeduates thereof that is toxic to the subject.

In embodiments, the processing comprises increasing excretion of the toxic derivative, e.g., decreasing the synthesis of the toxic derivative.

Exemplary bacterial taxa that can process an exogenous substance include those described herein, e.g., in Tables 1-6.

In embodiments, methods described herein comprise increasing the processing (e.g., amount of substance processed and/or rate at which the substance is processed), e.g., least 5% (e.g., at least 5%, 10%, 15%, 20%, 25%, 30%, 40%, 50%, 60%, 70%, 80%, 90%, 95%, 97%, 98%, 99%, or at least 100%), or at least 1.5-fold, 2-fold, 3-fold, 4-fold, 5-fold, 10-fold, 15-fold, 20-fold, 25-fold, 50-fold, 100-fold, 250-fold, 500-fold, at least 1000-fold, or more), e.g., relative to a reference level (e.g., level of processing that occurs in the subject prior to administration of the glycan composition).

In embodiments, methods described herein comprise decreasing the processing (e.g., amount of substance processed and/or rate at which the substance is processed), e.g., by at least least 5% (e.g., at least 5%, 10%, 15%, 20%, 25%, 30%, 40%, 50%, 60%, 70%, 80%, 90%, 95%, 97%, 98%, or at least 99%), or at least 1.5-fold, 2-fold, 3-fold, 4-fold, 5-fold, 10-fold, 15-fold, 20-fold, 25-fold, 50-fold, 100-fold, 250-fold, 500-fold, at least 1000-fold, or more), e.g., relative to a reference level (e.g., level of processing that occurs in the subject prior to administration of the glycan composition).

### Area in intestine of targeting/processing

In embodiments, the exogenous substance is present in, passes through, and/or travels to, the gastrointestinal tract (e.g., the stomach, small intestine, and/or large intestine). In embodiments, the exogenous substance is present in, passes through, and/or travels to, a region of the small intestine (e.g., the duodenum, jejunum, or ileum). In embodiments, the exogenous substance is present in, passes through, and/or travels to, a region of the large intestine (e.g., cecum, colon, or rectum). In embodiments, the exogenous substance is present in, passes through, and/or travels to the colon. In embodiments, the exogenous substance is present systemically in (e.g., is present in, passes through, and/or travels to, the circulation of) the subject. In embodiments, the exogenous substance accummulates locally, e.g., in an organ (e.g., the liver or kidney) in the subject.

In embodiments, the exogenous substance is processed (e.g., as described herein) in the gastrointestinal tract (e.g., the stomach, small intestine, and/or large intestine), e.g., a region of the small intestine (e.g., the duodenum, jejunum, or ileum); or a region of the large intestine (e.g., cecum, colon, or rectum). In embodiments, the exogenous substance is processed (e.g., as described herein) in the colon.

### Exemplary processes and effects

Tables 1, 2, 3, 4, 5, and 6 include exemplary processing enzymes, exemplary exogenous substances, exemplary bacterial taxa, examples of the action of the enzymes on the exogenous substances, and/or examples of effects that a glycan composition described herein can have on the enzyme and/or taxa. These effects include desired effects, e.g., to increase drug efficacy (e.g., where the exogenous substance comprises a drug) and/or decrease drug toxicity, and/or reduce exposure to toxic metabolites/substances.

**Table 1. Processes mediated by microbial enzymes**

| **Row Number** | **Enzyme** | **Action of Enzyme** | **Substrate (e.g., exogenous substance)** | **Enzyme can be present in the following microbes** | **Desired effect on the enzyme and/or taxa (e.g., in order to increase drug efficacy or decrease toxicity)^{1,2}** | **Exemplary diseases/conditions applicable to the enzyme/substrate/t axa** | **Exemplary glycans that can be used to achieve desired effect** |
|---|---|---|---|---|---|---|---|
| 1 | Microbial arylamine N-acetyltransfer ase | Inactivates substrate | 5-aminosalicylic acid (the bioactive component of the drug sulfasalazine) | | Inhibit or reduce levels/activity | Rheumatoid arthritis | |
| 2 | Microbial azoreductase | Activates substrate to active drug form (thereby increasing bioavailability) | Prodrug, e.g., protonsil, an antibacterial drug | | Increase levels/activity | Bacterial infection | |
| 3 | Microbial azoreductase | Activates substrate to active drug form | Prodrug, e.g., sulfasalazine | Bacteroides sp., Enterococcus faecalis, Lactobacillus sp. | Increase levels/activity (converts the prodrug protonsil into 5-aminosalicylic acid | Rheumatoid arthritis | man100, glu100, man75gal25, man52glu29gal19, g1u50ga150,ara100, FOS, ga1100, glu60man40 |
| 4 | Beta-glucuronidase (e.g., uridine diphosphate (UDP)-glucuronosylt ransferase), e.g., in microbes | Adds glucuronic acid to substrate (thereby interfering with the biological activity of the substrate and/or increasing their elimination). | Pharmaceutical agents, e.g., used to treat cancer or inflammation, hormones, bile acids | | Inhibit or reduce levels/activity of the enzyme to decrease inactivation or decrease eliminatinon of the drug | Cancer, inflammation | |
| 5 | Microbial beta-glucuronidase (e.g., in intestine) | Removes sugar moiety from SN-38 glucuronide, resulting in a toxic compound to intestinal epithelial cells (the prodrug is irrenotecan) | SN-38 glucuronide | Gut microbes (e.g., Proteobacteria, Firmicutes, or Actinobacteria) | Inhibit or reduce levels/activity of the enzyme to thereby decrease the production of a toxic metabolite | Cancer, e.g., colorectal cancer, drug-induced side effects (e.g., diarrhea) | man100, ga1100, man52glu29ga119, man75gal25, glu33ga133man34, glu100, glu50gal50, lactulose, glu33ga133ara33, FOS, glu60man40, ara100 |
| 6 | Microbial beta-glucuronidase | Liberates the glucuronide, e.g., enabling reabsorption | Glucuronide from an NSAID | | Inhibit or reduce levels/activity | Inflammation, drug-induced side effects (e.g., diarrhea) | |
| | (e.g., in intestine) | of the aglycone (the compound that exists after the removal of a glycosyl group) into enterocytes (where the NSAIDs are further metabolized into reactive metabolites, which are toxic). | | | | | |
| 7 | Bacterial reductase (e.g., cardiac glycoside reductase) | Inactivates the substrate to form dihydrodigoxin (which decreases the bioavailability) | digoxin | Eggerthella lenta, e.g., strain DSM2243 | Inhibit/reduce levels/activity of the enzyme to thereby increase the activity of drug | Cardiac diseases/disorders, e.g., cardiac arrhythmia, heart failure | ara100, ga133man33ara33, glu33ga133ara33, glu60man40, man75gal25, man100, ga1100, FOS, glu33gal33man34, glu100, man52glu29gal19, glu50gal50 |
| 8 | Microbial enzyme | Metabolizes a substrate to p-cresol | Tyrosine and/or phenylalanine | Firmicutes (e.g., Clostridium difficile), Bacteroidetes, Actinobacteria, and/or Fusobacteria | Inhibit/reduce levels/activity (in order to reduce levels of p-cresol, which competes with acetaminophen as substrates of SULT1A1) | Pain, fever, drug-induced toxicity (e.g., from acetaminophen) | man100, ga1100, man52glu29ga119, man75gal25, glu33gal33man34, glu33ga133ara33, gal33man33ara33 |
| 9 | Microbial enzyme | | Statin, e.g., simvastatin | Microbes that metabolize bile acids | Increase levels/activity (e.g., in order to increase absorption/levels of statins) | High cholesterol, coronary artery disease | |
| 10 | Microbial bile salt hydrolase | Deconjugates taurine-conjugated bile acids into free bile acids | Taurine-conjugated bile acid (e.g., tauro-betamuricholic acid) | Firmicutes (e.g., Lactobacillus) | Inhibit/reduce levels/activity (e.g., which can be done with an antioxidant, e.g., tempol) | Obesity, e.g., diet-induced obesity | ara100, fos, gal100, lactulose, man75gal25, |
| 11 | microbial enzyme | Hydrolyzes ellagitannin to ellagic acid | ellagitannin | Actinobacteria, e.g., Gordonibacter | Increase levels/activity | | man100, ga1100, man75gal25, man52glu29ga119, glu33ga133man34, glu33ga133ara33, glu50ga150, glu100, lactulose, glu60man40, FOS, ara100 |
| 12 | microbial enzyme | Metabolizes ellagic acid to a urolithin (which has antioxidant, anticancer, anti-inflammatory, and/or anti-microbial properties) | Ellagic acid | Actinobacteria, e.g., Gordonibacter | Increase levels/activity | | man100, gal100, man75gal25, man52glu29ga119, glu33ga133man34, glu33ga133ara33, glu50gal50, glu100, lactulose, glu60man40, FOS, ara100 |
| 13 | microbial enzyme | Metabolizes phytoestrogen to molecules that bind estrogen receptors (e.g., may have protective effects against breast cancer) | Phytoestrogen (e.g., isoflavone, lignan), e.g., a glycosidic isoflavone such as daidzin | Actinobacteria, Bacteroidetes, Firmicutes | Increase levels/activity | | gal100, man75gal25, man 100, glu33ga133man34, man52glu29ga119, glu100, glu50gal50, glu33ga133ara33, glu60man40, gal33man33ara33 |
| 14 | Glycosidic cleavage and reduction of an α,β-unsaturated ketone, e.g., by a microbial enzyme | Daidzin metabolized to equol (which binds estrogen receptorbeta and may be associated with lower risk/incidence of breast cancer) | daidzin | Enterococcus faecium, Lactobacillus mucosae, Bifidobacterium sp., Eggerthella sp. | increase levels/activity of the enzyme to thereby increase the levels of equol | Breast cancer | glu60man40, ga133man33ara33, man75gal25, glu50gal50, man100, FOS, lactulose |
| 15 | Microbial enzyme | Metabolizes pinoresinol and secoisolariciresinol to enterodiol and enterolactone (which may be protective against breast cancer) | Lignan (from plants), e.g., pinoresinol, secoisolariciresinol | E. faecalis, E. lenta, Blautia product, Eubacterium limosum, Clostridium scindens, Lactonifactor longoviformis, Clostridium saccharogumia, P. product, | increase levels/activity | | glu100, glu60man40, ara100, ga133man33ara33, glu33ga133ara33, glu50ga150, man75gal25, man52glu29ga119, glu33gal33man34 |
| 16 | Microbial beta-glucuronidase | Reactivates glucuronidated substrate (detoxified by hepatic glucuronidation), by removing the conjugate, generating a toxic compound | Heterocyclic amine (e.g., formed during the burning of meat), which can be carcinogenic. E.g., 2-amino-3-methylimidazo[4,5-f]quinolone (IQ), 2-amino-1-methyl-6-phenylimidazo[4,5-b]pyridine (PhIP), 2-amino-3,8-dimethylimidazo[4,5-f]-quinoxaline (MelQx). | Bacteria that carry the *uidA* gene, e.g., Escherichia coli | Inhibit/reduce levels/activity of the enzyme, thereby reducing the generation of a toxic (e.g., carcinogenic) compound | cancer | ara100, ga133man33ara33, glu33ga133ara33, glu60man40, man75gal25, man100, FOS, glu33ga133man34, man52glu29ga119, gal100, lactulose, glu100, glu50gal50 |
| 17 | Microbial glycyl radical enzyme | Metabolizes choline containing compound to form trimethylamine (TMA), which is linked to higher cholesterol and risk of cardiac conditions | Choline containing compound, e.g., L-carnitine | Microbes in the colon, e.g., Firmicutes, Proteobacteria, Actinobacteria (e.g., not including Bacteroides), and/or bacteria carrying the choline utilization (*cut*) gene cluster | Inhibit/reduce levels/activity of the enzyme, thereby reducing levels of TMA | High cholesterol or a cardiac condition, atherosclerosis | |
| 18 | Microbial enzyme | Converts sweetener to a compound that can be toxic. | Non-caloric artificial sweetener, e.g., cyclamate, xylitol, saccharin | Enterococcus, Clostridium, Corynebacterium, Campylobacter, Escherichia | Inhibit/reduce levels/activity | | ara100, ga133man33ara33, glu33ga133ara33, glu60man40, man75gal25, man100, FOS glu33ga133man34, man52glu29ga119, gal100, lactulose, glu100, glu50gal50 |
| | | E.g., converts cyclamate to cyclohexylamine, which can be toxic | | | | | |
| 19 | Microbial enzyme | Metabolizes melamine to cyanuric acid, which can lead to renal toxicity | melamine | Gut microbes, e.g., Klebsiella terrigena | Inhibit/reduce levels/activity | Renal condition, e.g., renal failure | |
| 20 | Microbial enzyme | Metabolizes bile acid to secondary bile acid, which inhibits growth of Clostridium difficile | Bile acid | Clostridium scindens | Increase levels/activity | | |
| 21 | Microbial cinnamoyl esterase | Hydrolyzes esters of caffeic acid and ferulic acid to form the active compounds, e.g., caffeic acid, ferulic acid, and p-coumaric acid (having antioxidant and anti-inflammatory properties) | Conjugated hydroxycinnamate (e.g., found in foods, such as fruits, vegetables, cereals, and coffee) | Gut microbes, e.g., Bifidobacterium, Lactobacillus, Escherichia | Increase levels/activity, e.g., of the enzyme, thereby increasing the levels of the active compounds, e.g., caffeic acid, ferulic acid, and p-coumaric acid | Inflammation | |
| 22 | Microbial enzyme | Hydrolyzes cycasin into the carcinogenic glycoside, methylazoxymethanol | Cycasin, e.g., found in some plants | microbe | Inhibit/reduce levels/activity of the enzyme, thereby decreasing the levels of methylazoxymethanol | cancer | |
| 23 | Microbial enzyme | Liberates aglycone with anticancer properties from anthocyanins | anthocyanin | microbe | Increase levels/activity, e.g., of the enzyme, thereby increasing levels of aglycones having anticancer properties | cancer | |
| 24 | Microbial enzyme, e.g., oxalate: forma te antiporter, formyl-CoA transferase, oxalyl-CoA decarboxylase | Detoxify oxalate | Oxalate (a strong chelating agent that binds to free metal cations, thereby leading to kidney stones, renal failure, hyperoxaluria, and cardiac conduction disorders), e.g., in some plants | Oxalobacter formigenes | Increase levels/activity, e.g., of the enzyme, thereby decreasing levels of oxalate | kidney stones, renal failure, hyperoxaluria, and cardiac conduction disorders | |
| 25 | Microbial phosphorylase , e.g., thymidine phosphorylase or uridine phosphorylase | Deglycosylates the active agent, sorivudine | Antiviral agent, sorivudine (active against herpes simplex virus type 1 and varicella-zoster virus) | Enterobacteria, e.g., K. pneumoniae | Inhibit/reduce levels/activity of microbe or enzyme(s), thereby decreasing the deglycosylation (inactivation) of sorivudine | Viral infection, e.g., herpes simplex virus type 1 and varicella-zoster virus | ara100, ga133man33ara33, glu60man40, FOS, man75gal25, glu33ga133man34, glu33ga133ara33, man52glu29ga119, man100, ga1100, glu100, lactulose, glu50gal50 |
| | ¹In embodiments of any method described herein, the method can include achieving one or more of the following effects on the enzyme and/or bacterial taxa listed in this table. In embodiments, provided herein are compositions for use in achieving one or more of the following effects on the enzyme and/or bacterial taxa listed in this table. | | | | | | |
| | ²Inhibiting or reducing levels/activity can include inhibiting or reducing the levels/activity by at least 5% (e.g., at least 5%, 10%, 20%, 30%, 40%, 50%, 60%, 70%, 80%, 90%, 95%, 99%, 100%, 1.5-fold, 2-fold, 3-fold, 4-fold, 5-fold, 10-fold, 15-fold, 20-fold, 50-fold, 100-fold, 500-fold, 1000-fold, or more). Increasing levels/activity can include increasing levels/activity by at least 5% (e.g., at least 5%, 10%, 20%, 30%, 40%, 50%, 60%, 70%, 80%, 90%, 95%, 99%, 100%, 1.5-fold, 2-fold, 3-fold, 4-fold, 5-fold, 10-fold, 15-fold, 20-fold, 50-fold, 100-fold, 500-fold, 1000-fold, or more). | | | | | | |

**Table 2. Processes mediated by host enzymes**

| **Enzyme** | **Action of Enzyme** | **Substrate (e.g., exogenous substance)** | **Enzyme can be present in the following microbes** | **Desired effect on the enzyme and/or taxa (e.g., in order to increase drug efficacy or decrease toxicity)^{1,2}** | **Exemplary diseases/conditions applicable to the enzyme/substrate/taxa** |
|---|---|---|---|---|---|
| Host (human) carboxylesterase | Converts substrate to bioactive compound SN-38 | Irinotecan (CPT-11) (a prodrug for treatment of colorectal cancer) | | Increase levels/activity | Cancer, e.g., colorectal cancer, drug-induced side effects (e.g., diarrhea) |
| Host Beta-glucuronidase (e.g., in liver) | Re-activates SN-38 (thereby causing increased toxicity) | SN-38 | | Inhibit or reduce levels/activity | Cancer, e.g., colorectal cancer, drug-induced side effects (e.g., diarrhea) |
| | Glucuronidates SN-38 to SN-38 glucuronide (the prodrug is irrenotecan) | | | | |
| Host Beta-glucuronidase (e.g., in liver) | Glucuronidates substrate to a glucuronide | NSAID (e.g., diclofenac, indomethacin, or ketoprofen) | | Inhibit or reduce levels/activity | Inflammation, drug-induced side effects (e.g., diarrhea) |
| Liver cytochrome P450s (CYPs) | | Pentobarbital | | | anesthesia |
| Liver enzyme(s) | Metabolizes substrate to acetaminophen sulfate (inactive), acetaminophen glucuronide (inactive), and N-acetyl-*p-*benzoquinone imine (NAPQI) (toxic). | acetaminophen | | | Pain, fever, drug-induced toxicity (e.g., from acetaminophen) |
| Human cytosolic sulfotransferase lAl (SULT1A1) | Converts p-cresol to p-cresol sulfate; converts acetaminophen to acetaminophen sulfate (inactive/non-toxic), which leads to less formation of toxic NAPQI | p-cresol and acetaminophen | | | Pain, fever, drug-induced toxicity (e.g., from acetaminophen) |
| Host CYP450 | Confers protection against carcinogenic PAHs | Polycyclic aromatic hydrocarbon (PAH), e.g., benzo[a]pyrene, e.g., found in some plant and animal foods, e.g., meats cooked over open flame | Microbe, which upregulates expression of host CYP450 enzyme(s) | Increase levels/activity of microbe, which thereby increases host CYP450 enzyme(s), thereby conferring protection against carcinogenic PAHs | cancer |
| Host 5-FU catabolic enzyme, dihydropyrimidine dehydrogenase (DPD) | Metabolizes 5-FU, preventing its toxic buildup | Antiviral agent, sorivudine, in combination with 5-fluorouracil (5-FU) | Microbe, which generates a metabolite of sorivudine, e.g., (E)-5-(2-bromovinyl)uracil (BVU). BVU inhibits the 5-FU catabolic enzyme, dihydropyrimidine dehydrogenase (DPD) | Decrease levels of the microbe, thereby preventing the toxic buildup of 5-FU, e.g., in patients prescribed both sorivudine and 5-FU | Herpes zoster, e.g., in a cancer patient |
| ¹In embodiments of any method described herein, the method can include achieving one or more of the following effects on the enzyme and/or bacterial taxa listed in this table. In embodiments, provided herein are compositions for use in achieving one or more of the following effects on the enzyme and/or bacterial taxa listed in this table. | | | | | |
| ²Inhibiting or reducing levels/activity can include inhibiting or reducing the levels/activity by at least 5% (e.g., at least 5%, 10%, 20%, 30%, 40%, 50%, 60%, 70%, 80%, 90%, 95%, 99%, 100%, 1.5-fold, 2-fold, 3-fold, 4-fold, 5-fold, 10-fold, 15-fold, 20-fold, 50-fold, 100-fold, 500-fold, 1000-fold, or more). | | | | | |
| Increasing levels/activity can include increasing levels/activity by at least 5% (e.g., at least 5%, 10%, 20%, 30%, 40%, 50%, 60%, 70%, 80%, 90%, 95%, 99%, 100%, 1.5-fold, 2-fold, 3-fold, 4-fold, 5-fold, 10-fold, 15-fold, 20-fold, 50-fold, 100-fold, 500-fold, 1000-fold, or more). | | | | | |

**Table 3. Additional exemplary interactions between microbes and exogenous substances**

| **Row Number** | **Enzyme** | **Action of Enzyme** | **Substrate (e.g., exogenous substance)** | **Enzyme can be present in the following microbes** | **Desired effect on the enzyme and/or taxa (e.g., in order to increase drug efficacy or decrease toxicity)^{1,2}** | **Exemplary diseases/conditi ons applicable to the enzyme/substra te/taxa** | **Exemplary glycans that can be used to achieve desired effect** |
|---|---|---|---|---|---|---|---|
| 1 | | | Cyclophosphamide (e.g., for cancer therapy) | Gram-positive bacteria, e.g., described herein | Increase levels/activity | cancer | |
| 2 | | | CpG-oligonucleotide immunotherapy for cancer | Bacteria, e.g., described herein, e.g., gram negative bacteria, e.g., that produce lipopolysaccharide | Increase levels/activity | Cancer | FOS, ara100, gal33man33ara33, glu60man40, glu33ga133ara33, glu50gal50, glu100, man52glu29gal19, glu33ga133man34, man 100, man75gal25, gal1100 |
| 3 | | | oxaliplatin | bacteria, e.g., described herein | Increase levels/activity | cancer | |
| 4 | | | Programmed death ligand-1 (PDL1) inhibitor (e.g., antibody) | Bifidobacteria | Increase levels/activity | cancer | |
| 5 | | | Cytotoxic T lymphocyte protein 4 (CTLA4) inhibitor (e.g., antibody) | Bacteroides , e.g., Bacteroides thetaiotaomicron and/or Bacteroides fragilis | Increase levels/activity | cancer | Man72ga125, glu33gal33man34, glu50ga150, man100, glu100, man52glu29gal19, ara100, FOS, gal100, glu60man40 |
| 6 | | | Anti-inflammatory drugs to treat inflammatory bowel disease, e.g., tumor necrosis factor (TNF) inhibitors (e.g., antibodies) | Staphylococcus | Increase levels/activity | Inflammation, e.g., inflammatory bowel disease | Man100, glu60man40 |
| 7 | | | Dietary polyphenol (e.g., anthocyanin (ACN) and/or proanthocyanidin (PAC)) | Akkermansia muciniphila (thought to preserve the integrity of the gut mucus layer) | Increase levels/activity (e.g., in order to reduce adiposity, insulin resistance, and/or inflammation, e.g., in obese subjects) | Metabolic syndrome, insulin resistance, diet-induced weight gain, adiposity, inflammation, e.g., intestinal inflammation, oxidative stress, conditions with the gut mucus layer | |
| 8 | | | Emulsifying agent, e.g., carboxymethylcellulose, polysorbate-80 | Bacteroidetes (e.g., Bacteroidales), mucolytic bacteria such as Ruminococcus gnavus | Inhibit/reduce levels/activity, e.g., in order to decrease risk/incidence of metabolic syndrome, inflammation. | | ara100, lactulose, gal33man33ara33, glu60man40, man 100, glu33ga133ara33, glu33gal33man34, man75gal25, glu50gal50, man52glu29gall9, gal1100, glu100 |
| 9 | | | Tacrolimus | Faecalibacterium prausnitzii | increase levels/activity, e.g., in order to increase tacrolimus efficacy | | |
| 10 | | Inactivatio n of L-DOPA and/or decrease its bioavailab ilitv | Dopamine precursor, levodopa (L-DOPA) | Helicobacter pylori (which can directly bind to L-DOPA, decreasing its bioavailabilitv) | Inhibit/reduce levels/activity of H. pylori or an enzyme or reactive oxygen species that inactivates L-DOPA, thereby increasing the levels of active L-DOPA and/or its bioavailability | Parkinson disease, peptic ulceration in Parkinson's patients | |
| 11 | | | Heterocyclic amine (HCA), e.g., formed during charring of meat, poultry and/or fish, e.g., 2-amino-1-methyl-6-pehylimidazol[4,5-b]pyridine (PhIP), 2-amino-3-methylimidazo[4,5-f]quinolone (IQ), 2-amino-3,8-dimethylimidazo[4,5-f]quinoxaline (MelQx), 3-amino-1-methyl-5H-pyrido(4,3-b)indole (Trp-P-2), other diet-derived mutagens | Gut microbes, e.g., Lactic acid bacteria (which can directly bind to a HCA) | Increase levels of the microbes, which directly bind to the HCA, thereby potentially preventing induction of DNA damage and preneoplastic lesions. | cancer | |
| | ¹In embodiments of any method described herein, the method can include achieving one or more of the following effects on the enzyme and/or bacterial taxa listed in this table. In embodiments, provided herein are compositions for use in achieving one or more of the following effects on the enzyme and/or bacterial taxa listed in this table. | | | | | | |
| | ²Inhibiting or reducing levels/activity can include inhibiting or reducing the levels/activity by at least 5% (e.g., at least 5%, 10%, 20%, 30%, 40%, 50%, 60%, 70%, 80%, 90%, 95%, 99%, 100%, 1.5-fold, 2-fold, 3-fold, 4-fold, 5-fold, 10-fold, 15-fold, 20-fold, 50-fold, 100-fold, 500-fold, 1000-fold, or more). | | | | | | |
| | Increasing levels/activity can include increasing levels/activity by at least 5% (e.g., at least 5%, 10%, 20%, 30%, 40%, 50%, 60%, 70%, 80%, 90%, 95%, 99%, 100%, 1.5-fold, 2-fold, 3-fold, 4-fold, 5-fold, 10-fold, 15-fold, 20-fold, 50-fold, 100-fold, 500-fold, 1000-fold, or more). | | | | | | |

**Table 4: Genus level Microbial Constituents of the GI tract**

| Phylum | Class | Genus |
|---|---|---|
| Actinobacteria | Actinobacteria | Actinomyces, Adlercreutzia, Atopobium, Bifidobacterium, Collinsella, Corynebacterium, Eggerthella, Mobiluncus, Propionibacterium, Rothia, Slackia |
| B acteroidetes | B acteroidia | Alistipes, Bacteroides, Dysgonomonas, Odoribacter, Parabacteroides, Porphyromonas, |
| | | Prevotella, Tannerella |
| | Flavobacteria | Capnocytophaga |
| Firmicutes | Bacilli | Bacillus, Enterococcus, Gemella, Granulicatella, Lactobacillus, Lactococcus, Staphylococcus, Streptococcus, Turicibacter, Weissella |
| | Clostridia | Acidaminococcus, Anaerococcus, Anaerofilum, Anaerofustis, Anaerostipes, Anaerotruncus, Anaerovorax, Bacteroides, Bacteroides, Blautia, Clostridium, Coprococcus, Dehalobacterium, Dialister, Dorea, Eubacterium, Faecalibacterium, Finegoldia, Lachnobacterium, Lachnospira, Megamonas, Megasphaera, Mitsuokella, Moryella, Oribacterium, Oscillospira, Peptococcus, Peptoniphilus, Peptostreptococcus, Phascolarctobacterium, Pseudobutyrivibrio, Roseburia, Ruminococcus, Ruminococcus, Selenomonas, Subdoligranulum, Veillonella |
| Fusobacteria | Fusobacteria | Fusobacterium, Leptotrichia |
| | Betaproteobacteria | Comamonas, Herbaspirillum, Lautropia, Neisseria, Oxalobacter, Sutterella |
| | Deltaproteobacteria | Bilophila, Desulfovibrio, LE30 |
| | Epsilonproteobacteria | Campylobacter, Helicobacter |
| | Gammaproteobacteria | Actinobacillus, Aggregatibacter, Citrobacter, Escherichia, Haemophilus, Klebsiella, Moraxella, Pseudomonas, Raoultella |
| Spirochaetes | Spirochaetes | Treponema |
| Synergistetes | Synergistetia | Cloacibacillus, Synergistes |
| Tenericutes | Erysipelotrichi | Bulleidia, Catenibacterium, Clostridium, Coprobacillus, Holdemania, RFN20 |

| | Mollicutes | Asteroleplasma, Mycoplasma |
|---|---|---|
| Verrucomicrobia | Verrucomicrobiae | Akkermansia |
| Euryarchaeota | Methanobacteria | Methanobrevibacter |

**Table 5: Genus level microbial constituents predominant in the large intestine (compared to small intestine) in healthy humans.**

| Phylum | Class | Genus |
|---|---|---|
| Bacteroidetes | Bacteroidia | Bacteroides, Butyricimonas, Odoribacter, Parabacteroides, Prevotella |
| Firmicutes | Clostridia | Anaerotruncus, Phascolarctobacterium, Ruminococcus, |
| Proteobacteria | Deltaproteobacteria | Bilophila |
| Verrucomicrobia | Verrucomicrobiae | Akkermansia |

**Table 6: Genus level microbial constituents predominant in the small intestine (compared to large intestine) in healthy humans.**

| Phylum | Class | Genus |
|---|---|---|
| Actinobacteria | Actinobacteria | Crvocola, Mvcobacterium |
| Firmicutes | Bacilli | Enterococcus, Lactococcus, Streptococcus, Turicibacter |
| | Clostridia | Blautia, Coprococcus, Holdemania, Pseudoramibacter Eubacterium |
| Proteobacteria | Alphaproteobacteria | Agrobacterium, Sphingomonas |
| | Betaproteobacteria | Achromobacter, Burkholderia, Ralstonia |

### Methods of affecting drug toxicity/efficacy

Bacterial taxa can process (e.g., using bacterial enzyme(s)) a substance, e.g., a drug, to generate or release a toxic compound/molecule. Bacterial taxa can also process (e.g., using bacterial enzyme(s)) a toxic substance such that it is less toxic, e.g., bacterial taxa can detoxify the toxic substance. In other examples, bacterial taxa can process (e.g., using bacterial enzyme(s)) a substance, e.g., prodrug, to convert it to an active form, e.g., thereby increasing its efficacy. In yet other examples, bacterial taxa can process (e.g., using bacterial enzyme(s)) a substance, e.g., a drug, e.g., active drug form, such that it is less active or not active. Methods for modulating microbial taxa and microbial activity toward exogenous substances are provided herein comprising administering a glycan composition described herein in an effective amount to modulate the microbial taxa and/or microbial activity toward exogenous substances. The compositions and methods described herein can modulate one or more bacterial taxa and/or one or more bacterial enzymes such that toxicity from substances such as drugs is reduced, and/or efficacy of drugs is increased. For example, the compositions and methods described herein can decrease the level of bacterial taxa (and/or decrease the activity of bacterial enzyme(s)) that generate release of toxic compounds/molecules. In some examples, the compositions and methods described herein can increase the level of bacterial taxa (and/or increase the activity of bacterial enzyme(s)) that detoxify substances. In other examples, the compositions and methods described herein can increase the level of bacterial taxa (and/or increase the activity of bacterial enzyme(s)) that increase the efficacy of a drug, e.g., that convert a prodrug into active form. In yet other examples, the compositions and methods described herein can decrease the level of bacterial taxa (and/or decrease the activity of bacterial enzyme(s)) that inactivate a drug or convert it into less active (e.g., inactive) form.

Provided herein are glycan compositions for use in methods for reducing drug toxicity in a human subject. In embodiments, the methods comprise administering to the subject the glycan composition as claimed in an amount effective to reduce drug toxicity. In embodiments, the subject has previously been administered, is being administered, or will be administered the drug (e.g., the drug associated with the toxicity). In embodiments, the glycan composition is administered in an amount and/or for a time sufficient to reduce the drug toxicity (and related symptoms, such as, e.g., cytotoxicity, diarrhea, constipation, nausea, dizziness, weight loss, etc.) in the subject (e.g., relative to a reference level, e.g., the drug toxicity in the subject prior to administration of the glycan composition). In embodiments, the method further comprises administering the drug to the subject, e.g., in combination with the glycan composition. In embodiments, the drug toxicity is reduced by at least least 5% (e.g., at least 5%, 10%, 15%, 20%, 25%, 30%, 40%, 50%, 60%, 70%, 80%, 90%, 95%, 97%, 98%, or at least 99%), or at least 1.5-fold, 2-fold, 3-fold, 4-fold, 5-fold, 10-fold, 15-fold, 20-fold, 25-fold, 50-fold, 100-fold, 250-fold, 500-fold, at least 1000-fold, or more) relative to a reference level (e.g., the drug toxicity in the subject prior to administration of the glycan composition).

Drug toxicity refers to one or more adverse effects in a subject resulting from the administration of a drug to the subject. The adverse effects can range from discomfort (e.g., symptoms, such as, e.g., cytotoxicity, diarrhea, constipation, nausea, dizziness, weight loss, etc.) to death in some cases. Drug toxicity can result from a number of causes including off target activity, on target toxicity, processing of the drug into a toxic metabolite or intermediate, an inappropriate dosage (e.g., too high of a dosage) for a subject, prolonged used of the drug, and/or interaction of the drug with a second drug or substance. The toxicity of a drug depends on a number of factors, including age, preexisting conditions, genetic makeup, and/or presence of other drugs or metabolites thereof in the subject. Mechanistically, drug toxicity can be due to the production of a toxic metabolite or intermediate of the drug, an on target adverse effect (e.g., where the drug binds to the correct target/receptor but is provided at an inappropriate concentration, displays suboptimal kinetics, and/or is used for the wrong indication) or an off target adverse effect (e.g., where the drug binds to the wrong target/receptor).

Drug toxicity can be measured qualitatively and/or quantitatively. In embodiments, a method (e.g., quantitative method) for measuring drug toxicity can include assays (e.g., in vitro assays) for detecting apoptosis and/or necrosis of a host cell, e.g., a host cell that lines the gastrointestinal tract or a section thereof. In embodiments, the presence of apoptosis and/or necrosis in the cell indicates that the drug is toxic. In embodiments, a lesser extent of apoptosis and/or necrosis (e.g., fewer apoptotic/necrotic cells) in the cells indicates reduced drug toxicity, e.g., compared to prior to treatment with a glycan composition described herein.

In embodiments, a method (e.g., quantitative method) for measuring drug toxicity can include measuring the number of or prevalence of particular microbial taxa (e.g., a microbial taxa described herein) in a sample from the subject, e.g., a stool sample. In embodiments, a decrease (e.g., loss) in the abundance of microbial taxa and/or a decrease (e.g., loss) in diversity of microbial taxa indicates drug toxicity, e.g., compared to prior to treatment with the drug. In embodiments, an increase in the abundance of microbial taxa and/or a increase in diversity of microbial taxa indicates decreased drug toxicity, e.g., compared to before treatment with a glycan composition described herein.

In embodiments, a method for measuring drug toxicity can include measuring the level of one or more inflammatory markers and/or other biomarker indicative of a response to injury in the subject, e.g., a sample from the subject. The level of inflammatory markers and/or other biomarkers can be measured using standard methods. In embodiments, the inflammatory marker includes one or more pro-inflammatory cytokines, e.g., TNF-α, IL-1, IL-6, and/or IL-10.

In embodiments, a qualitative method for measuring drug toxicity can include detecting one or more symptoms of drug toxicity in the subject, e.g., constipation, diarrhea, inflammation, and/or vomiting.

Also provided herein are glycan compositions for use in methods for increasing drug efficacy, potency, and/or bioavailability in a subject comprising administering to the subject the glycan composition as claimed. In embodiments, the subject has previously been administered, is being administered, or will be administered the drug. In embodiments, the glycan composition is administered in an effective amount and/or for a sufficient time to increase the drug efficacy, potency, and/or bioavailability in the subject (e.g., relative to a reference level, e.g., the drug toxicity in the subject prior to administration of the glycan composition).

Drug efficacy refers to the ability of a drug to produce an effect, e.g., a desired effect, e.g., increasing GI motility or lowering cholesterol levels. In embodiments, drug efficacy includes a drug's intrinsic activity, which can be expressed as the amount of a biological effect produced per unit of drug-receptor complex formed. A drug's bioavailability refers to the proportion of the drug that enters the circulation of a subject after administration and is thus able to elicit an effect. Drug potency refers to the measure of drug activity expressed in terms of the amount of drug required to produce an effect with a predetermined intensity.

Drug efficacy can be measured qualitatively and/or quantitatively. In embodiments, drug efficacy can be measured by detecting an improvement in (e.g., lack or milder form of) one or more symptoms of the disease/disorder that the drug was intended to treat. In embodiments, drug efficacy can be measured in vitro, e.g., in a cell or tissue sample, e.g., cell culture, by determining a functional readout (e.g., protein, e.g., enzyme level or activity, production of a molecule such as a second messenger, posttranslational modification such as phosphorylation of a protein, or changes in gene expression) after incubation of the cell or tissue sample with the drug. In other embodiments, drug efficacy can be measured ex vivo or in vivo, e.g., by determining a functional readout after administration of the drug or incubation ex vivo with a sample (e.g., a fecal sample) from the subject.

Drug potency can be measured by determining the drug's EC₅₀ (half maximal effective concentration), which is the concentration of the drug at which the effect is 50% of Eₘₐₓ (the maximum possible effect for the drug). The lower the EC₅₀, the higher the potency of the drug. EC₅₀ of a drug can be determined by standard methods, e.g., measuring a functional readout in a cell or tissue sample, e.g., cell culture, after administration of increasing doses of the drug.

Drug bioavailability can be measured by determining the area under the plasma concentration-time curve (AUC), which is directly proportional to the total amount of drug (e.g., unmodified drug) that reaches the systemic circulation of a subject.

In some embodiments, the compositions and methods can achieve one or more desired effects described in Tables 1, 2, or 3. In embodiments, the methods comprise modulating the activity of an enzyme and/or level of a microbe described in Tables 1, 2, or 3, or Tables 4, 5, or 6. In embodiments, the methods comprise administering the composition to a subject that has been, is being, or will be administered an exogenous substance described herein, e.g., in Tables 1, 2, or 3.

Also, disclosed herein are methods of identifying or selecting a treatment regimen for a subject. The application discloses a method comprising the steps of:a) acquiring a value for the presence or level of a bacterial taxa or a microbial metabolite or an enzymatic activity in the subject; b) responsive to the value, selecting a glycan composition to treat the subject; and
c) administering the glycan composition in an effective amount and/or for a sufficient time to treat the subject.

In a further disclosure, the method further comprises a step prior to step (a), of selecting a subject that has been administered, is being administered, or will be administered an exogenous substance, e.g., an environmental toxin or toxicant, a pharmaceutical agent or drug, a dietary component, a food additive, or a drug additive.

Provided herein are also methods of selecting a subject for treatment using a glycan composition described herein. In some disclosures, the subject is selected based on his or her exposure to an exogenous substance, e.g., exogenous substance described herein. In embodiments, the subject is selected for treatment if he/she has been administered (has been exposed to or contacted with), is being administered (being exposed to or contacted with), or will be administered (will be exposed to or contacted with) an exogenous substance, e.g., an environmental toxin or toxicant, a pharmaceutical agent or drug, a dietary component, a food additive, a drug additive. In some disclosures, the exogenous substance comprises an allergen. In other disclosures, the subject is selected if he/she has a disease/condition, e.g., an immune disease, an infectious disease, a metabolic disease, a neurodegenerative disease, a cancer, an allergy, or another disease or disorder or detrimental condition, including a precondition or predisposition to develop a disease or disorder. In some disclosures, the subject is selected if he/she is (e.g., determined to be) deficient in an enzyme activity (e.g., enzyme level and/or enzyme specific activity), e.g., a microbial enzyme activity. In some disclosures, the subject is selected if he/she has (e.g., is determined to have) an overabundance of an enzyme and/or an overactive enzyme, e.g., a microbial enzyme. In embodiments, the enzyme is an enzyme described herein, e.g., in Table 1, 2, or 3. In embodiments, the enzyme is an enzyme having an activity or an enzyme catalyzing a reaction described herein, e.g., in the "Processing of exogenous substances" section herein. In embodiments, the subject is selected if he/she is (e.g., determined to be) deficient in a bacterial taxa. In embodiments, the subject is selected if he/she has (e.g., is determined to be have) an overabundance of a bacterial taxa. In embodiments, the bacterial taxa comprises a bacterial taxa that is beneficial, e.g., a bacterial taxa for which an increase in levels is desired according to Tables 1, 2, 3, and 4-6. In embodiments, the bacterial taxa comprises a bacterial taxa that is detrimental, e.g., a bacterial taxa for which a decrease in levels is desired according to Table 1, 2, 3, and 4-6.

### Glycan polymer compositions and manufacture thereof

Glycan compositions can comprise the glycans described herein, dietary fibers, such as, e.g., FOS (fructooligosaccharide), other sugars (e.g., monomers, dimers, such as, e.g., lactulose) and sugar alcohols, and optionally other components, such as, e.g., polyphenols, fatty acids, peptides, micronutrients, etc., such as those described in WO 2016/172658, "MICROBIOME REGULATORS AND RELATED USES THEREOF", and microbes, such as bacteria.

Glycan preparations described in WO 2016/122889 "GLYCAN THERAPEUTICS AND RELATED METHODS THEREOF" and WO 2016/172657, "GLYCAN THERAPEUTICS AND METHODS OF TREATMENT", are suitable for in the methods and compositions described herein.

Preparations comprising glycans can be generated using a non-enzymatic catalyst, e.g., the polymeric catalyst described in WO 2012/118767, "POLYMERIC ACID CATALYSTS AND USES THEREOF" or by other suitable methods. Methods to prepare the polymeric and solid-supported catalysts described herein can be found in WO 2014/031956, "POLYMERIC AND SOLID-SUPPORTED CATALYSTS, AND METHODS OF DIGESTING CELLULOSIC MATERIALS USING SUCH CATALYSTS." The glycans generated, e.g., by using the catalyst, for example as described in WO 2016/007778, "OLIGOSACCHARIDE COMPOSITIONS AND METHODS FOR PRODUCING THEREOF" are suitable for the methods and compositions described herein.

In some embodiments, glycan polymers are made using a glycosidase enzyme molecule under conditions suitable to generate glycan polymers.

In some embodiments, glycan polymers are made using solid-phase oligosaccharide synthesis, e.g., using a variety of protection groups to accomplish glycan polymer synthesis. Examplary methods are described in "Solid-Phase Oligosaccharide Synthesis and Combinatorial Carbohydrate Libraries", Peter H. Seeberger and Wilm-Christian Haase, American Chemical Society, 2000; and "Opportunities and challenges in synthetic oligosaccharide and glycoconjugate research", Thomas J. Boltje et al., Nat Chem. 2009 November 1; 1(8): 611-622.

### Glycan preparation properties

Glycan may have any one or more of the characterisitics and properties disclosed in WO2016/122889, WO2016/172657, WO 2016/007778, and WO2016/172658.

The glycan polymers produced by the methods described herein may comprise oligosaccharides. In some embodiments, the glycan polymers comprise homo-oligosaccharides (or homoglycans), wherein all the monosaccharides in a polymer are of the same type.

In some embodiments, the glycan polymers comprise hetero-oligosaccharides (or heteroglycans), wherein more than one type of monosaccharide is present in the polymer. In some embodiments, the glycan polymers have one or more of the properties described herein. In some embodiments, the glycan polymer preparation has one or more of the bulk properties described herein.

### Degree of polymerization (DP)

In some embodiments, glycan polymer preparations are produced, e.g., using a method described herein, that are polydisperse, exhibiting a range of degrees of polymerization.

Optionally, the preparations may be fractionated, e.g. representing 60%, 65%, 70%, 75%, 80%, 85%, 90%, 95%, 97%, 98%, or greater than 98% short (about DP1-2), medium (about DP3-10), long (about DP11-18), or very long (about DP>18) species. In one embodiment, a polydisperse, fractionated glycan polymer preparation is provided comprising at least 85%, 90%, or at least 95% medium-length species with a DP of about 3-10. In one embodiment, a polydisperse, fractionated glycan polymer preparation is provided comprising at least 85%, 90%, or at least 95% long-length species with a DP of about 11-18. In one embodiment, a polydisperse, fractionated glycan polymer preparation is provided comprising at least 85%, 90%, or at least 95% very long-length species with a DP of about 18-30.

Optionally, the preparations may be fractionated, e.g. representing 60%, 65%, 70%, 75%, 80%, 85%, 90%, 95%, 97%, 98%, or greater than 98% short (about DP1-2) or medium (about DP3-10) glycans in the preparation. Alternatively, or in addition to fractionation, the small DP fraction (e.g. monomers and dimers) are subjected to enzymatic fermentation, e.g. with suitable yeasts to break down these sugars. In one embodiment, a polydisperse, fractionated glycan polymer preparation is prepared using a method described herein, comprising at least 85%, 90%, or at least 95% of glycans with a DP of about 3-10.

In some embodiments, about 55%, 60%, 65%, 70%, 75%, 80%, 85%, 90%, 95%, or about 97% of the glycan polymers of the glycan preparation have a DP of at least DP3, DP4, DP5, DP6 or DP7. In some embodiments, about 55%, 60%, 65%, 70%, 75%, 80%, 85%, 90%, 95%, or about 97% of the glycan polymers of the glycan preparation have a DP from about DP3 to about DP10, from about DP3 to about DP8, from about DP3 to about DP6, from about DP3 to about DP5, from about DP3 to about DP4, from about DP2 to about DP4, from about DP2 to about DP5, from about DP2 to about DP6, from about DP2 to about DP8, or from about DP2 to about DP10. In some embodiments, less than 1%, 2%, 3%, 5%, 10%, 15%, 20%, 25%, 30%, 40%, or less than 50% of the glycan polymers of the glycan preparation have a DP of DP2 or less.

In some embodiments, about 55%, 60%, 65%, 70%, 75%, 80%, 85%, 90%, 95%, or about 97% of the glycan polymer preparation has a DP of between 2 and 25, between 3 and 25, between 4 and 25, between 5 and 25, between 6 and 25, between 7 and 25, between 8 and 25, between 9 and 25, between 10 and 25, between 2 and 30, between 3 and 30, between 4 and 30, between 5 and 30, between 6 and 30, between 7 and 30, between 8 and 30, between 9 and 30, or between 10 and 30. In one embodiment, the glycan polymer preparation has a degree of polymerization (DP) of at least 3 and less than 30 glycan units.

In some embodiments, about 55%, 60%, 65%, 70%, 75%, 80%, 85%, 90%, 95%, or about 97% of the glycan polymer preparation has a DP of at least 5 and less than 30 glycan units. In some embodiments, about 55%, 60%, 65%, 70%, 75%, 80%, 85%, 90%, 95%, or about 97% of the glycan polymer preparation has a DP of at least 8 and less than 30 glycan units. In some embodiments, about 55%, 60%, 65%, 70%, 75%, 80%, 85%, 90%, 95%, or about 97% of the glycan polymer preparation has a DP of at least 10 and less than 30 glycan units. In some embodiments, about 55%, 60%, 65%, 70%, 75%, 80%, 85%, 90%, 95%, or about 97% of the glycan polymer preparation has a DP of between 3, 4, 5, 6, 7, 8 and 10, 11, 12, 13, 14, 15, 16, 17, 18, 19, 20 glycan units. In some embodiments, about 55%, 60%, 65%, 70%, 75%, 80%, 85%, 90%, 95%, or about 97% of the glycan polymer preparation has a DP of between 10, 11, 12, 13, 14, 15, 16, 17, 18, 19 and 20, 21, 22, 23, 24, 25, 26, 27, 28, 29, 30 glycan units. In some embodiments, about 55%, 60%, 65%, 70%, 75%, 80%, 85%, 90%, 95%, or about 97% of the glycan polymer preparation has a DP of between 3, 4, 5, 6, 7, 8, 9, 10 and 20, 21, 22, 23, 24, 25, 26, 27, 28 glycan units. In one embodiment, about 55%, 60%, 65%, 70%, 75%, 80%, 85%, 90%, 95%, or about 97% of the glycan polymer preparation has a DP of at least 2. In one embodiment, about 55%, 60%, 65%, 70%, 75%, 80%, 85%, 90%, 95%, or about 97% of the glycan polymer preparation has a DP of at least 3.

### Average DP

In some embodiments, the glycan polymer preparation has an average degree of polymerization (average DP) of about DP2, DP3, DP4, DP5, DP6, DP7, DP8, or DP9. In some embodiments, the glycan polymer preparation has an average degree of polymerization (average DP) of between about 2 and about 10, between about 2 and about 8, between about 2 and about 6, between about 2 and about 4, between about 3 and about 10, between about 3 and about 8, between about 3 and about 6, or between about 3 and about 4.

In some embodiments, about 55%, 60%, 65%, 70%, 75%, 80%, 85%, 90%, 95%, or about 97% of the glycan polymer preparation has an average degree of polymerization (DP) of about DP5, DP6, DP7, DP8, DP9, DP10, DP11, or DP12. In some embodiments, the average DP of the glycan polymer preparation is between about DP5 and DP10, between about DP6 and DP10, between about DP6 and DP12, between about DP6 and DP14, between about DP8 and DP12, between about DP8 and DP14, between about DP8 and DP16, between about DP10 and DP16 between about DP10 and DP18, between about DP4 and DP18, between about DP6 and DP18, or between about DP8 and DP18.

### Average Molecular weight

In some embodiments, about 55%, 60%, 65%, 70%, 75%, 80%, 85%, 90%, 95%, or about 97% of the glycan polymers of the preparation have an average molecular weight of about 200, 250, 300, 350, 400, 450, 500, 550, 600, 650, 700, 750, 800, 850, 900, 950, 1000, 1050, 1100, 1150, 1200, 1250, 1300, 1350, 1400, 1450, 1500, 1550, 1600, 1650, 1700, 1750, 1800 g/mol and less than 400, 500, 600, 700, 800, 900, 1000, 1100, 1200, 1300, 1400, 1500, 1600, 1700, 1800, 1900, 2000, 2100, 2200, 2300, 2400, 2500, 2600, 2700, 2800, 2900, 3000, 3100, 3200, 3300, 3400, 3500, 3600, 3700, 3800, 3900, 4000, 4100, 4200, 4300, 4400, 4500, 4600, 4700, 4800, 4900, and 5000 g/mol.

### Degree of branching (DB)

In some embodiments, the glycan preparations range in structure from linear to branched. Branched glycans may contain at least one glycan subunit being linked via an alpha or a beta glycosidic bond so as to form a branch. The branching rate or degree of branching (DB) may vary, such that the glycan polymers of a preparation comprise at least 1, at least 2, at least 3, at least 4, at least 5, or at least about 6 branching points in the glycan polymer. In some embodiments, the glycan polymers of the glycan preparation are unbranched (DB=0).

In some embodiments, the glycan preparations (e.g. oligo- or polysaccharides) range in structure from linear to highly branched. Unbranched glycans may contain only alpha linkages or only beta linkages. Unbranched glycans may contain at least one alpha and at least one beta linkage. Branched glycans may contain at least one glycan unit being linked via an alpha or a beta glycosidic bond so as to form a branch. The branching rate or degree of branching (DB) may vary, such that about every 2^{nd} , 3^{rd} , 4^{th} , 5^{th} , 6^{th} , 7^{th} , 8^{th} , 9^{th} , 10^{th} , 15^{th} , 20^{th} , 25^{th} , 30^{th} , 35^{th} , 40^{th} , 45^{th} , 50^{th} , 60^{th} , or 70^{th} unit comprises at least one branching point. For example, animal glycogen contains a branching point approximately every 10 units.

In some embodiments, preparations of glycan polymer are provided, wherein the preparation comprises a mixture of branched glycans, wherein the average degree of branching (DB, branching points per residue) is 0.01. 0.02, 0.03, 0.04, 0.05, 0.06, 0.07, 0.08, 0.09, 0.1, 0.2, 0.3, 0.4, 0.5, or 0.6,. In some embodiments, preparations of glycan polymers are provided, wherein the avarage degree of branching is at least 0.01, 0.05, 0.1, 0.2, 0.3, or at least 0.4. In some embodiments, preparations of glycan polymers are provided, wherein the avarage degree of branching is between about 0.01 and 0.1, 0.01 and 0.2, 0.01 and 0.3, 0.01 and 0.4, 0.01 and 0.5, 0.01 and 0.6. In some embodiments, preparations of glycan polymers are provided, wherein the avarage degree of branching is between about 0.05 and 0.1, 0.05 and 0.2, 0.05 and 0.3, 0.05 and 0.4, 0.05 and 0.5, 0.05 and 0.6. In some embodiments, preparations of glycan polymers are provided, wherein the avarage degree of branching is not 0. In some embodiments, preparations of glycan polymers are provided, wherein the avarage degree of branching is not between at least 0.1 and less than 0.4 or at least 0.2 and less than 0.4. In some embodiments, the preparations of glycan polymers comprise linear glycans. In some embodiments, the preparations of glycan polymers comprise glycans that exhibit a branched or branch-on-branch structure.

In some embodiments, preparations of glycan polymers are provided wherein the avarage degree of branching (DB) is not 0, but is at least 0.01, 0.05, 0.1, or at least 0.2, or ranges between about 0.01 and about 0.2 or between about 0.05 and 0.1.

### Glycosidic bonds and linkages

Linkages between the individual glycan subunits found in preparations of glycan polymers may include alpha 1->2, alpha 1->3, alpha 1->4, alpha 1->5, alpha 1->6, alpha 2-> 1, alpha 2->3, alpha 2->4, alpha 2->6, beta 1->2, beta 1->3, beta 1->4, beta 1->5, beta 1->6, beta 2->1, beta 2->3, beta 2->4, and beta 2->6.

In some embodiments, the glycan polymer preparations comprise only alpha linkages. In some embodiments, the glycan polymers comprise only beta linkages. In some embodiments, the glycan polymers comprise mixtures of alpha and beta linkages.

In some embodiments, the alpha:beta glycosidic bond ratio in a preparation is about 1:1, 2:1, 3:1, 4:1, or 5:1. In some embodiments, the beta:alpha glycosidic bond ratio in a preparation is about 1:1, 2:1, 3:1, 4:1, or 5:1.

In some embodiments, the alpha:beta glycosidic bond ratio in a preparation is about 0.8:1, 0.9:1, 1:1, 1.2:1, 1.5:1, 1.7:1, 2:1, 2.2:1, 2.5:1, 2.7:1, 3:1, 4:1, or about 5:1,.

In some embodiments, the glycan polymers of the glycan polymer preparation comprise both alpha- and beta-glycosidic bonds selected from the group consisting of 1->2 glycosidic bond, a 1->3 glycosidic bond, a 1->4 glycosidic bond, a 1->5 glycosidic bond and a 1->6 glycosidic bond. In some embodiments, the glycan polymer preparation comprises at least two or at least three alpha and beta 1->2 glycosidic bonds, alpha and beta 1->3 glycosidic bonds, alpha and beta 1->4 glycosidic bonds, alpha and beta 1->5 glycosidic bonds, and/or alpha and beta 1->6 glycosidic bonds.

In some embodiments, the glycan polymers of the glycan preparation comprise substantially all alpha- or beta configured glycan subunits, optionally comprising about 1%, 2%, 3%, 4% 5%, 6%, 7%, 8%, 9%, 10%, 11%, 12%, 13%, 14%, 15%, 16%, 17%, 18%, 19%, or 20% of the respective other configuration.

In some embodiments, the preparations of glycan polymers comprise at least 1%, 2%, 3%, 4%, 5%, 6%, 7%, 8%, 9%, 10%, 15%, 20%, 25%, 30%, 35%, 40%, 45%, 50%, 55%, 60%, 65%, 70%, 75%, 80%, 85%, 90%, 95%, 97%, 98%, 99%, at least 99.9% or even 100% glycans with alpha glycosidic bonds. In some embodiments, the preparations of glycan polymers comprise at least 1%, 2%, 3%, 4%, 5%, 6%, 7%, 8%, 9%, 10%, 15%, 20%, 25%, 30%, 35%, 40%, 45%, 50%, 55%, 60%, 65%, 70%, 75%, 80%, 85%, 90%, 95%, 97%, 98%, 99%, at least 99.9% or even 100% glycans with beta glycosidic bonds. In some embodiments, preparations of glycan polymers are provided, wherein at least 10%, 15%, 20%, 25%, 30%, 35%, 40%, 45%, 50%, 55%, 60%, 65%, 70%, 75%, 80%, or at least 85% of glycans with glycosidic bonds that are alpha glycosidic bonds, at least 10%, 15%, 20%, 25%, 30%, 35%, 40%, 45%, 50%, 55%, 60%, 65%, 70%, 75%, 80%, or at least 85% of glycans with glycosidic bonds that are beta glycosidic bonds, and wherein the percentage of alpha and beta glycosidic bonds does not exceed 100%.

In some embodiments, preparations of glycan polymers are provided, wherein at least 1%, 2%, 3%, 4%, 5%, 6%, 7%, 8%, 9%, 10%, 15%, 20%, 25%, 30%, 35%, 40%, 45%, 50%, 55%, 60%, 65%, 70%, 75%, 80%, 85%, 90%, 95%, 97%, 98%, 99%, at least 99.9% or even 100% of glycan glycosidic bonds are one or more of: 1->2 glycosidic bonds, 1->3 glycosidic bonds, 1->4 glycosidic bonds, and 1->6 glycosidic bonds. In some embodiments, preparations of glycan polymers are provided, wherein at least 1%, 2%, 3%, 4%, 5%, 6%, 7%, 8%, 9%, 10%, 15%, at least 20%, or 25% each of glycan glycosidic bonds are 1->2, 1->3, 1->4, and 1->6 glycosidic bonds. Optionally, the preparations of glycan polymers further comprise at least 1%, 2%, 3%, 4%, 5%, 6%, 7%, 8%, 9%, 10%, 15%, 20%, 25%, 30%, 35%, 40%, 45%, 50%, 55%, 60%, 65%, 70%, 75%, 80%, or at least 85% of glycan glycosidic bonds that are selected from the group consisting of : alpha 2-> 1, alpha 2->3, alpha 2->4, alpha 2->6, beta 2->1, beta 2->3, beta 2->4, and beta 2->6, glycosidic bonds.

In some embodiments, the glycan polymers of the glycan preparation comprise at least two glycosidic bonds selected from the group consisting of alpha 1->2 and alpha 1->3, alpha 1->2 and alpha 1->4, alpha 1->2 and alpha 1->6, alpha 1->2 and beta 1->2, alpha 1->2 and beta 1->3, alpha 1->2 and beta 1->4, alpha 1->2 and beta 1->6, alpha 1->3 and alpha 1->4, alpha 1->3 and alpha 1->6, alpha 1->3 and beta 1->2, alpha 1->3 and beta 1->3, alpha 1->3 and beta 1->4, alpha 1->3 and beta 1->6, alpha 1->4 and alpha 1->6, alpha 1->4 and beta 1->2, alpha 1->4 and beta 1->3, alpha 1->4 and beta 1->4, alpha 1->4 and beta 1->6, alpha 1->6 and beta 1->2, alpha 1->6 and beta 1->3, alpha 1->6 and beta 1->4, alpha 1->6 and beta 1->6, beta 1->2 and beta 1->3, beta 1->2 and beta 1->4, beta 1->2 and beta 1->6, beta 1->3 and beta 1->4, beta 1->3 and beta 1->6, and beta 1->4 and beta 1->6.

### L- and D-forms

In some embodiments, preparations of glycan polymers are provided, wherein at least one glycan subunit is a sugar in L-form. In some embodiments, preparations of glycans are provided, wherein at least one glycan subunit is a sugar in D-form. In some embodiments, preparations of glycans are provided, wherein the glycan subunits are sugars in L- or D-form as they naturally occur or are more common (e.g. D-glucose, D-xylose, L-arabinose).

In some embodiments, the preparation of glycan polymers (e.g. oligosaccharides and polysaccharides) comprises a desired mixture of L- and D-forms of glycan subunits, e.g. of a desired ratio, such as: 1:1, 1:2, 1:3, 1:4, 1:5 L- to D-forms or D- to L-forms.

In some embodiments, the preparation of glycan polymers comprises a desired mixture of L- and D-forms of glycan units, e.g. of a desired ratio, such as: 1:1, 1:2, 1:3, 1:4, 1:5, 1:6, 1:7, 1:8, 1:9, 1:10, 1:12, 1:14, 1:16, 1:18, 1:20, 1:25, 1:30, 1:35, 1:40, 1:45, 1:50, 1:55, 1:60, 1:65, 1:70, 1:75, 1:80, 1:85, 1:90, 1:100, 1:150 L- to D-forms or D- to L-forms.

In some embodiments, the preparation of glycan polymers comprises glycans with substantially all L- or D-forms of glycan subunits, optionally comprising about 1%, 2%, 3%, 4% 5%, 6%, 7%, 8%, 9%, 10%, 11%, 12%, 13%, 14%, 15%, 16%, 17%, 18%, 19%, or 20% of the respective other form.

### Glycan unit content

In some embodiments, preparations of glycan polymers are provided, wherein at least one glycan subunit is a tetrose, a pentose, a hexose, or a heptose. Optionally, the glycan subunits involved in the formation of the glycans of the glycan polymer preparation are varied. Examples of monosaccharide glycan subunits include hexoses, such as glucose, galactose, and fructose, and pentoses, such as xylose. Monosaccharides generally have the chemical formula: Cₓ(H₂O)_{y}, where conventionally x ≥ 3. Monosaccharides can be classified by the number x of carbon atoms they contain, for example: those (2) triose (3) tetrose (4), pentose (5), hexose (6), and heptose (7). The monosaccharide glycan subunits may exist in an acyclic (open-chain) form. Open-chain monosaccharides with same molecular graph may exist as two or more stereoisomers. The monosaccharides may also exist in a cyclic form through a nucleophilic addition reaction between the carbonyl group and one of the hydroxyls of the same molecule. The reaction creates a ring of carbon atoms closed by one bridging oxygen atom. In these cyclic forms, the ring usually has 5 (furanoses) or 6 atoms (pyranoses).

In some embodiments, the preparation of glycan polymers comprises a desired mixture of different monosaccharide glycan subunits, such as a mixture of a those (2), a triose (3), tetrose (4), pentose (5), hexose (6), or heptose (7). In some embodiments, the glycan polymers of the glycan polymer preparation comprise a desired mixture of a pentose (5) and a hexose (6).

In some embodiments, the preparation of glycan polymers comprises a desired mixture of two, three, four or five different glycan subunits, such as a mixture of, e.g., i) one or more glycan subunits selected from monosaccharides, selected from glucose, a galactose, an arabinose, a mannose, a fructose, a xylose, a fucose, and a rhamnose; ii) one or more glycan subunits selected from disaccharides selected from acarviosin, n-acetyllactosamine, allolactose, cellobiose, chitobiose, glactose-alpha-1,3-galactose, gentiobiose, isomalt, isomaltose, isomaltulose, kojibiose, lactitol, lactobionic acid, lactose, lactulose, laminaribiose, maltitol, maltose, mannobiose, melibiose, melibiulose, neohesperidose, nigerose, robinose, rutinose, sambubiose, sophorose, sucralose, sucrose, sucrose acetate isobutyrate, sucrose octaacetate, trehalose, turanose, vicianose, and xylobiose; iii) one or more glycan subunits selected from amino sugars selected from acarbose, N-acetylemannosamine, N-acetylmuramic acid, N-acetylnueraminic acid, N-acetyletalosaminuronic acid, arabinopyranosyl-N-methyl-N-nitrosourea, D-fructose-L-histidine, N-glycolyneuraminic acid, ketosamine, kidamycin, mannosamine, 1B-methylseleno-N-acetyl-D-galactosamine, muramic acid, muramyl dipeptide, phosphoribosylamine, PUGNAc, sialyl-Lewis A, sialyl-Lewis X, validamycin, voglibose, N-acetylgalactosamine, N-acetylglucosamine, aspartylglucosamine, bacillithiol, daunosamine, desosamine, fructosamine, galactosamine, glucosamine, meglumine, and perosamine; iv) one or more glycan subunits selected from deoxy sugars selected from 1-5-ahydroglucitol, cladinose, colitose, 2-deoxy-D-glucose, 3-deoxyglucasone, deoxyribose, dideoxynucleotide, digitalose, fludeooxyglucose, sarmentose, and sulfoquinovose; v) one or more glycan subunits selected from imino sugars selected from castanospermine, 1-deoxynojirimycin, iminosugar, miglitol, miglustat, and swainsonine; one or more glycan subunits selected from sugar acids selected from N-acetylneuraminic acid, N-acetyltalosamnuronic acid, aldaric acid, aldonic acid, 3-deoxy-D-manno-oct-2-ulosonic acid, glucuronic acid, glucosaminuronic acid, glyceric acid, N-glycolylneuraminic acid, iduronic acid, isosaccharinic acid, pangamic acid, sialic acid, threonic acid, ulosonic acid, uronic acid, xylonic acid, gluconic acid, ascorbic acid, ketodeoxyoctulosonic acid, galacturonic acid, galactosaminuronic acid, mannuronic acid, mannosaminuronic acid, tartaric acid, mucic acid, saccharic acid, lactic acid, oxalic acid, succinic acid, hexanoic acid, fumaric acid, maleic acid, butyric acid, citric acid, glucosaminic acid, malic acid, succinamic acid, sebacic acid, and capric acid; vi) one or more glycan subunits selected from short-chain fatty acids selected from formic acid, acetic acid, propionic acid, butryic acid, isobutyric acid, valeric acid, and isovaleric acid; and vii) one or more glycan subunits selected from sugar alcohols selected from methanol, ethylene glycol, glycerol, erythritol, threitol, arabitol, ribitol, xylitol, mannitol, sorbitol, galactitol, iditol, volemitol, fucitol, inositol, maltotritol, maltotetraitol, and polyglycitol.

Exemplary glycans are described by a three-letter code representing the monomeric sugar component followed by a number out of one hundred reflecting the percentage of the material that monomer constitutes. Thus, 'glu100' is ascribed to a glycan generated from a 100% D-glucose (glycan unit) input and 'glu50gal50' is ascribed to a glycan generated from 50% D-glucose and 50% D-galactose (glycan units) input or, alternatively from a lactose dimer (glycan unit) input. As used herein: xyl = D-xylose; ara = L-arabinose; gal = D-galactose; glu = D-glucose; rha = L-rhamnose; fuc = L-fucose; man = D-mannose; sor = D-sorbitol; gly = D-glycerol; neu = NAc-neuraminic acid.

In some embodiments, the preparation of glycan polymers comprises one glycan unit A selected from i) to vii) above, wherein glycan unit A comprises 100% of the glycan unit input. For example, in some embodiments, the glycan polymer preparation is selected from the homoglycans xyl100, rha100, ara100, gal100, glu100, and man100. In some embodiments, the glycan polymer preparation is selected from the homo-glycans fuc100 and fru100.

In some embodiments, the preparation of glycan polymers comprises a mixture of two glycan units A and B selected independently from i) to vii) above, wherein A and B may be selected from the same or a different group i) to vii) and wherein A and B may be selected in any desired ratio (e.g. anywhere from 1-99% A and 99-1% B, not exceeding 100%).

For example, in some embodiments, the glycan polymer preparation is selected from the hetero-glycans ara50gal50, ara50gal50, xyl75gal25, ara80xyl20, ara60xyl40, ara50xyl50, glu80man20, glu60man40, man80glu20, man60glu40, xyl75ara25, gal75xyl25, Man80gal20, gal75xyl25, Man66gal33, Man75gal25, glu80gal20, glu60gal40, glu40gal60, glu20gal80, gal80man20, gal60man40, gal40man60, glu80xyl20, glu60xyl40, glu40xyl60, glu20xyl80, glu80ara20, glu60ara40, glu40ara60, glu20ara80, ga180xyl20, gal60xyl40, gal40xyl60, gal20xyl80, gal80ara20, gal60ara40, gal40ara60, gal20ara80, man80xyl20, man60xyl40, man40xyl60, man20xyl80, man80ara20, man60ara40, man40ara60, man20ara80, xyl80ara20, xyl60ara40, glu50gal50, and man62glu38.

In some embodiments, the preparation of glycan polymers comprises a mixture of three glycan units A, B and C selected independently from i) to vii) above, wherein A, B and C may be selected from the same or a different group i) to vii) and wherein A, B and C may be selected in any desired ratio (e.g. anywhere from 1-99% A, 1-99% B, 1-99% C, not exceeding 100%).

For example, in some embodiments, the glycan polymer preparation is selected from the hetero-glycans xyl75glu12gal12, xyl33glu33gal33, xyl75glu12gal12, glu33gal33fuc33, glu33gal33nman33, glu33gal33xyl33, glu33gal33ara33, gal33man33xyl33, gal33man33ara33, man52glu29gal19, Glu33Man33Xyl33, Glu33Man33Ara33, Glu33Xyl33Ara33, Gal33Man33Xyl33, Gal33Man33Ara33, Gal33Xyl33Ara33, Man33Xyl33Ara33, Glu90Gal5Man5, Glu80Gal10Man10, Glu60Gal20Man20, Glu40Gal30Man30, Glu20Gal40Man40, Glu10Gal45Man45, Glu5Gal90Man5, Glu10Gal80Man10, Glu20Gal60Man20, Glu30Gal40Man30, Glu40Gal20Man40, Glu45Gal10Man45, Glu5Gal5Man90, Glu10Gal10Man80, Glu20Gal20Man60, Glu30Gal30Man40, Glu40Gal40Man20, and Glu45Gal45Man10.

In some embodiments, the preparation of glycan polymers comprises a mixture of four glycan units A, B, C and D selected independently from i) to vii) above, wherein A, B, C and D may be selected from the same or a different group i) to vii) and wherein A, B, C and D may be selected in any desired ratio (e.g. anywhere from 1-99% A, 1-99% B, 1-99% C, 1-99% D, not exceeding 100%).

In some embodiments, the preparation of glycan polymers comprises a mixture of five glycan units A, B, C, D and E selected independently from i) to vii) above, wherein A, B, C, D and E may be selected from the same or a different group i) to vii) and wherein A, B, C, D and E may be selected in any desired ratio (e.g. anywhere from 1-99% A, 1-99% B, 1-99% C, 1-99% D, 1-99% E, not exceeding 100%).

In some embodiments, preparationsof glycan polymers are provided, wherein at least one glycan subunit is selected from the group consisting of a glucose, a galactose, an arabinose, a mannose, a fructose, a xylose, a fucose, and a rhamnose.

In some embodiments, the preparation of glycan polymers comprises a desired mixture of two different monosaccharide glycan subunits, such as a mixture of, e.g., glucose and galactose, glucose and arabinose, glucose and mannose, glucose and fructose, glucose and xylose, glucose and fucose, glucose and rhamnose, galactose and arabinose, galactose and mannose, galactose and fructose, galactose and xylose, galactose and fucose, and galactose and rhamnose, arabinose and mannose, arabinose and fructose, arabinose and xylose, arabinose and fucose, and arabinose and rhamnose, mannose and fructose, mannose and xylose, mannose and fucose, and mannose and rhamnose, fructose and xylose, fructose and fucose, and fructose and rhamnose, xylose and fucose, xylose and rhamnose, and fucose and rhamnose, e.g. a in a ratio of 1:1, 1:2, 1:3, 1:4, or 1:5 or the reverse ratio thereof, or a in a ratio of 1:1, 1:2, 1:3, 1:4, 1:5, 1:6, 1:7, 1:8, 1:9, 1:10, 1:12, 1:14, 1:16, 1:18, 1:20, 1:25, 1:30, 1:35, 1:40, 1:45, 1:50, 1:55, 1:60, 1:65, 1:70, 1:75, 1:80, 1:85, 1:90, or 1:100 or the reverse ratio thereof.

In some embodiments, the preparation of glycan polymers comprises a desired mixture of three different monosaccharide glycan subunits, such as a mixture of, e.g. for glucose-containing glycan preparations, glucose, galactose and arabinose; glucose, galactose and mannose; glucose, galactose and fructose; glucose, galactose and xylose; glucose, galactose and fucose, glucose, galactose and rhamnose; glucose, arabinose, and mannose; glucose, arabinose and fructose; glucose, arabinose and xylose; glucose, arabinose and fucose; glucose, arabinose and rhamnose; glucose, mannose and fructose; glucose, mannose and xylose; glucose, mannose and fucose; glucose, mannose rhamnose; glucose, fructose and xylose; glucose, fructose and fucose; glucose, fructose and rhamnose; glucose, fucose and rhamnose, e.g. a in a ratio of 1:1:1, 1:2:1, 1:3:1, 1:4:1, 1:5:1, 1:1:2, 1:2:2, 1:3:2, 1:4:2, 1:1:3, 1:2:3, 1:3:3, 1:1:4, 1:2:4, 1:1:5, 1:2:5, , etc., or . a in a ratio of 1:1:1, 1:2:1, 1:3:1, 1:4:1, 1:5:1, 1:6:1, 1:7:1, 1:8:1, 1:9:1, 1:10:1, 1:12:1, 1:14:1, 1:16:1, 1:18:1, 1:20:1, 1:1:2, 1:2:2, 1:3:2, 1:4:2, 1:5:2, 1:6:2, 1:7:2, 1:8:2, 1:9:2, 1:10:2, 1:1:3, 1:2:3, 1:3:3, 1:4:3, 1:5:3, 1:6:3, 1:7:3, 1:8:3, 1:9:3, 1:10:3, 1:1:4, 1:2:4, 1:3:4, 1:4:4, 1:5:4, 1:6:4, 1:7:4, 1:8:4, 1:9:4, 1:10:4, 1:1:5, 1:2:5, 1:3:5, 1:4:5, 1:5:5, 1:6:5, 1:7:5, 1:8:5, 1:9:5, 1:10:5, etc.

In some embodiments, the preparation of glycan polymers does not comprise N-acetylgalactosamine or N-acetylglucosamine. In some embodiments, the preparation of glycans does not comprise sialic acid. In some embodiments, the preparation of glycan polymers does not comprise a lipid and fatty acid. In some embodiments, the preparation of glycan polymers does not comprise an amino acid.

### Furanose: Pyranose

In some embodiments, preparations of glycan polymers are provided, wherein at least one glycan subunit is a furanose sugar. In some embodiments, preparations of glycans are provided, wherein at least one glycan subunit is a pyranose sugar. In some embodiments, glycan polymers comprise mixtures of furanose and pyranose sugars. In some embodiments, the furanose: pyranose sugar ratio in a preparation is about 0.1:1, 0.2:1, 0.3:1, 0.4:1, 0.5:1, 0.6:1, 0.7:1, 0.8:1, 0.9:1, 1:1, 1.2:1, 1.5:1, 1.7:1, 2:1, 2.2:1, 2.5:1, 2.7:1, 3:1, 4:1, 5:1, or about 6:1 or the furanose: pyranose sugar ratio in a preparation is about 7:1, 8:1, 9:1, or about 10:1..

In some embodiments, the preparation of glycan polymers comprises substantially all furanose or pyranose sugar, optionally comprising 1%, 2%, 3%, 4% 5%, 6%, 7%, 8%, 9%, 10%, 11%, 12%, 13%, 14%, 15%, 16%, 17%, 18%, 19%, or 20% of the respective other sugar.

In some embodiments, the preparation of glycan polymers comprises substantially all pyranose sugar and no more than about 0.1%, 02%, 0.5%, 1%, 2%, 3%, 4%, or no more than 5% of glycan units in the preparation in furanose form. In some embodiments, no more than 3%, 2% or no more than 1% of monomeric glycan units in the preparation are in furanose form.

### Salts

In some embodiments, the preparation of glycan polymers comprises a glycan subunit or plurality of glycan subunits present in a salt form (e.g., a pharmaceutically acceptable salt form), such as, e.g., a hydrochlorate, hydroiodate, hydrobromate, phosphate, sulfate, methanesulfate, acetate, formate, tartrate, malate, citrate, succinate, lactate, gluconate, pyruvate, fumarate, propionate, aspartate, glutamate, benzoate, ascorbate salt.

### Derivatization

If desired, the monosaccharide or oligosaccharide glycan subunits of the glycans are further substituted or derivatized, e.g., hydroxyl groups can be etherified or esterified. For example, the glycans (e.g. oligo- or polysaccharide) can contain modified saccharide units, such as 2 '-deoxyribose wherein a hydroxyl group is removed, 2'-fluororibose wherein a hydroxyl group is replace with a fluorine, or N- acetylglucosamine, a nitrogen-containing form of glucose (e.g., 2'-fluororibose, deoxyribose, and hexose). The degree of substitution (DS, average number of hydroxyl groups per glycosyl unit) can be 1, 2, or 3, or another suitable DS. In some embodiments, 1%, 2%, 3%, 4% 5%, 6%, 7%, 8%, 9%, 10%, 11%, 12%, 13%, 14%, 15%, 16%, 17%, 18%, 19%, 20%, 25%, 30%, 35%, 40%, 45%, 50%, 55%, 60%, 65%, 70%, 80%, 85%, 90%, 95%, 96%, 97%, 98%, 99%, or 100% of glycan subunits are substituted or derivatized. In some embodiments, the degree of substitution varies between subunits, e.g., a certain percentage is not derivatized, exhibits a DS of 1, exhibits a DS of 2, or exhibits a DS of 3. Any desired mixture can be generated, e.g. 0-99% of subunits are not derivatized, 0-99% of subunits exhibit a DS of 1, 0-99% of subunits exhibit a DS of 2, and 0-99% of subunits exhibit a DS of 3, with the total making up 100%. The degree of substitution can be controlled by adjusting the average number of moles of substituent added to a glycosyl moiety (molar substitution (MS)). The distribution of substituents along the length of the glycan oligo- or polysaccharide chain can be controlled by adjusting the reaction conditions, reagent type, and extent of substitution. In some embodiments, the monomeric subunits are substituted with one or more of an acetate ester, sulfate half-ester, phosphate ester, or a pyruvyl cyclic acetal group.

### Solubility

In some embodiments, the glycan polymers in a preparation are highly soluble. In some embodiments, glycan polymer preparations can be concentrated to at least to 55 Brix, 65 Brix, 60 Brix, 65 Brix, 70 Brix, 75 Brix, 80 Brix, or at least 85 Brix without obvious solidification or crystallization at 23 °C (final solubility limit). In some embodiments, glycan polymer preparations are concentrated to at least about 0.5 g/ml, 1 g/ml, 1.5 g/ml, 2 g/ml, 2.5 g/ml, 3 g/ml, 3.5 g/ml or at least 4 g/ml without obvious solidification or crystallization at 23 °C (final solubility limit).

In some embodiments, the glycan polymer preparations (e.g. oligosaccharides) are branched, e.g. have an average DB of at least 0.01, 0.05, or 0.1 and has a final solubility limit in water of at least about 70 Brix, 75 Brix, 80 Brix, or at least about 85 Brix at 23 °C or is at least about 1 g/ml, 2 g/ml or at least about 3 g/ml.

In some embodiments, the preparation of glycan polymers has a final solubility limit of at least 0.001 g/L, 0.005 g/L, 0.01 g/L, 0.05 g/L, 0.1 g/L, 0.2 g/L, 0.3 g/L, 0.4 g/L, 0.5 g/L, 0.6 g/L, 0.7 g/L, 0.8 g/L, 0.9 g/L, 1g/L, 5 g/L, 10 g/L, 20 g/L, 30 g/L, 40 g/L, 50 g/L, 100 g/L, 200 g/L, 300 g/L, 400 g/L, 500 g/L, 600 g/L, 700 g/L, 800 g/L, 900 g/L, 1000 g/L in deionized water, or in a suitable buffer such as, e.g., phosphate-buffered saline, pH 7.4 or similar physiological pH) and at 20 °C. In some embodiments, the preparation of glycan polymers is greater than 50%, greater than 60%, greater than 70%, greater than 80%, greater than 90%, greater than 95%, greater than 96%, greater than 97%, greater than 98%, greater than 99%, or greater than 99.5% soluble with no precipitation observed at a concentration of greater than 0.001 g/L, 0.005 g/L, 0.01 g/L, 0.05 g/L, 0.1 g/L, 0.2 g/L, 0.3 g/L, 0.4 g/L, 0.5 g/L, 0.6 g/L, 0.7 g/L, 0.8 g/L, 0.9 g/L, 1g/L, 5 g/L, 10 g/L, 20 g/L, 30 g/L, 40 g/L, 50 g/L, 100 g/L, 200 g/L, 300 g/L, 400 g/L, 500 g/L, 600 g/L, 700 g/L, 800 g/L, 900 g/L, 1000 g/L in deionized water, or in a suitable buffer such as, e.g., phosphate-buffered saline, pH 7.4 or similar physiological pH) and at 20 °C.

### Sweetness

In some embodiments, the preparation of glycan polymers has a desired degree of sweetness. For example, sucrose (table sugar) is the prototype of a sweet substance. Sucrose in solution has a sweetness perception rating of 1, and other substances are rated relative to this (e.g., fructose, is rated at 1.7 times the sweetness of sucrose). In some embodiments, the sweetness of the preparation of glycan polymers ranges from 0.1 to 500,000 relative to sucrose. In some embodiments, the relative sweetness is 0.1, 0.2, 0.3, 0.4, 0.5, 0.6, 0.7, 0.8, 0.9, 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 80, 90, 100, 150, 200, 250, 300, 350, 400, 450, 500, 550, 600, 650, 700, 750, 800, 850, 900, 950, 1000, 2000, 3000, 4000, 5000, 6000, 7000, 8000, 9000, 10000, 25000, 50000, 75000, 100000, 150000, 200000, 250000, 300000, 350000, 40000, 450000, 500000, or more than 500,000 relative to sucrose (with sucrose scored as one). In some embodiments, the preparation of glycan polymers is mildly sweet, or both sweet and bitter.

In some embodiments, the preparation of glycan polymers, e.g. a preparation that is substantially DP2+ or DP3+ (e.g. at least 80%, 90%, or at least 95%, or a fractionated preparation of DP2+ or DP3+), is substantially imperceptible as sweet and the relative sweetness is about 0, 0.0001, 0.001, 0.005, 0.01, 0.05, 0.1, 0.2, 0.3, 0.4, 0.5, 0.6, 0.7, or about 0.8 relative to sucrose (with sucrose scored as one).

Glycan polymer preparations can be characterized by any suitable methods including those described in WO2016/122889, WO2016/172657, WO 2016/007778, and WO2016/172658.

In embodiments, glycan compositions and glycan preparations may comprise one or more (e.g., two, three, four, five, six or more) of the following properties (including bulk properties):
a) the glycan polymer comprising at least one of glucose, galactose, arabinose, mannose, fructose, xylose, fucose, or rhamnose,
b) a high degree of polymerization (DP), e.g. at least about 50%, 60%, 70%, 80%, 90%, 95%, 98%, 99% of polymers range in DP from about 30-100,000, about 30-50,000, about 30-10,000, about 30-5,000, about 30-1,000, about 30-500, about 30-200, about 30-100, or about 3-50,
c) a low degree of polymerization, e.g. at least about 50%, 60%, 70%, 80%, 90%, 95%, 98%, 99% of polymers range in DP from about 2-29, about 2-25, about 2-20, about 2-15, about 2-10, about 2-8, about 2-6, about 3-8, or about 4-8,
d) a high viscosity e.g., ranging from about 100-10,000 mPas, 100-5,000 mPas, 100-1,000 mPas, 100-500 mPas, in water at 20° C,
e) a low viscosity, e.g., ranging from about 1-99 mPas, 1-50 mPas, 1-10 mPas, 1-5 mPas, 25-75 mPas, or 10-50 mPas, in water at 20° C,
f) a high final solubility limit in water of at least about 60, 70, or at least about 75 Brix at 23° C,
g) a low final solubility limit in water of no more than 5, 10, 20, 30, 40, 50 Brix at 23° C, or insolubility (e.g. no more than 0.1 Brix)
h) a caloric value of about 0.1 cal/g to 3 cal/g, 0.1 cal/g to 2 cal/g, 0.1 cal/g to 1.5 cal/g, 0.1 cal/g to 1 cal/g, 0.1 cal/g to 0.5 cal/g,
i) a non-caloric value (e.g., about 0 cal/g to 0.09 cal/g, 0 cal/g to 0.05 cal/g or about 0 cal/g to 0.01 cal/g
j) a low degree of digestibility, wherein no more than about 30%, 20%, 10%, 5%, 1%, 0.5% of the glycan polymer is digestible by a human glycosidase (e.g., alpha-amylase)
k) a high degree of digestibility, wherein at least 50%, 60%, 70%, 80%, 90%, 95% of the glycan polymer is digestible by a human glycosidase (e.g., alpha-amylase)
l) a low degree of fermentability, wherein no more than about 40%, 30%, 20%, 10%, 5%, 1%, 0.5% of the glycan polymer is fermentable by a human (e.g., colonic) microbial community or a single bacterial strain,
m) a high degree of fermentability, wherein at least 50%, 60%, 70%, 80%, 90%, 95% of the glycan polymer is fermentable by a human (e.g. colonic) microbial community or a single bacterial strain,
n) a slow rate of fermentation, wherein no more than about 0.5%, 1%, 2%, 5%, 10%, or 15% of the glycan polymer is fermented by a human (e.g., colonic) microbial community or a single bacterial strain in 12-24 hours,
o) a fast rate of fermentation, wherein at least about 15%, 20%, 30%, 40%, or 50% of the glycan polymer is fermented by a human (e.g. colonic) microbial community or a single bacterial strain in 12-24 hours,
p) a high degree of gastrointestinal tolerance (e.g., is tolerated by a subject in high daily doses, e.g. at least about 5 g/day, 10 g/day, 15 g/day, 20 g/day, 30 g/day, 40 g/day, 50 g/day, 60 g/day, or 70 g/day without substantial side effects, e.g. such as bloating, excess gas, GI discomfort, diarrhea or constipation).

Glycan compositions described herein can comprise one or more sugars and/or sugar alcohols. Compositions can comprise a simple sugar (such as a monosaccharide, a disaccharide, a trisaccharide, a tetrasacchaaride or a pentasaccharide), a sugar alcohol, or any combination thereof. In some embodiments, composition comprises a metabolizable sugar or metabolizable sugar alcohol, wherein the sugar or sugar alcohol is metabolized in the gastrointestinal tract of the host. The sugars, and sugar alcohols disclosed in WO 2016/172658, are suitable for use in methods and compositions described herein. In embodiments, a composition described herein, e.g., glycan composition described herein, can comprise polyphenols, fatty acids (e.g., short chain fatty acids), amino acids, peptides, and micronutrients, e.g., as described herein and in WO 2016/172658 and in Table 7.

**Table 7. Exemplary constituents of glycan compositions: Sugars, Sugar Alcohols, Amino Acids, Vitamins, Minerals, Fatty Acids, and Polyphenols**

| Compound | Examples |
|---|---|
| Sugar | glucose, galactose, N-acetylglucosamine, N-acetylgalactosamine, fructose, fucose, mannose, N-acetylmannosamine, glucuronic acid, N-acetylglucuronic acid, galactosuronic acid, N-acetylgalactosuronic acid, xylose, arabinose, rhamnose, ribose, sucrose, sorbose, lactose, maltose, lactulose, tagatose, kojibiose, nigerose, isomaltose, trehalose, sophorose, laminaribiose, gentiobiose, turanose, maltulose, palatinose, gentiobiulose, mannobiose, melibiulose, rutinulose, xvlobiose |
| Sugar Alcohol | sorbitol, mannitol, lactitol, erythritol, glycerol, arabitol, maltitol, xylitol, ribitol, threitol, galactitol, fucitol, iditol, inositol |
| Amino Acid | alanine, arginine, asparagine, aspartic acid, cysteine, glutamic acid, glutamine, glycine, histidine, isoleucine, leucine, lysine, methionine, phenylalanine, proline, serine, threonine, tryptophan, tyrosine, valine |
| Vitamin | pantothenate, thiamine, riboflavin, niacin, pyridoxol, biotin, folate, 4-aminobenzoate, cobinamide, phenyolyl cobamide, 5-methylbenzimidazolyl cobamide, cobalamin, pyridoxine, pyridoxamine, ergadenylic acid, cyanocobalamin, choline, retinol, a carotenoid, zeaxanthin |
| Element/Mineral | chloride, sodium, calcium, magnesium, nitrogen, potassium, manganese, iron, zinc, nickel, copper, cobalt |
| Fatty Acid | acetic acid, propionic acid, butryic acid, isobutyric acid, valeric acid, isovaleric acid, hexanoic acid, octanoic acid, formic acid, oxalic acid, glyoxylic acid, glycolic acid, acrylic acid, malonic acid, pyruvic acid, lactic acid, succinic acid, acetoacetic acid, fumaric acid, maleic acid, oxaloacetic acid, malic acid, tartaric acid, crotonic acid, glutaric acid, alpha-ketoglutaric acid, caproic acid, adipic acid, citric acid, aconitic acid, isocitric acid, sorbic acid, enanthic acid, pimelic acid, benzoic acid, salicylic acid, caprylic acid, phthalic acid, pelargonic acid, trimesic acid, cinnamic acid, capric acid, sebacic acid, stearic acid, oleic acid, linoleic acid, α-linolenic acid, γ-linolenic acid, stearidonic acid |
| Polyphenol | Anthocyanins, Chalcones, Dihydro-chalcones, Dihydro-flavonols, Flavanols, Flavanones, Flavones, Flavonols, Isoflavonoids, Lignans, Non-phenolic metabolites, Alkylmethoxy-phenols, Alkylphenols,, Betacyanins, Capsaicinoids, Curcuminoids, Dihydro-capsaicins, Furano-coumarins, Hydroxy-benzaldehydes, Hydroxybenzoketones, Hydroxycinnam-aldehydes, Hydroxy-coumarins, Hydroxyphenyl-alcohols, Hydroxy-phenylpropenes, Methoxyphenols, Naphtoquinones, Phenolic terpenes, Tyrosols, Hydroxybenzoic acids, Hydroxy-cinnamic acids, Hydroxyphenylacetic acids, Hydroxy-phenylpropanoic acids, Hydroxyphenylpentanoic acids, Stilbenes, catechin, ellagitannin, isoflavone, flavonol, flavanone, anthocyanin, lignin, alkylmethoxyphenol, alkylphenol, curcuminoid, furanocoumarin, hydroxybenzaldehyde, hydroxybenzoketone, hydroxycinnamaldehyde, hydroxycoumarin, hydroxyphenylpropene, methoxyphenol, naphtoquinone, phenolic terpenes, tyrosols |

### Probiotics

In embodiments, a composition described herein, e.g., glycan composition described herein, can comprise commensal or probiotic bacterial taxa, e.g., those described in Tables 4-6, and bacteria that are generally recognized as safe (GRAS) or known commensal or probiotic microbes. In embodiments, a composition described herein, e.g., glycan composition described herein, can comprise a bacterial taxa described in Tables 1-3. In some embodiments, probiotic or commensal bacterial taxa (or preparations thereof) may be administered to a subject receiving the glycan preparations.

In some embodiments, the composition further comprises at least about 1% (w/w) of a probiotic or commensal bacterium or a combination thereof (e.g., at least about 2%, about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, about 96%, about 97%, about 98%, about 99%, or more).

Probiotic microorganisms may also be included in the glycan compositions, or used in combination with a glycan composition described herein. A probiotic microorganism is also referred to a probiotic. Probiotics can include the metabolites generated by the probiotic microorganisms during fermentation. These metabolites may be released to the medium of fermentation, e.g., into a host organism (e.g., subject), or they may be stored within the microorganism. Probiotic microorganism includes bacteria, bacterial homogenates, bacterial proteins, bacterial extracts, bacterial ferment supernatants and combinations thereof, which perform beneficial functions to the host animal, e.g., when given at a therapeutic dose.

Useful probiotic microorganisms include at least one lactic acid and/or acetic acid and/or propionic acid producing bacteria, e.g., microbes that produce lactic acid and/or acetic acid and/or propionic acid by decomposing carbohydrates such as glucose and lactose. Preferably, the probiotic microorganism is a lactic acid bacteria. In embodiments, lactic acid bacteria include Lactobacillus, Leuconostoc, Pediococcus, Streptococcus, and Bifidobacterium. Suitable probioitc microorganisms can also include other microorganisms which beneficially affect a host by improving the hosts intestinal microbial balance, such as, but not limited to yeasts such as Saccharomyces, Debaromyces, Candida, Pichia and Torulopsis, molds such as Aspergillus, Rhizopus, Mucor, and Penicillium and Torulopsis, and other bacteria such as but not limited to the genera Bacteriodes, Clostridium, Fusobacterium, Melissococcus, Propionibacterium, Enterococcus, Lactococcus, Staphylococcus, Peptostreptococcus, Bacillus, Pediococcus, Micrococcus, Leuconostoc, Weissella, Aerococcus, and Oenococcus, and combinations thereof.

Non-limiting examples of lactic acid bacteria useful in the disclosure herein include strains of Streptococcus lactis, Streptococcus cremoris, Streptococcus diacetylactis, Streptococcus thermophilus, Lactobacillus bulgaricus, Lactobacillus acidophilus, Lactobacillus helveticus, Lactobacillus bifidus, Lactobacillus casei, Lactobacillus lactis, Lactobacillus plantarum, Lactobacillus rhamnosus, Lactobacillus delbruekii, Lactobacillus thermophilus, Lactobacillus fermentii, Lactobacillus salivarius, Lactobacillus paracasei, Lactobacillus brevis, Bifidobacterium longum, Bifidobacterium infantis, Bifidobacterium bifidum, Bifidobcterium animalis, Bifidobcterium lactis, Bifidobcterium breve, Bifidobcterium adolescentis, and Pediococcus cerevisiae and combinations thereof, in particular Lactobacillus, Bifidobacterium, and combinations thereof

Probiotic microorganisms which are particularly useful in the present disclosure include those which (for human administration) are of human origin (or of the origin of the mammal to which the probiotic microorganism is being administered), are non-pathogenic to the host, resist technological processes (i.e. can remain viable and active during processing and in delivery vehicles), are resistant to gastric acidity and bile toxicity, adhere to gut epithelial tissue, have the ability to colonize the gastrointestinal tract, produce antimicrobial substances, modulate immune response in the host, and influence metabolic activity (e.g. cholesterol assimilation, lactase activity, vitamin production).

The probiotic microorganism can be included in the glycan preparations as a single strain or a combination of multiple strains, wherein the total number of bacteria in a dose of probiotic microorganism is from about 1 × 10³ to about 1 × 10¹⁴, or from about 1 × 10 to about 1 × 10¹², or from about 1 × 10⁷ to about 1 × 10¹¹ CFU per dose.

The probiotic microorganisms can be incorporated into the glycan preparations while the probiotic microorganism is alive but in a state of "suspended animation" or somnolence. Once freeze-dried, the viable cultures(s) of probiotic microorganism are handled so as to minimize exposure to moisture that would reanimate the cultures because, once reanimated, the cultures can experience high rates of morbidity unless soon cultured in a high moisture environment or medium. Additionally, the cultures are handled to reduce possible exposure to high temperatures (particularly in the presence of moisture) to reduce morbidity.

The probiotic microorganisms can be used in a powdered, dry form. The probiotic microorganisms can also be administered in the glycan preparation or in a separate glycan preparation, administered at the same time or different time as the glycan preparations.

Examples of probiotics include, but are not limited to, those that acidify the colon such as those from the genera Lactobacillus or Bifidobacterium, which are thought to maintain a healthy balance of intestinal microbiota by producing organic acids (lactic & acetic acids), hydrogen peroxide, and bacteriocins which are documents to inhibit enteric pathogens.

Other Lactobacillus bacteria which can be employed include, but are not limited to, L. crispatus, L. casei, L. rhamnosus, L. reuteri, L. fermentum, L. plantarum, L. sporogenes, and L. bulgaricus. Other probiotic bacteria suitable for the glycan compositions include Bifidobacterium lactis, B. animalis, B. bifidum, B. longum, B. adolescentis, and B. infantis.

In embodiments, a commensal bacterial taxa that can be used in and/or in combination with a composition described herein comprises Akkermansia, Anaerococcus, Bacteroides, Bifidobacterium (including Bifidobacterium lactis, B. animalis, B. bifidum, B. longum, B. adolescentis, B. breve, and B. infantis), Blautia, Clostridium, Corynebacterium, Dialister, Eubacterium, Faecalibacterium, Finegoldia, Fusobacterium, Lactobacillus (including, L. acidophilus, L. helveticus, L. bifidus, L. lactis, L. fermentii, L. salivarius, L. paracasei, L. brevis, L. delbruekii, L. thermophiles, L. crispatus, L. casei, L. rhamnosus, L. reuteri, L. fermentum, L. plantarum, L. sporogenes, and L. bulgaricus), Peptococcus, Peptostreptococcus, Peptoniphilus, Prevotella, Roseburia, Ruminococcus, Staphylococcus, and/or Streptococcus (including S. lactis, S. cremoris, S. diacetylactis, S. thermophiles).
In embodiments, a commensal bacterial taxa, e.g., GRAS strain, that can be used in and/or in combination with a composition described herein comprises Bacillus coagulans GBI-30, 6086; Bifidobacterium animalis subsp. Lactis BB-12; Bifidobacterium breve Yakult; Bifidobacterium infantis 35624; Bifidobacterium animalis subsp. Lactis UNO 19 (DR10); Bifidobacterium longum BB536; Escherichia coli M-17; Escherichia coli Nissle 1917; Lactobacillus acidophilus DDS-1; Lactobacillus acidophilus LA- 5; Lactobacillus acidophilus NCFM; Lactobacillus casei DN 114-001 {Lactobacillus casei Immunitas(s)/Defensis); Lactobacillus casei CRL431; Lactobacillus casei F19; Lactobacillus paracasei Stl l (or NCC2461); Lactobacillus johnsonii Lai (Lactobacillus LCI, Lactobacillus johnsonii NCC533); Lactococcus lactis L1A; Lactobacillus plantarum 299V; Lactobacillus reuteri ATTC 55730 (Lactobacillus reuteri SD2112); Lactobacillus rhamnosus ATCC 53013; Lactobacillus rhamnosus LB21; Saccharomyces cerevisiae {boulardii) lyo; mixture of Lactobacillus rhamnosus GR-1 and Lactobacillus reuteri RC-14; mixture of Lactobacillus acidophilus NCFM and Bifidobacterium lactis BB-12 or BL-04; mixture of Lactobacillus acidophilus CL1285 and Lactobacillus casei; and a mixture of Lactobacillus helveticus R0052, Lactobacillus rhamnosus R0011, and/or Lactobacillus rhamnosus GG (LGG).

### Synbiotics

Provided herein are combinations of microbes (e.g., bacterial taxa) with glycan compositions disclosed herein which can, e.g., be utilized by the microbes as their substrate for growth. Exogenously introduced microbes can provide a number of beneficial effects, such as, e.g., those described in Tables 1-3. This may occur by promoting the growth of the microbes (using the glycans), thereby allowing the microbes to outgrow other bacteria at the site of colonization.

Methods provided herein include administering one or more (e.g., one or more, two or more, three or more, four or more, and so on) bacterial taxa, such as those listed in Tables 1-3 or Tables 4-6 to a subject in combination with a glycan composition. Such a combination can increase, suppress, and/or alter certain bacterial taxa. Methods are provided herein to modulate the processing of an exogenous substance described herein, comprising administering one or more (e.g., one or more, two or more, three or more, four or more, and so on) bacterial taxa to a subject in combination with a glycan described herein to a subject. The subject can include a subject that has taken, is taking or will be taking an antibiotic. The subject can include a subject that is not taking or has not taken an antibiotic.

### Prebiotics

In some embodiments, the glycan compositions comprise a prebiotic substance. In some embodiments, prebiotics may be administered to a subject receiving the glycan preparations. Prebiotics are substantially non-digestible substances by the host that when consumed may provide a beneficial physiological effect on the host by selectively stimulating the favorable growth or activity of a limited number of indigenous bacteria in the gut (Gibson G R, Roberfroid M B. J Nutr. (1995) 125:1401-12.). A prebiotic such as a dietary fiber or prebiotic oligosaccharide (e.g. crystalline cellulose, wheat bran, oat bran, cone fiber, soy fiber, beet fiber and the like) may further encourage the growth of probiotic and/or commensal bacteria in the gut by providing a fermentable dose of carbohydrates to the bacteria and increase the levels of those microbial populations (e.g. lactobacilli and bifidobacteria) in the gastrointestinal tract.

Prebiotics may include, but are not limited to, various galactans and carbohydrate based gums, such as psyllium, guar, carrageen, gellan, lactulose, and konjac. In some embodiments, the prebiotic is one or more of galactooligosaccharides (GOS), lactulose, raffinose, stachyose, lactosucrose, fructo-oligosaccharides (FOS, e.g. oligofructose or oligofructan), inulin, isomaltooligosaccharide, xylo-oligosaccharides (XOS), paratinose oligosaccharide, isomaltose oligosaccharides (IMOS), transgalactosylated oligosaccharides (e.g. transgalactooligosaccharides), transgalactosylate disaccharides, soybean oligosaccharides (e.g. soyoligosaccharides), chitosan oligosaccharide (chioses), gentiooligosaccharides, soy- and pectin-oligosaccharides, glucooligosaccharides, pecticoligosaccharides, palatinose polycondensates, difructose anhydride III, sorbitol, maltitol, lactitol, polyols, polydextrose, linear and branched dextrans, pullalan, hemicelluloses, reduced paratinose, cellulose, beta-glucose, beta-galactose, beta-fructose, verbascose, galactinol, xylan, inulin, chitosan, beta-glucan, guar gum, gum arabic, pectin, high sodium alginate, and lambda carrageenan, or mixtures thereof.

Prebiotics can be found in certain foods, e.g. chicory root, Jerusalem artichoke, Dandelion greens, garlic, leek, onion, asparagus, wheat bran, wheat flour, banana, milk, yogurt, sorghum, burdock, broccoli, Brussels sprouts, cabbage, cauliflower, collard greens, kale, radish and rutabaga, and miso. In some embodiments, the microbiome regulators described herein are administered to a subject in conjunction with a diet that includes foods rich in prebiotics. Suitable sources of soluble and insoluble fibers are commercially available.

In some embodiments, a glycan composition comprises at least about 1% (w/w) of a prebiotic substance (e.g., at least about 2%, about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, about 96%, about 97%, about 98%, about 99%, or more). In embodiments, the glycan composition comprises FOS. In embodiments, the glycan composition comprises lactulose.

Changes in bacterial populations can be measured by the "prebiotic index." The prebiotic index considers increases in the growth rate of bifidobacteria, eubacteria, and lactobacilli as positive effects and increases in Clostridia, bacteriodes, sulphate-reducing bacteria, and Escherichia coli as negative effects. The prebiotic index (PI) relates to the sum of: (Bifidobacteria/total bacteria) + (Lactobacilli/total bacteria) - (Bacteroides/total bacteria) - (Clostridialtotal bacteria), (see Palframan et al, 2003, Lett Appl Microbiol 37:281-284). In embodiments, administration of the glycan composition to a subject may result in an increased prebiotic index. Administration of a glycan composition to a subject may result in an increase in: Bacteroides, Blautia, Clostridium, Fusobacterium, Eubacterium, Ruminococcus, Peptococcus, Peptostreptococcus, Akkermansia, Faecalibacterium, Roseburia, Prevotella, Bifidobacterium, Lactobacilli, Christensenella minuta, or a Christensenellaceae.

In some embodiments, the glycan composition comprises an antibiotic, an antifungal agent, an antiviral agent, or an anti-inflammatory agent (e.g. a cytokine, hormone, etc.).

In some embodiments, the glycan compositions further comprise a second therapeutic agent or preparation thereof, such as a drug.

For example, the second therapeutic agent is an anti-cancer drug. Examples of anti-cancer drugs include: checkpoint inhibitors (such as, e.g., anti-PD-1, anti-PD-L1, anti-CTLA4, anti-TIM-3, anti-LAG-3); vaccines (such as, e.g., autologous cancer vaccines, allogeneic cancer vaccines, neoantigen cancer vaccines, shared antigen cancer vaccines (e.g. NY-ESO-1)); targeted kinase inhibitors (such as, e.g., Imatinib mesylate, Ibrutinib, Neratinib, Palpociclib, Erlotinib, Lapatinib); antibodies (such as, e.g., Bevacizumab, Trastuzumab, Rituximab, Cetuximab); chemotherapeutics (such as, e.g., irinotecan, 5-flurouracil, lenalidomide, capecitabine, docetaxel), antibody-drug conjugates (e.g. ado-trastuzumab emtansine), and any other anti-cancer drug mentioned elsewhere herein.

For example, the second therapeutic agent is a pain-management drug. In some embodiments, the pain-management drug is an opioid, such as, e.g., codeine, fentanyl, hydrocodone, hydrocodone/acetaminophen, hydromorphone, meperidine, methadone, morphine, oxycodone, oxycodone and acetaminophen, or oxycodone and naloxone. In other embodiments, the pain-management drug is a non-opioid, such as, e.g., acetaminophen or nonsteroidal anti-inflammatory drugs (NSAIDs), such as aspirin and ibuprofen.

For example, the second therapeutic agent is a cardiac glycoside, sulfonamide (sulfa drug), nucleoside analogue, or aminosalicylate.

In some embodiments, the cardiac glycoside is digoxin, digitoxin, convallotoxin, antiarin, or oleandrin.

In some embodiments, the sulfonamide (sulfa drug) is an antimicrobial, e.g., a short acting antimicrobial, e.g., Sulfafurazole, Sulfacetamide, Sulfadiazine, Sulfadimidine, Sulfafurazole (sulfisoxazole), Sulfisomidine (sulfaisodimidine). In some embodiments, the sulfonamide (sulfa drug) is an antimicrobial, e.g., an intermediate-acting microbial, e.g., Sulfadoxine, Sulfamethoxazole, Sulfamoxole, or Sulfanitran. In some embodiments, the sulfonamide (sulfa drug) is an antimicrobial, e.g., a long-acting antimicrobial, e.g., Sulfadimethoxine, Sulfamethoxypyridazine, orSulfametoxydiazine. In some embodiments, the sulfonamide (sulfa drug) is an antimicrobial, e.g., an ultra-long-acting antimicrobial, e.g., Sulfadoxine, Sulfametopyrazine, or Terephtyl. In some embodiments, the sulfonamide (sulfa drug) is a Sulfonylurea, e.g., a anti-diabetic agents, e.g., Acetohexamide, Carbutamide, Chlorpropamide, Glibenclamide (glyburide), Glibornuride, Gliclazide, Glyclopyramide, Glimepiride, Glipizide, Gliquidone, Glisoxepide, Tolazamide, or Tolbutamide. In some embodiments, the sulfonamide (sulfa drug) is a diuretic, e.g., Acetazolamide, Bumetanide, Chlorthalidone, Clopamide, Furosemide, Hydrochlorothiazide, Indapamide, Mefruside, Metolazone, or Xipamide. In some embodiments, the sulfonamide (sulfa drug) is an anticonvulsant, e.g., Ethoxzolamide, Sultiame, Topiramate, or Zonisamide. In some embodiments, the sulfonamide (sulfa drug) is an antiretrovirals, e.g., Amprenavir (HIV protease inhibitor), Darunavir (HIV protease inhibitor), Delavirdine (non-nucleoside reverse transcriptase inhibitor), Fosamprenavir (HIV protease inhibitor), or Tipranavir (HIV protease inhibitor). In some embodiments, the sulfonamide (sulfa drug) is a Hepatitis C antiviral, e.g., Asunaprevir (NS3/4A protease inhibitor), Beclabuvir (NS5B RNA polymerase inhibitor), Dasabuvir (NS5B RNA polymerase inhibitor), Grazoprevir (NS3/4A protease inhibitor), Paritaprevir (NS3/4A protease inhibitor), or Simeprevir (NS3/4A protease inhibitor). In some embodiments, the sulfonamide (sulfa drug) is e.g., Apricoxib (COX-2 inhibitor), Bosentan (endothelin receptor antagonist), Brinzolamide (carbonic anhydrase inhibitor for glaucoma), Celecoxib (COX-2 inhibitor), Dofetilide (class III antiarrhythmic), Dorzolamide (anti-glaucoma carbonic anhydrase inhibitor), Dronedarone (class III antiarrhythmic), Ibutilide (class III antiarrhythmic), Parecoxib (COX-2 inhibitor), Probenecid (uricosuric), Sotalol (β blocker), Sulfasalazine (anti-inflammatory agent and a DMARD), Sumatriptan (antimigraine triptan), Tamsulosin (α blocker), or Udenafil (PDE5 inhibitor).

In some embodiments, the nucleoside analogues is a deoxyadenosine analogues, e.g., didanosine (ddI)(HIV) or vidarabine (antiviral). In some embodiments, the nucleoside analogue is an adenosine analogues, e.g., BCX4430 (Ebola). In some embodiments, the nucleoside analogues is a deoxycytidine analogue, e.g., cytarabine (chemotherapy), gemcitabine (Chemotherapy), emtricitabine (FTC)(HIV), lamivudine (3TC)(HIV, hepatitis B), or zalcitabine (ddC)(HIV). In some embodiments, the nucleoside analogue is a guanosine or deoxyguanosine analogue, e.g., abacavir (HIV), acyclovir, or entecavir (hepatitis B). In some embodiments, the nucleoside analogue is a thymidine or deoxythymidine analogue, e.g., stavudine (d4T), telbivudine (hepatitis B) or zidovudine (azidothymidine, or AZT)(HIV). In some embodiments, the nucleoside analogue is a deoxyuridine analogue, e.g., idoxuridine or trifluridine. In some embodiments, the nucleoside analogue is a Pyrimidine analogue, e.g., 5-Fluorouracil (5FU), Floxuridine (FUDR), Cytarabine (Cytosine arabinoside), or 6-azauracil (6-AU). In some embodiments, the nucleoside analogue is a purine analog, e.g., Azathioprine, Mercaptopurine, Thiopurines, Fludarabine, or Pentostatin. In some embodiments, the aminosalicylate is 4-Aminosalicylic acid, Balsalazide, Olsalazine, Sulfasalazine, or Mesalazine (5-Aminosalicylic acid).

For example, the second therapeutic agent is an anti-proliferative, anti-neoplastic or anti-tumor drugs or treatments. In some embodiments, such drugs or treatments include chemotherapeutic drugs, e.g., cytotoxic drugs (e.g., alkylating agents, antimetabolites, anti-tumor antibiotics, topoisomerase inhibitors, mitotic inhibitors, corticosteroids); cancer growth blockers such as tyrosine kinase inhibitors and proteasome inhibitors; other chemical drugs such as L-asparaginase and bortezomib (Velcade^{®}), anti-cancer drugs, e.g., checkpoint inhibitors (such as, e.g., anti-PD-1, anti-PD-L1, anti-CTLA4, anti-TIM-3, anti-LAG-3); vaccines (such as, e.g., autologous cancer vaccines, allogeneic cancer vaccines, neoantigen cancer vaccines, shared antigen cancer vaccines (e.g. NY-ESO-1)); targeted kinase inhibitors (such as, e.g., Imatinib mesylate, Ibrutinib, Neratinib, Palpociclib, Erlotinib, Lapatinib); or antibodies (such as, e.g., Bevacizumab, Trastuzumab, Rituximab, Cetuximab). Hormone therapies (or anti-hormone therapies) may be used, e.g., for hormone-sensitive cancers.

For example, the second therapeutic agent is a drug that is known to induce diarrhea or a drug that is known to induce constipation. In some embodiments the drugs known to induce diarrhea include 5-fluorouracil (5-FU), methotrexate, irinotecan, taxanes, monoclonal antibodies, and hormonal agents. In some embodiments the drugs known to induce constipation include vinca alkaloids, platinums (e.g., cisplatin), thalidomide and hormonal agents.

### Pharmaceutical Compositions, Medical Foods, Supplements, Food Ingredients, and Unit Dosage Form

Provided herein are pharmaceutical compositions comprising glycan compositions. Further provided herein are medical foods comprising glycan compositions. Still further provided herein are dietary supplements comprising glycan compositions. Still further provided herein are food ingredients comprising glycan compositions.

Optionally, the compositions comprise one or more of the following: i) a prebiotic substance, such as, e.g., a dietary fiber; ii) a bacterial taxa, such as, e.g., a probiotic bacterium; iii) a micronutrient, such as, e.g., a vitamin, mineral or polyphenol compound, iv) a therapeutic drug, such as, e.g., an anti-cancer drug, a pain management drug, a drug that manages treatment side-effects, a drug that manages metabolism, an anti-inflammatory drug, or an anti-microbial agent.

Pharmaceutical compositions, medical foods, supplements and unit dosage forms suitable for use in the methods and compositions described herein can be found in WO 2016/122889, WO 2016/172657, and WO 2016/172658.

In some embodiments, the glycan compositions do not contain a prebiotic substance. In some embodiments glycan compositions do not contain a probiotic bacterium.
In some embodiments, glycan compositions comprie one or more of glycan preparations described herein.

The glycan polymer preparations described herein may be formulated into any suitable dosage form, e.g. for nasal, oral, rectal or gastric administration. In some embodiments, the glycan polymer preparations described herein may be formulated for enteral administration. In some embodiments, the glycan polymer preparations described herein may be formulated for tube feeding (e.g. naso-gastric, oral-gastric or gastric feeding). The dosage forms described herein can be manufactured using processes that are known to those of skill in the art.

The dosage form may be a packet, such as any individual container that contains a glycan polymer preparation in the form of, e.g., a liquid (wash/rinse), a gel, a cream, an ointment, a powder, a tablet, a pill, a capsule, a depository, a single-use applicator or medical device (e.g. a syringe). For example, provided is also an article of manufacture, such as a container comprising a unit dosage form of the glycan polymer preparation, and a label containing instructions for use of such glycan polymer.

Forms of the compositions that can be used orally include tablets, push-fit capsules made of gelatin, as well as soft, sealed capsules made of gelatin and a plasticizer, such as glycerol or sorbitol. Tablets can be made by compression or molding, optionally with one or more accessory ingredients. Compressed tablets can be prepared by compressing in a suitable machine the active ingredient in a free-flowing form such as a powder or granules, optionally mixed with binders (e.g., povidone, gelatin, hydroxypropylmethyl cellulose), inert diluents, preservative, antioxidant, disintegrant (e.g., sodium starch glycolate, cross-linked povidone, cross-linked sodium carboxymethyl cellulose) or lubricating, surface active or dispersing agents. Molded tablets can be made by molding in a suitable machine a mixture of the powdered compound moistened with an inert liquid diluent. The tablets can optionally be coated or scored and can be formulated so as to provide slow or controlled release of the active ingredient therein. Tablets can optionally be provided with an enteric coating, to provide release in parts of the gut (e.g., colon, lower intestine) other than the stomach. All formulations for oral administration can be in dosages suitable for such administration. The push-fit capsules can contain the active ingredients in admixture with filler such as lactose, binders such as starches, and/or lubricants such as talc or magnesium stearate and, optionally, stabilizers. In soft capsules, the active compounds and/or other agents (e.g., prebiotics or probiotics) can be dissolved or suspended in suitable liquids, such as fatty oils, liquid paraffin, or liquid polyethylene glycols. In addition, stabilizers can be added. Dragee cores are provided with suitable coatings. For this purpose, concentrated sugar solutions can be used, which can optionally contain gum arabic, talc, polyvinyl pyrrolidone, carbopol gel, polyethylene glycol, or titanium dioxide, lacquer solutions, and suitable organic solvents or solvent mixtures. Dyestuffs or pigments can be added to the tablets or dragee coatings for identification or to characterize different combinations of active compound doses.

Formulations for oral use can also be presented as hard gelatin capsules wherein the active ingredient is mixed with an inert solid diluent, for example, calcium carbonate, calcium phosphate or kaolin, or as soft gelatin capsules wherein the active ingredient is mixed with water soluble carrier such as polyethylene glycol or an oil medium, for example peanut oil, liquid paraffin, or olive oil.

In one embodiment, a provided glycan polymer preparation includes a softgel formulation. A softgel can contain a gelatin based shell that surrounds a liquid fill. The shell can be made of gelatin, plasticizer (e.g., glycerin and/or sorbitol), modifier, water, color, antioxidant, or flavor. The shell can be made with starch or carrageenan. The outer layer can be enteric coated. In one embodiment, a softgel formulation can include a water or oil soluble fill solution, or suspension of a composition covered by a layer of gelatin.

Solid formulations for oral use may comprise an enteric coating, which may control the location at which a glycan polymer preparation is absorbed in the digestive system. For example, an enteric coating can be designed such that a glycan polymer preparation does not dissolve in the stomach but rather travels to the small intestine, where it dissolves. An enteric coating can be stable at low pH (such as in the stomach) and can dissolve at higher pH (for example, in the small intestine). Material that can be used in enteric coatings includes, for example, alginic acid, cellulose acetate phthalate, plastics, waxes, shellac, and fatty acids (e.g., stearic acid, palmitic acid).

Formulations for oral use may also be presented in a liquid dosage from. Liquid preparations can be in the form of, for example, aqueous or oily suspensions, solutions, emulsions syrups or elixirs, or can be presented as a dry product for reconstitution with water or other suitable vehicle before use. Such liquid preparations can contain conventional additives, such as suspending agents, for example sorbitol, methyl cellulose, glucose syrup, gelatin, hydroxyethyl cellulose, carboxymethyl cellulose, aluminum stearate gel or hydrogenated edible fats, emulsifying agents, for example lecithin, sorbitan monooleate, acacia; nonaqueous vehicles (which can include edible oils), for example almond oil, oily esters such as glycerine, propylene glycol, or ethyl alcohol; preservatives, for example methyl or propyl p-hydoxybenzoate or sorbic acid, and, if desired, conventional flavoring or coloring agents. In some embodiments, liquid formulations can comprise, for example, an agent in water-in-solution and/or suspension form; and a vehicle comprising polyethoxylated castor oil, alcohol, and/or a polyoxyethylated sorbitan mono-oleate with or without flavoring. Each dosage form may comprise an effective amount of a glycan polymer and can optionally comprise pharmaceutically inert agents, such as conventional excipients, vehicles, fillers, binders, disintegrants, pH adjusting substances, buffer, solvents, solubilizing agents, sweeteners, coloring agents, and any other inactive agents that can be included in pharmaceutical dosage forms for administration. Examples of such vehicles and additives can be found in Remington's Pharmaceutical Sciences, 17th edition (1985).

The pharmaceutical compositions provided herein can be in unit-dosage forms or multiple-dosage forms. A unit-dosage form, as used herein, refers to physically discrete unit suitable for administration to human in need thereof. In an embodiment, the unit-dosage form is provided in a package. Each unit-dose can contain a predetermined quantity of an active ingredient(s) sufficient to produce the desired therapeutic effect, in association with other pharmaceutical carriers or excipients. Examples of unit-dosage forms include, but are not limited to, ampoules, syringes, and individually packaged tablets and capsules. Unit-dosage forms can be administered in fractions or multiples thereof. A multiple-dosage form is a plurality of identical unit-dosage forms packaged in a single container, which can be administered in segregated unit-dosage form. Examples of multiple-dosage forms include, but are not limited to, vials, bottles of tablets or capsules, or bottles of pints or gallons. In another embodiment, the multiple dosage forms comprise different pharmaceutically active agents. For example, a multiple dosage form can be provided which comprises a first dosage element comprising a composition comprising a glycan polymer and a second dosage element comprising a prebiotic, a therapeutic agent and/or a probiotic, which can be in a modified release form. In this example a pair of dosage elements can make a single unit dosage. In one embodiment, a kit is provided comprising multiple unit dosages, wherein each unit comprises a first dosage element comprising a composition comprising a glycan polymer preparation and a second dosage element comprising probiotic, a pharmaceutical agent, a prebiotic or a combination thereof, which can be in a modified release form. In another embodiment, the kit further comprises a set of instructions.

In some embodiments, the unit-dosage form comprises between about 1 mg to about 100 g of the glycan polymer preparation (e.g., a glycan polymer disclosed herein). For example, the unit-dosage form may comprise about 50 mg to about 50 g, about 500 mg to about 50 g, about 5 g to about 50 g, about 100 mg to about 100 g, about 1 g to about 100 g, about 10 g to about 100 g, about 1 g to about 10 g, about 1 g to about 20 g, about 1 g to about 30 g, about 1 g to about 40 g, about 1 g to about 50 g, about 1 g to about 60 g, about 1 g to about 70 g, about 1 g to about 80 g, about 1 g to about 90 g, about 1 g to about 100 g, about 1 g to about 150 g, about 1 g to about 200 g of the glycan polymer.

In other embodiments, the unit-dosage form comprises between about 0.001 mL to about 1000 mL of the glycan polymer (e.g., a glycan polymer disclosed herein). For example, the unit-dosage form may comprise about 0.001 mL to about 950 mL, about 0.005 mL to about 900 mL, about 0.01 mL to about 850 mL, about 0.05 mL to about 800 mL, about 0.075 mL to about 750 mL, about 0.1 mL to about 700 mL, about 0.25 mL to about 650 mL, about 0.5 mL to about 600 mL, about 0.75 mL to about 550 mL, about 1 mL to about 500 mL, about 2.5 mL to about 450 mL, about 5 mL to about 400 mL, about 7.5 mL to about 350 mL, about 10 mL to about 300 mL, about 12.5 mL to about 250 mL, about 15 mL to about 200 mL, about 17.5 mL to about 150 mL, about 20 mL to about 100 mL, or about 25 mL to about 75 mL of the glycan polymer.

In certain embodiments, the unit-dosage form comprises about 0.001 mL to about 10 mL, about 0.005 mL to about 7.5 mL, about 0.01 mL to about 5 mL, about 0.05 mL to about 2.5 mL, about 0.1 mL to about 1 mL, about 0.25 mL to about 1 mL, or about 0.5 mL to about 1 mL of the glycan polymer. In other embodiments, the unit-dosage form comprises about 0.01 mL to about 10 mL, about 0.025 mL to about 7.5 mL, about 0.05 mL to about 5 mL, or about 0.1 mL to about 2.5 mL of the glycan polymer. In other embodiments, the unit-dosage form comprises about 0.1 mL to about 10 mL, about 0.25 mL to about 7.5 mL, about 0.5 mL to about 5 mL, about 0.5 mL to about 2.5 mL, or about 0.5 mL to about 1 mL of the glycan polymer.

In some embodiments, the unit-dosage form, e.g., a tablet, capsule (e.g., a hard capsule, push-fit capsule, or soft capsule), or softgel, has a body length of between about 0.1 inches to about 1.5 inches (e.g., about 0.5 inches and about 1 inch), or about 5 mm to about 50 mm (e.g., about 10 mm to about 25 mm). In some embodiments, the unit-dosage form. e.g., a tablet, capsule (e.g., a hard capsule, push-fit capsule, or soft capsule), or softgel, has an external diameter of about 0.05 inches to about 1 inch (e.g., about 0.1 inches to about 0.5 inches), or about 1 mm to about 25 mm (e.g., about 5 mm to about 10 mm).

Each unit-dosage form of the glycan polymer may have a caloric value of between about 0.01 kcal and about 1000 kcal. For example, the unit-dosage form may have a caloric value of about 0.01 kcal to about 100 kcal, about 0.05 kcal to about 50 kcal, about 0.1 kcal to about 10 kcal, about 0.25 kcal to about 2.5 kcal, about 0.5 kcal to about 5 kcal, about 0.75 kcal to about 7.5 kcal, about 1 kcal to 10 kcal, about 5 kcal to about 50 kcal, or about 10 kcal to about 100 kcal. In certain embodiments, the unit-dosage form of the glycan polymer has a caloric value of between 10 kcal to about 500 kcal.. In certain embodiments, the unit-dosage form of the glycan polymer has a caloric value of between 1 kcal to about 100 kcal. In certain embodiments, the unit-dosage form of the glycan polymer has a caloric value of between 0.1 kcal to about 10 kcal.

In still other embodiments, the unit-dosage form may have a caloric value of about 0.001 kcal to about 10 kcal, about 0.005 kcal to about 10 kcal, about 0.01 kcal to about 10 kcal, about 0.025 kcal to about 25 kcal, about 0.05 kcal to about 50 kcal, about 0.075 kcal to about 75 kcal, about 0.1 kcal to 100 kcal, about 0.25 kcal to about 10 kcal, about 0.5 kcal to about 5 kcal, about 0.25 kcal to about 25 kcal, or about 0.1 kcal to about 1 kcal.

The unit-dosage form of the glycan polymer may be formulated to dissolve in an aqueous solution (e.g., water, milk, juice, and the like) and is orally administered as a beverage, syrup, solution, or suspension. For example, the unit-form dosage of the glycan polymer may comprise a cube, packet, lozenge, pill, tablet, capsule, candy, powder, elixir, or concentrated syrup formulated for dissolving into an aqueous solution prior to oral administration. In other embodiments, the unit-dosage form of the glycan polymer may comprise a cube, packet, lozenge, pill, tablet, capsule, candy, powder, elixir, or concentrated syrup formulated to dissolve in vivo, e.g., in the mouth, stomach, intestine, or colon of the subject upon oral administration.

In some embodiments, the glycan polymer preparation is administered enterically. This preferentially includes oral administration, or by an oral or nasal tube (including nasogastric, nasojejunal, oral gastric, or oral jejunal). In other embodiments, administration includes rectal administration (including enema, suppository, or colonoscopy).

The dosage forms described herein can be manufactured using processes that are known to those of skill in the art. For example, for the manufacture of tablets, an effective amount of a prebiotic can be dispersed uniformly in one or more excipients or additives, for example, using high shear granulation, low shear granulation, fluid bed granulation, or by blending for direct compression. Excipients and additives include diluents, binders, disintegrants, dispersants, lubricants, glidants, stabilizers, surfactants, antiadherents, sorbents, sweeteners, and colorants, or a combination thereof. Diluents, also termed fillers, can be used to increase the bulk of a tablet so that a practical size is provided for compression. Non-limiting examples of diluents include lactose, cellulose, microcrystalline cellulose, mannitol, dry starch, hydrolyzed starches, powdered sugar, talc, sodium chloride, silicon dioxide, titanium oxide, dicalcium phosphate dihydrate, calcium sulfate, calcium carbonate, alumina and kaolin. Binders can impart cohesive qualities to a tablet formulation and can be used to help a tablet remain intact after compression. Non-limiting examples of suitable binders include starch (including corn starch and pregelatinized starch), gelatin, sugars (e.g., glucose, dextrose, sucrose, lactose and sorbitol), celluloses, polyethylene glycol, alginic acid, dextrin, casein, methyl cellulose, waxes, natural and synthetic gums, e.g., acacia, tragacanth, sodium alginate, gum arabic, xantan gum, and synthetic polymers such as polymethacrylates, polyvinyl alcohols, hydroxypropylcellulose, and polyvinylpyrrolidone. Lubricants can also facilitate tablet manufacture; non-limiting examples thereof include magnesium stearate, calcium stearate, stearic acid, glyceryl behenate, and polyethylene glycol. Disintegrants can facilitate tablet disintegration after administration, and non-limiting examples thereof include starches, alginic acid, crosslinked polymers such as, e.g., crosslinked polyvinylpyrrolidone, croscarmellose sodium, potassium or sodium starch glycolate, clays, celluloses (e.g., carboxymethylcelluloses (e.g., carboxymethylcellulose (CMC), CMC-Na, CMC-Ca)), starches, gums and the like. Non-limiting examples of suitable glidants include silicon dioxide, talc, and the like. Stabilizers can inhibit or retard drug decomposition reactions, including oxidative reactions. Surfactants can also include and can be anionic, cationic, amphoteric or nonionic. Exemplary sweeteners may include stevia extract, aspartame, sucrose, alitame, saccharin, and the like. If desired, the tablets can also comprise nontoxic auxiliary substances such as pH buffering agents, preservatives, e.g., antioxidants, wetting or emulsifying agents, solubilizing agents, coating agents, flavoring agents (e.g., mint, cherry, anise, peach, apricot, licorice, raspberry, vanilla), and the like. Additional excipients and additives may include aluminum acetate, benzyl alcohol, butyl paraben, butylated hydroxy toluene, calcium disodium EDTA, calcium hydrogen phosphate dihydrate, dibasic calcium phosphate, tribasic calcium phosphate, candelilla wax, carnuba wax, castor oil hydrogenated, cetylpyridine chloride, citric acid, colloidal silicone dioxide, copolyvidone, corn starch, cysteine HCl, dimethicone, disodium hydrogen phosphate, erythrosine sodium, ethyl cellulose, gelatin, glycerin, glyceryl monooleate, glyceryl monostearate, glycine, HPMC pthalate, hydroxypropylcellulose, hydroxyl propyl methyl cellulose, hypromellose, iron oxide red or ferric oxide, iron oxide yellow, iron oxide or ferric oxide, magnesium carbonate, magnesium oxide, magnesium stearate, methionine, methacrylic acid copolymer, methyl paraben, silicified microcrystalline cellulose, mineral oil, phosphoric acid, plain calcium phosphate, anhydrous calcium phosphate, polaxamer 407, polaxamer 188, plain polaxamer, polyethylene oxide, polyoxy140 stearate, polysorbate 80, potassium bicarbonate, potassium sorbate, potato starch, povidone, propylene glycol, propylene paraben, propyl paraben, retinyl palmitate, saccharin sodium, selenium, silica, silica gel, fumed silica, sodium benzoate, sodium carbonate, sodium citrate dihydrate, sodium crossmellose, sodium lauryl sulfate, sodium metabisulfite, sodium propionate, sodium starch, sodium starch glycolate, sodium stearyl fumarate, sorbic acid, sorbitol, sorbitan monooleate, pregelatinized starch, succinic acid, triacetin, triethyl citrate, vegetable stearin, vitamin A, vitamin E, vitamin C, or a combination thereof. The amounts of these excipients and additives can be properly selected based on their relation to other components and properties of the preparation and production method.

Immediate-release formulations of an effective amount of a glycan polymer preparation can comprise one or more combinations of excipients that allow for a rapid release of a pharmaceutically active agent (such as from 1 minute to 1 hour after administration).Controlled-release formulations (also referred to as sustained release (SR), extended-release (ER, XR, or XL), time-release or timed-release, controlled-release (CR), or continuous-release) refer to the release of a glycan polymer preparation from a dosage form at a particular desired point in time after the dosage form is administered to a subject.

In one embodiment a controlled release dosage form begins its release and continues that release over an extended period of time. Release can occur beginning almost immediately or can be sustained. Release can be constant, can increase or decrease over time, can be pulsed, can be continuous or intermittent, and the like. In one embodiment, a controlled release dosage refers to the release of an agent from a composition or dosage form in which the agent is released according to a desired profile over an extended period of time. In one aspect, controlled-release refers to delayed release of an agent from a composition or dosage form in which the agent is released according to a desired profile in which the release occurs after a period of time.

Pharmaceutical carriers or vehicles suitable for administration of the compounds provided herein include all such carriers known to those skilled in the art to be suitable for the particular mode of administration. In addition, the compositions can one or more components that do not impair the desired action, or with components that supplement the desired action, or have another action.

In a further aspect, the dosage form can be an effervescent dosage form. Effervescent means that the dosage form, when mixed with liquid, including water and saliva, evolves a gas. Some effervescent agents (or effervescent couple) evolve gas by means of a chemical reaction which takes place upon exposure of the effervescent disintegration agent to water or to saliva in the mouth. This reaction can be the result of the reaction of a soluble acid source and an alkali monocarbonate or carbonate source. The reaction of these two general compounds produces carbon dioxide gas upon contact with water or saliva. An effervescent couple (or the individual acid and base separately) can be coated with a solvent protective or enteric coating to prevent premature reaction. Such a couple can also be mixed with previously lyophilized particles (such as a glycan polymer). The acid sources can be any which are safe for human consumption and can generally include food acids, acid and hydrite antacids such as, for example: citric, tartaric, amalic, fumeric, adipic, and succinics. Carbonate sources include dry solid carbonate and bicarbonate salt such as sodium bicarbonate, sodium carbonate, potassium bicarbonate and potassium carbonate, magnesium carbonate and the like. Reactants which evolve oxygen or other gasses and which are safe for human consumption are also included. In one embodiment citric acid and sodium bicarbonate are used.

In another aspect, the dosage form can be in a candy form (e.g., matrix), such as a lollipop or lozenge. In one embodiment an effective amount of a glycan polymer is dispersed within a candy matrix. In one embodiment the candy matrix comprises one or more sugars (such as dextrose or sucrose). In another embodiment the candy matrix is a sugar-free matrix. The choice of a particular candy matrix is subject to wide variation. Conventional sweeteners (e.g., sucrose), sugar alcohols suitable for use with diabetic patients (e.g., sorbitol or mannitol), or other sweeteners (e.g., sweeteners described herein) may be employed. The candy base can be very soft and fast dissolving, or can be hard and slower dissolving. Various forms will have advantages in different situations.

A candy mass composition comprising an effective amount of the glycan polymer can be orally administered to a subject in need thereof so that an effective amount of the glycan polymer will be released into the subject's mouth as the candy mass dissolves and is swallowed. A subject in need thereof includes a human adult or child.

The dosage forms described herein can also take the form of pharmaceutical particles manufactured by a variety of methods, including but not limited to high-pressure homogenization, wet or dry ball milling, or small particle precipitation (e.g., nGimat's NanoSpray). Other methods useful to make a suitable powder formulation are the preparation of a solution of active ingredients and excipients, followed by precipitation, filtration, and pulverization, or followed by removal of the solvent by freeze-drying, followed by pulverization of the powder to the desired particle size. In one embodiment, the pharmaceutical particles have a final size of 3-1000 microns, such as at most 3, 4, 5, 6, 7, 8, 9, 10, 20, 30, 40, 50, 60, 70, 80, 90, 100, 150, 200, 250, 300, 350, 400, 450, 500, 550, 600, 650, 700, 750, 800, 850, 900, 950, 1000 microns. In another embodiment, the pharmaceutical particles have a final size of 10-500 microns. In another embodiment, the pharmaceutical particles have a final size of 50-600 microns. In another embodiment, the pharmaceutical particles have a final size of 100-800 microns.

In another aspect, the disclosure provides a method of making a unit-dosage form described herein, comprising providing a glycan polymer (e.g., a glycan polymer described herein); formulating the glycan polymer into a unit-dosage form (e.g., a unit-dosage form described herein), packaging the unit-dosage form, labelling the packaged unit-dosage form, and/or selling or offering for sale the packaged and labeled unit-dosage form.

The unit-dosage forms described herein may also be processed. In one embodiment, the processing comprises one or more of: processing the dosage form into a pharmaceutical composition, e.g., formulating, combining with a second component, e.g., an excipient or buffer; portioning into smaller or larger aliquots; disposing into a container, e.g., a gas or liquid tight container; packaging; associating with a label; shipping or moving to a different location. In one embodiment, the processing comprises one or more of: classifying, selecting, accepting or discarding, releasing or withholding, processing into a pharmaceutical composition, shipping, moving to a different location, formulating, labeling, packaging, releasing into commerce, or selling or offering for sale, depending on whether the predetermined threshold is met. In some embodiments, the processed dosage forms comprise a glycan polymer described herein.

In some embodiments, the processing comprises one or more of: processing the dosage form into a pharmaceutical composition, e.g., formulating, combining with a second component, e.g., an excipient or buffer; portioning into smaller or larger aliquots; disposing into a container, e.g., a gas or liquid tight container; packaging; associating with a label; shipping or moving to a different location. In one embodiment, the processing comprises one or more of: classifying, selecting, accepting or discarding, releasing or withholding, processing into a pharmaceutical composition, shipping, moving to a different location, formulating, labeling, packaging, releasing into commerce, or selling or offering for sale, depending on the determination.

In another embodiment, an oral dosage form is provided comprising a glycan polymer preparation, wherein the oral dosage form is a syrup. The syrup can comprise about 1%, 5%, 10%, 15%, 20%, 25%, 30%, 35%, 40%, 45%, 50%, 55%, 60%, 65%, 70%, 75%, 80%, or 85% solid. The syrup can comprise about 15%, 20%, 25%, 30%, 35%, 40%, 45%, or 50% liquid, for example, water. The solid can comprise a glycan polymer preparation. The solid can be, for example, about 1-96%, 10-96%, 20-96%, 30-96%, 40-96%, 50-96%, 60-96%, 70-96%, 80-96%, or 90-96% glycan polymer preparation. In another embodiment, a glycan polymer preparation is formulated as a viscous fluid.

In one embodiment, the composition comprises a foaming component, a neutralizing component, or a water-insoluble dietary fiber. A foaming component can be at least one member selected from the group consisting of sodium hydrogencarbonate, sodium carbonate, and calcium carbonate. In one embodiment a neutralizing component can be at least one member selected from the group consisting of citric acid, L-tartaric acid, fumaric acid, L-ascorbic acid, DL-malic acid, acetic acid, lactic acid, and anhydrous citric acid. In one embodiment a water-insoluble dietary fiber can be at least one member selected from the group consisting of crystalline cellulose, wheat bran, oat bran, cone fiber, soy fiber, and beet fiber. The formulation can contain a sucrose fatty acid ester, powder sugar, fruit juice powder, and/or flavoring material.

In some embodiments, the dosage forms are formulated to release the pharmaceutical compositions comprising glycan polymer preparations in a specific region(s) of the GI tract, such as the small or the large intestine. In some embodiments, the dosage forms are formulated to release the pharmaceutical compositions comprising glycan polymer preparations in a specific region(s) of the GI tract, such as the cecum, ascending colon, transverse colon, descending colon, sigmoid colon, and/or rectum.

In some embodiments, the dosage form for the glycan polymer preparations described herein is an enzyme-responsive delivery system. For example, trypsin responsive polymers can be made using hydrogels that are crosslinked by peptides that are degraded by trypsin. Trypsin is active in the small intestine. Trypsin-responsive delivery systems can be used to target delivery of the glycan polymer preparations to the small intestine. In another example, enzyme-digestible hydrogels consisting of poly(vinyl pyrrolidone) crosslinked with albumin are degraded in the presence of pepsin.

In some embodiments, the dosage form for the glycan polymer preparations described herein is a delivery device that enables prolonged retention at a specific site in the GI tract. For example, a gastroretentive delivery system enables prolonged release of the glycan polymer preparations to the stomach. Gastroretentive delivery may be used for the glycan polymer preparations that modulate bacteria in the stomach or in the upper small intestine.

In some embodiments, the dosage form for the glycan polymer preparations described herein is a mucoadhesive delivery system that adheres to the mucosal surfaces of the stomach. They are typically composed of polymers with numerous hydrogen-bonding groups, e.g., crosslinked polyacrylic acids, sodium carboxymethyl cellulose, sodium alginate, carrageenan, Carbopol 934P, or thiolated polycarbophil.

In some embodiments, the dosage form for the glycan polymer preparations described herein is an expanding delivery system that rapidly increases in size in the stomach, which slows its passage through the pylorus. Such systems include systems that unfold in the stomach. For example, geometric shapes such as tetrahedrons, rings, disks, etc. can be packed into a gelatin capsule. When the capsule dissolves, the shape unfolds. The systems can be composed of one or more erodible polymer (e.g., hydroxypropyl cellulose), one or more nonerodible polymer (e.g., polyolefins, polyamides, polyurethanes). The glycan polymer may then be dispersed within the polymer matrix. The retention times can be fine-tuned by the polymer blend. Alternatively, devices made out of elastic polymers that are stable in the acidic pH of the stomach but dissolve in the neutral/alkaline conditions further along the GI tract can be used. Such polymer formulations can prevent intestinal obstruction when the device exits the stomach. Supramolecular polymer gels crosslinked by hydrogen bonds between carboxyl groups may also be used, e.g. composed of poly(acryloyl 6-aminocaproic acid) (PA6ACA) and poly(methacrylic acid-co-ethyl acrylate) (EUDRAGIT L 100-55). Other systems include swellable excipients, such as collagen sponges. For example, a hydrogel matrix (e.g. a swellable core: polyvinyl pyrrolidone XL, Carbopol 934P, calcium carbonate) swells 2-50 times in the stomach. Superporous hydrogel composites swell to hundreds of times their original volume in a few minutes. Some systems exploit gas generation to achieve expansion, e.g. carbon dioxidegenerating, expandable systems that are surrounded by a hydrophilic membrane.

In some embodiments, the dosage form for the glycan polymer preparations described herein is a density-controlled delivery system. These systems are designed to either float or sink in gastric fluids, which delays their emptying from the stomach. For example, high-density systems enable the device to settle to the bottom of the stomach, below the pylorus, and thus avoid stomach emptying. Other systems are low-density/floating systems. Such devices may, e.g., comprise entrapped air in hollow chambers or may incorporate low-density materials like fats, oils, or foam powder. Low density may be achieved through swelling, e.g. hydrocolloid containing capsules dissolve upon contacting gastric fluid and the hydrocolloids swell to form a mucous body. Alternative polymers include: chitosans, sodium alginate, and glycerol monooleate matrix. Low density may be achieved through gas generation. For example, tablets loaded with carbonate and optionally citric acid generate carbon dioxide after contact with acidic aqueous media. The carbon dioxide generated is entrapped within the gelling hydrocolloid causing the system to float. Hydrocolloids include hydroxypropyl methylcellulose and Carbopol 934P.

In some embodiments, the dosage form for the glycan polymer preparations described herein employs a design to retain a device in the small or large intestine. The location-specific nature of the device is provided by a specific triggering method, e.g. pH, enzyme, etc. These include systems designed for mucoadhesion and also microneedle pills. Microneedle pills comprise a drug reservoir spiked with microneedles that is encapsulated in a pH-responsive coating. When the pill reaches the desired location in the GI tract and the coating dissolves, the microneedles enable the pill to become stuck to the lining of the GI tract. In other embodiments, the microneedle pills comprise a capsule that consists of two chemical compartments filled with citric acid and sodium bicarbonate, respectively. As the pill dissolves in the digestive system, barriers between the two substances erode, allowing them to mix and create a chemical reaction that pushes micro-needles of saccharides through the outer layer of the capsule and into the lining of the small intestine. The saccharide needles can be filled with drugs that are delivered into nearby blood vessels as the saccharide is absorbed.

In some embodiments, the dosage form for the glycan polymer preparations described herein employs a pH sensitive polymer coating. For example, pH-dependent polymers (bi- or triphasic) can be insoluble at low pH levels (e.g. acid resistance in the stomach, pH 1-2) and become increasingly soluble as pH rises, e.g. to about 5.5 - 6.2 in the duodenum, to about pH 5.7 in the ascending colon, to about pH 6.4 in the cecum, to about pH 6.6 in the transverse colon, to about pH 7.0 in the descending colon, to about 7.2 - 7.5 in the ileum, or to about pH 7.5 in the distal small intestine. In one example, TARGIT^{™} technology may be used for site-specific delivery of the glycan polymer preparations in the gastrointestinal (GI) tract. The system employs pH-sensitive coatings onto injection-moulded starch capsules to target the terminal ileum and colon.

In some embodiments, the dosage form for the glycan polymer preparations described herein is a delayed release system or time controlled release system. Such systems usually employ enteric coatings that may be combined with pH sensitive and time release functions. For example, ETP (enteric coated time-release press coated) tablets may be used that are composed of three components: a glycan polymer-containing core tablet (rapid release function), a presscoated, swellable hydrophobic polymer layer (e.g. hydroxypropyl cellulose layer (HPC), and a time release function. The duration of lag phase can be controlled either by weight or composition of polymer layer and an enteric coating layer (acid resistance function).

In some embodiments, the dosage form for the glycan polymer preparations described herein employs Eudragit^{®} enteric coatings of tablets and capsules. Other suitable synthetic polymers include: Shellac, ethyl cellulose, cellulose acetate phthalate, hydroxypropylmethyl cellulose, polyvinyl acetate phthalate and poly glutamic acid coatings, such as poly-γ-glutamic acid (γ-PGA). These coatings combine both mucoadhesive and pH-dependent release strategies. To enhance colon targeted delivery Eudragits^{®} are methacrylic co-polymers with varying side group compositions that alter the pH at which they are soluble. For example, for Eudragit^{®}coated systems no significant drug release occurs in the stomach (e.g. at pH 1.4) and in the small intestine (e.g. at pH 6.3), while significant drug release can be seen at pH 7.8 in the ileocaecal region.

In some embodiments, the dosage form for the glycan polymer preparations described herein is a microbial-triggered system, such as a polysaccharide based delivery system. Polysaccharide based delivery systems contain biodegradable and mucoadhesive polymer coatings, including coatings of chitosan and pectin. Other suitable natural polymers include, e.g., guar gum, inulin, cyclodextrin, dextran, amylase, chondrotin sulphate, and locust bean gum. These delivery systems can be used to target the glycan polymer to the small intestine. Coatings with naturally occurring polysaccharides like guar gum, xanthan gum, chitosan, alginates, etc. are degraded by colonic gut microbiota, e.g. enzymes such as, xylosidase, arabinosidase, galactosidase etc. For example, CODES^{™} technology may be used to deliver the glycan polymer preparations. This system combines the polysaccharide coating with a pH-sensitive coating. In some embodiments, the system consists of a core tablet coated with three layers of polymer coatings: The outer coating is composed of Eudragit L. This coating gets dissolved in the duodenum and exposes the next coating. The next coating is composed of Eudragit E. This layer allows the release of lactulose present in the inner core. The lactulose gets metabolized into short chain fatty acids that lower the surrounding pH where the Eudragit E layer dissolves. The dissolving of Eudragit E results in the exposure of the glycan polymer. The bacteria present in the colon are responsible for the degradation of polysaccharides that are released from the core tablet. The degradation of polysaccharides may result in organic acids formation that lowers the pH of the contents surrounding the tablet.

In some embodiments, the dosage form for the glycan polymer preparations described herein is a pressure-controlled delivery system. The system employs the fact that higher pressures are encountered in the colon than in the small intestine. For example, for ethylcellulose systems that are insoluble in water, the release of glycan polymers occurs following disintegration of a water-insoluble polymer capsule as a result of pressure in the lumen of the colon. The release profile may be adjusted by varying the thickness of the ethylcellulose, the capsule size and/or density of the capsule.

In some embodiments, the dosage form for the glycan polymer preparations described herein is a pulsatile colon targeted delivery system. For example, the system can be a pulsincap system. The capsule which is employed comprises a plug that is placed in the capsule that controls the release of the glycan polymer. A swellable hydrogel (e.g. hydroxyl propyl methyl cellulose (HPMC), poly methyl methacrylate or polyvinyl acetate) seals the drug content. When the capsule gets in contact with a fluid the plug is pushed off from the capsule and the glycan polymer is released. The release profile can be controlled by varying the length and/or point of intersection of the plug with the capsule body. Another system is a port system. The capsule body is enclosed in a semi-permeable membrane. The insoluble plug consists of an osmotically active agent and the glycan polymer. When the capsule gets in contact with a fluid the semi-permeable membrane permits inflow of the fluid which increases pressure in the capsule body. This leads to an expelling of the plug and release of the glycan polymer.

In some embodiments, the dosage form for the glycan polymer preparations described herein is an osmotically controlled colon targeted delivery system. An exemplary system, OROS-CT, consists of osmotic units (up to 5 or 6 push pull units) encapsulated in a hard gelatin capsule. The push pull units are bilayered with outer enteric impermeable membrane and inner semi-permeable membrane. The internal, central part of the push pull consists of the drug layer and push layer. The glycan polymer is released through the semi-permeable membrane. The capsule body enclosing the push pull units is dissolved immediately after administration. In the GI tract the enteric impermeable membrane prevents water absorption. The enteric coating is dissolved in small intestine (higher pH, >7), water enters the unit through the semi-permeable membrane causing push layer to swell and force out the glycan polymer.

In some embodiments, the dosage form for the glycan polymer preparations described herein is "smart pill" which can be used to release the glycan polymer just before reaching the ileocecal valve.

In some embodiments, the dosage form for the glycan polymer preparations described herein is a rectally administered formulation. For example, enemas introduce a glycan polymer preparation in liquid formulation into the rectum. The volume administered is typically less than 10 mL. Suppositories introduce a glycan polymer preparation into the rectum. Suppositories are solid dosage forms that melt or dissolve when inserted into the rectum, releasing the glycan polymers. Typical excipients for suppository formulations include cocoa butter, polyethylene glycols, and agar.

### Dosage forms

The glycan compositions for the recited use described herein may be formulated into any suitable dosage form, e.g. for oral or enteral administration or formulated for injection. Suitable dosage forms for use in the methods and compositions described herein can be found in WO 2016/122889, WO 2016/172657, and WO 2016/172658.

The dosage forms described herein can be manufactured using processes that are known to those of skill in the art. The dosage form may be suitable for any route of administration, including orally or parenterally, such as intravenously, intramuscularly, subcutaneously, intraorbitally, intracapsularly, intraperitoneally, intrarectally, intracisternally, intratumorally, intravasally, intradermally or by passive or facilitated absorption through the skin.

The dosage form may be a packet, such as any individual container that contains a glycan composition in the form of, e.g., a liquid (wash/rinse), a solid, a gel, a cream, an ointment, a powder, a tablet, a pill, a capsule, a lozenge, a suppository, a depository, a single-use applicator, a softgel or medical device (e.g. a syringe). For example, provided is also an article of manufacture, such as a container comprising a unit dosage form of the glycan composition, and a label containing instructions for use of such glycan composition.

The compositions provided herein can be in unit-dosage forms or multiple-dosage forms. A unit-dosage form, as used herein, refers to physically discrete unit suitable for administration to human in need thereof. In an embodiment, the unit-dosage form is provided in a package. Each unit-dose can contain a predetermined quantity of an active ingredient(s) sufficient to produce the desired therapeutic effect, in association with other pharmaceutical carriers or excipients. Examples of unit-dosage forms include ampoules, syringes, and individually packaged tablets and capsules. Unit-dosage forms can be administered in fractions or multiples thereof. A multiple-dosage form is a plurality of identical unit-dosage forms packaged in a single container, which can be administered in segregated unit-dosage form.

### Kits

Kits also are disclosed. For example, a kit can comprise unit dosage forms of the glycan polymer preparation, and a package insert containing instructions for use of the glycan polymer in treatment of a gastrointestinal disorder or condition. The kits include a glycan polymer preparation in suitable packaging for use by a subject in need thereof. Any of the compositions described herein can be packaged in the form of a kit. A kit can contain an amount of a glycan polymer preparation (optionally additionally comprising a prebiotic substance, a probiotic bacterium, and/or a second therapeutic agent) sufficient for an entire course of treatment, or for a portion of a course of treatment. Doses of a glycan polymer preparation can be individually packaged, or the glycan polymer preparation can be provided in bulk, or combinations thereof. Thus, in one disclosure, a kit provides, in suitable packaging, individual doses of a glycan polymer preparation that correspond to dosing points in a treatment regimen, wherein the doses are packaged in one or more packets.

In one disclosure, the glycan polymer preparation can be provided in bulk in a single container, or in two, three, four, five, or more than five containers. For example, \each container may contain enough of a glycan polymer preparation for a particular week of a treatment program that runs for a month. If more than one bulk container is provided, the bulk containers can be suitably packaged together to provide sufficient glycan polymer preparation for all or a portion of a treatment period. The container or containers can be labeled with a label indicating information useful to the subject in need thereof or the physician performing the treatment protocol, such as, e.g. dosing schedules.

The glycan polymer preparation can be packaged with other suitable substances, such as probiotic bacteria, prebiotic substances or other substances, as described herein. The other substance or substances can be packaged separately from the glycan polymer preparation, or mixed with the glycan polymer preparation, or combinations thereof. Thus, in one disclosure, kits include a dosage form containing all the ingredients intended to be used in a course of treatment or a portion of a course of treatment, e.g., a glycan polymer preparation and optionally buffers, excipients, etc., a probiotic, prebiotic or a polymer agent. In one disclosure, a glycan polymer preparation is packaged in one package or set of packages, and additional components, such as probiotic bacteria, prebiotics, and therapeutic agents are packaged separately from the glycan polymer preparation.

Kits can further include written materials, such as instructions, expected results, testimonials, explanations, warnings, clinical data, information for health professionals, and the like. In one disclosure, the kits contain a label or other information indicating that the kit is only for use under the direction of a health professional. The container can further include scoops, syringes, bottles, cups, applicators or other measuring or serving devices.

### Medical Food

Also provided herein are preparations of glycan polymers formulated as a medical food. Any glycan polymer preparation described herein may be formulated as a medical food as well as pharmaceutical compositions that comprise glycan polymer preparations.

A medical food is defined in section 5(b)(3) of the Orphan Drug Act (21 U.S.C. 360ee(b)(3)). Medical food is formulated to be consumed (oral intake) or administered enterally (e.g. feeding/nasogastric tube) under medical supervision, e.g. by a physician. It is intended for the specific dietary management of a disease or condition, such as, e.g. dysbiosis or a GI-tract disease. Medical foods as used herein do not include food that is merely recommended by a physician as part of an overall diet to manage the symptoms or reduce the risk of a disease or condition. Medical foods comprising a preparation of glycan polymers are foods that are synthetic (e.g., formulated and/or processed products, such as, being formulated for the partial or exclusive feeding of a patient by oral intake or enteral feeding by tube) and not naturally occurring foodstuff used in a natural state.

In some embodiments, the subject has limited or impaired capacity to ingest, digest, absorb, or metabolize ordinary foodstuffs or certain nutrients. In other embodiments, the subject has other special medically determined nutrient requirements, the dietary management of which cannot be achieved by the modification of the normal diet alone. Medical foods comprising a preparation of glycan polymers are administered to a subject in need thereof under medical supervision (which may be active and ongoing) and usually, the subject receives instructions on the use of the medical food. Medical foods may comprise one or more food additives, color additives, GRAS excipients and other agents or substances suitable for medical foods. Medical food preparations may be nutritionally complete or incomplete formulas.

### Dietary Supplements

Any glycan polymer preparation described herein may be formulated as a dietary supplement, e.g, for use in a method described herein. Dietary supplements are regulated under the Dietary Supplement Health and Education Act (DSHEA) of 1994. A dietary supplement is a product taken by mouth that contains a "dietary ingredient" intended to supplement the diet. The "dietary ingredients" in these products may include, in addition to a glycan polymer preparation described herein, one or more of: vitamins, minerals, herbs or other botanicals, amino acids, and substances such as enzymes, organ tissues, glandulars, and metabolites. Dietary supplements can also be extracts or concentrates, and may be found in many forms such as tablets, capsules, softgels, gelcaps, liquids, or powders. They can also be in other forms, such as a bar, but if they are, information on their label must not represent the product as a conventional food or a sole item of a meal or diet. DSHEA requires that every supplement be labeled a dietary supplement and not as a general food.

### Food Ingredient

Any glycan polymer preparation described herein may be formulated as a food ingredient or food additive, e.g, for use in a method described herein. Food ingredients may be generally recognized as safe (GRAS) or may require FDA authorization. Glycan polymer preparations can be added to any desireable food, e.g. beverages (incl., e.g., fruit juices), dairy products (e.g., milk, yogurt, cheese), cereals (any grain products), bread, spreads, etc.

A glycan preparation may be formulated as a food. The term "food" as defined in the Federal Food, Drug and Cosmetic Act (21 U.S.C. Section 321(a)(f)) refers to articles used for food or drink for man or other animals, chewing gum, and articles used for components of any such article. Food is formulated to be consumed (oral intake). Foods may comprise, in addition to a glycan preparation, one or more food additives, color additives, GRAS excipients and other agents or substances suitable for foods. Food preparations may be nutritionally complete or incomplete formulas.

### Methods of Modulating Microbial Taxa

The compounds and compositions provided herein may be used in methods to modulate a bacterial taxa (e.g. 1, 2, 3, 4, 5 or more taxa) present in the microbiota of a subject. In some embodiments, modulation comprises a change in the structure of the microbiota, such as a change in the relative composition of a taxa or a change in the relative abundance of a taxa, e.g., relative to another taxa or relative to what would be observed in the absence of the modulation. In other embodiments, modulation comprises a change in a function of the microbiota, such as a change in gene expression, level of a gene product (e.g., RNA or protein), or metabolic output of the microbiota, or a change in a functional pathway of the host (e.g, a change in gene expression, level of a gene product, or metabolic output of a host cell or host process). Methods of modulating microbial taxa disclosed in WO 2016/122889 and WO 2016/172657, are suitable for use in methods described herein.

The methods describe herein include administering to a subject a composition described herein, e.g., comprising a glycan composition described herein, in an amount effective to modulate taxa. In some embodiments, the abundance of a bacterial taxa may increase relative to other taxa (or relative from one point in time to another) when the composition is administered and the increase can be at least a 5%, 10%, 25% 50%, 75%, 100%, 250%, 500%, 750% increase or at least a 1000% increase. The abundance of a bacterial taxa may also decrease relative to other taxa (or relative from one point in time to another) when the composition is administered and the decrease can be at least a 5%, 10%, 25% 50%, 75%, 85%, 90%, 95%, 96%, 97%, 98%, 99% decrease, or at least a 99.9% decrease. Administration of the composition can modulate the abundance of the desired and/or non-desired bacterial taxa in the subject's gastrointestinal microbiota.

In some embodiments, the composition described herein, e.g., comprising a glycan composition described herein, modulates (e.g. increases or decreases) the growth of one or more bacterium, such as, e.g., those that belong to genera Bacteroides, Odoribacter, Parabacteroides, Alistipes, Blautia, Clostridium, Coprococcus, Dorea, Eubacterium, Lachnospira, Roseburia, Ruminococcus, Faecalibacterium, Oscillospira, and Subdoligranulum which can be found in the GI tract. In some embodiments, the composition described herein, e.g., comprising a glycan composition described herein, modulates (e.g. increases or decreases) the growth of one or more bacterium, such as, e.g., of the genus Akkermansia, Anaerofilum, Bacteroides, Blautia, Bifidobacterium, Butyrivibrio, Clostridium, Coprococcus , Dialister, Dorea, Fusobacterium, Eubacterium, Faecalibacterium, Lachnospira, Lactobacillus, Phascolarctobacterium, Peptococcus, Peptostreptococcus, Prevotella, Roseburia, Ruminococcus, and Streptococcus, and/or one or more of the species Akkermansia municiphilia, Christensenella minuta, Clostridium coccoides, Clostridium leptum, Clostridium scindens, Dialister invisus, Eubacterium rectal, Eubacterium eligens, Faecalibacterium prausnitzii, Streptococcus salivarius, and Streptococcus thermophilus.

In some embodiments, the composition described herein, e.g., comprising a glycan composition described herein modulates (e.g. increases or decreases) the growth of at least two bacterial taxa selected from Prevotella, Akkermansia, Bacteroides, Clostridium (Erysipelotrichaceae), Clostridium (Clostridiaceae), Bifidobacterium, Aggregatibacter, Clostridium (Peptostreptococcaveae), Parabacteroides, Lactobacillus, and Enterococcus.

In some embodiments, a composition described herein, e.g., comprising a glycan composition described herein, modulates (e.g. increases or decreases) the growth of one or more bacterial taxa residing in the GI tract, such as, e.g., those that belong to genera Bacteroides, Odoribacter, Parabacteroides, Alistipes, Blautia, Clostridium, Coprococcus, Dorea, Eubacterium, Lachnospira, Roseburia, Ruminococcus, Faecalibacterium, Oscillospira, and Subdoligranulum which can be found in the GI tract. In some embodiments, a composition described herein, e.g., comprising a glycan composition described herein, modulates (e.g. increases or decreases) the growth of one or more bacterial taxa, such as those that are thought to be associated with a healthy gastrointestinal state, e.g., one or more of the genus Akkermansia, Anaerofilum, Bacteroides, Blautia, Bifidobacterium, Butyrivibrio, Clostridium, Coprococcus , Dialister, Dorea, Fusobacterium, Eubacterium, Faecalibacterium, Lachnospira, Lactobacillus, Phascolarctobacterium, Peptococcus, Peptostreptococcus, Prevotella, Roseburia, Ruminococcus, and Streptococcus, and/or one or more of the species Akkermansia municiphilia, Christensenella minuta, Clostridium coccoides, Clostridium leptum, Clostridium scindens, Dialister invisus, Eubacterium rectal, Eubacterium eligens, Faecalibacterium prausnitzii, Streptococcus salivarius, and Streptococcus thermophilus. In some embodiments, the composition described herein, e.g., comprising a glycan composition described herein, modulates (e.g. increases or decreases) the growth of one or more bacterial taxa, such as taxa of the phylum Verrucomicrobia, e.g., those of the genus Akkermansia.

In some embodiments, a composition described herein, e.g., comprising a glycan composition described herein, modulates (e.g. increases or decreases) the growth of one or more bacterial taxa predominantly residing in the small intestine. For example, the composition described herein, e.g., comprising a glycan composition described herein, modulates one or more (2, 3, 4, 5, 6, 7, 8, 9, 10 or more) bacterial taxa that reside predominantly in the small intestine, such as, e.g. Actinobacteria, Firmicutes (Bacilli, Clostridia), and Proteobacteria (Alphaproteobacteria, Betaproteobacteria). In some embodiments, a composition described herein, e.g., comprising a glycan composition described herein, modulates one or more (2, 3, 4, 5, 6, 7, 8, 9, 10 or more) bacterial taxa that reside predominantly in the small intestine selected from the genera: Cryocola, Mycobacterium, Enterococcus, Lactococcus, Streptococcus, Turicibacter, Blautia, Coprococcus, Holdemania, Pseudoramibacter Eubacterium, Agrobacterium, Sphingomonas, Achromobacter, Burkholderia, and Ralstonia.

In some embodiments, a composition described herein, e.g., comprising a glycan composition described herein, modulates (e.g. increases or decreases) the growth of one or more bacterial taxa predominantly residing in the large intestine. For example, a composition described herein, e.g., comprising a glycan composition described herein, modulates one or more (2, 3, 4, 5, 6, 7, 8, 9, 10 or more) bacterial taxa that reside predominantly in the large intestine, such as, e.g. Bacteroidetes, Firmicutes (Clostridia), Verrucomicrobia, and Proteobacteria (Deltaproteobacteria). In some embodiments, a composition described herein, e.g., comprising a glycan composition described herein, modulates one or more (2, 3, 4, 5, 6, 7, 8, 9, 10 or more) bacterial taxa that reside predominantly in the large intestine selected from the genera: Bacteroides, Butyricimonas, Odoribacter, Parabacteroides, Prevotella, Anaerotruncus, Phascolarctobacterium, Ruminococcus, Bilophila, and Akkermansia.

In some embodiments, the composition described herein, e.g., comprising a glycan composition described herein, modulates (e.g. increases or decreases) the growth of one or more bacterial taxa predominantly residing in the cecum, such as, e.g. Actinobacteria, Bacteroides, Bacilli, Clostridia, Mollicutes, Alpha Proteobacteria, and Verrucomicrobia.

In some embodiments, a composition described herein, e.g., comprising a glycan composition described herein, modulates (e.g. increases or decreases) the growth of one or more bacterial taxa predominantly residing in the ascending colon, such as, e.g. Actinobacteria, Bacteroides, Bacilli, Clostridia, Fusobacteria, Beta Proteobacteria, DeltalEpsilon Proteobacteria, Gamma Proteobacteria, and Verrucomicrobia.

In some embodiments, the composition described herein, e.g., comprising a glycan composition described herein, modulates (e.g. increases or decreases) the growth of one or more bacterial taxa predominantly residing in the traverse colon, such as, e.g. Actinobacteria, Bacteroides, Clostridia, Mollicutes, Fusobacteria, and Gamma Proteobacteria.

In some embodiments, a composition described herein, e.g., comprising a glycan composition described herein, modulates (e.g. increases or decreases) the growth of one or more bacterial taxa predominantly residing in the descending colon, such as, e.g. Bacteroides, Clostridia, Mollicutes, Fusobacteria, DeltalEpsilon Proteobacteria and Verrucomicrobia.

In some embodiments, a composition described herein, e.g., comprising a glycan composition described herein, modulates (e.g. increases or decreases) the growth of one or more bacterial taxa predominantly residing in the sigmoid colon, such as, e.g. Actinobacteria, Bacteroides, Bacilli, Clostridia, Mollicutes, Alpha Proteobacteria, Beta Proteobacteria, and Verrucomicrobia.

In some embodiments, a composition described herein, e.g., comprising a glycan composition described herein, modulates (e.g. increases or decreases) the growth of one or more bacterial taxa predominantly residing in the rectum, such as, e.g. Bacteroides, Clostridia, Mollicutes, Alpha Proteobacteria, Gamma Proteobacteria, and Verrucomicrobia.

In some embodiments, a composition described herein, e.g., comprising a glycan composition described herein, modulates (e.g. stimulate/increase or suppress/decrease) the growth of one or more (e.g. 1, 2, 3, 4, 5, 6, 7, 8, 9, 10, or more) bacterial taxa of genera including, e.g. Alistipes, Akkermansia, Anaerofilum, Bacteroides, Blautia, Bifidobacterium, Butyrivibrio, Clostridium, Coprococcus , Dialister, Dorea, Fusobacterium, Eubacterium, Faecalibacterium, Lachnospira, Lactobacillus, , Odoribacter, Oscillospira, Parabacteroides, Phascolarctobacterium, Peptococcus, Peptostreptococcus, Prevotella, Roseburia, Ruminococcus, and Streptococcus and Subdoligranulum.

In some embodiments, a composition described herein, e.g., comprising a glycan composition described herein, modulates (e.g. stimulate/increase or suppress/decrease) the growth of one or more (e.g. 1, 2, 3, 4, 5, 6, 7, 8, 9, 10, or more) microbial taxa of genera Akkermansia, Anaerofilum, Bacteroides, Blautia, Bifidobacterium, Butyrivibrio, Clostridium, Coprococcus , Dialister, Dorea, Fusobacterium, Eubacterium, Faecalibacterium, Lachnospira, Lactobacillus, Phascolarctobacterium, Peptococcus, Peptostreptococcus, Prevotella, Roseburia, Ruminococcus, and Streptococcus and of the species Akkermansia municiphilia, Christensenella minuta, Clostridium coccoides, Clostridium leptum, Clostridium scindens, Dialister invisus, Eubacterium rectal, Eubacterium eligens, Faecalibacterium prausnitzii, Streptococcus salivarius, and Streptococcus thermophilus.

In some embodiments, a composition described herein, e.g., comprising a glycan composition described herein, modulates (e.g. substantially increase or substantially decrease) the growth (and the total number) of (or substantially increase or substantially decrease the relative representation/abundance in the total (gastrointestinal) community) of one or more of (e.g. 1, 2, 3, 4, 5, 6, 7, 8, 9, 10, or more) bacterial taxa listed in Tables 1-3 or 4-6.

In some embodiments, a composition described herein, e.g., comprising a glycan composition described herein, substantially increases the growth, e.g. the total number or the relative representation/abundance in the total (gastrointestinal) community) of one or more of (e.g. 1, 2, 3, 4, 5, 6, 7, 8, 9, 10, or more) bacterial taxa listed in Tables 1-3 or 4-6.

In some embodiments, a composition described herein, e.g., comprising a glycan composition described herein, substantially decreases the growth, e.g. the total number or the relative representation/abundance in the total (gastrointestinal) community) of one or more of (e.g. 1, 2, 3, 4, 5, 6, 7, 8, 9, 10, or more) bacterial taxa listed in Tables 1-3 or 4-6.

In some embodiments, a composition described herein, e.g., comprising a glycan composition described herein, substantially increases and decreases the growth, e.g. the total number or the relative representation/abundance in the total (gastrointestinal) community) of one or more of (e.g. 1, 2, 3, 4, 5, 6, 7, 8, 9, 10, or more) bacterial taxa listed in Tables 1-3 or 4-6.

In certain embodiments, the ratio of certain bacterial taxa or their relative abundance may be shifted. Such shifts may be measured with respect to the ratio present in the subject prior to administration of the pharmaceutical glycan composition, or to a control group not taking the pharmaceutical glycan composition.

### Proteomic Analysis of Microbial Populations

Preparations of glycan compositions may be selected based on their ability to modulate the expression of microbial proteins, e.g., enzymes, associated with the processing of exogenous substances as described, e.g., in Tables 1-3. Suitable methods for proteomic analysis of microbial populations can be found in WO 2016/122889 and WO 2016/172657. In some embodiments, proteomic analysis can be performed following protocols described in e.g., Cordwell, Exploring and exploiting bacterial proteomes, Methods in Molecular Biology, 2004, 266:115.

### Identification of microbial (e.g. bacterial) constituents

Microbial modulation (e.g., of representation/abundance of a taxa) by the glycan compositions described herein, e.g., occurring in vivo in the GI tract can be analyzed using any number of methods known in the art and described herein. Suitable methods can be found in WO WO 2016/122889, WO 2016/172657, and WO 2016/172658. In some embodiments, quantitative PCR (qPCR) can be used as a method to determine whether a glycan composition can result in a shift of the population of bacteria in the GI tract.

In some embodiments, microbial constituents can be identified by characterizing the DNA sequence of microbial 16S small subunit ribosomal RNA gene (16S rRNA gene). In other embodiments, a microbial composition can be identified by characterizing nucleotide markers or genes, in particular highly conserved genes (e.g., "house-keeping" genes), or a combination thereof, or whole genome shotgun sequence (WGS).

### Administration to a subject

The glycan compsitions, pharmaceutical compositions and therapeutic agents described herein can be administered to a subject in need thereof by various routes (e.g., systemically or locally) including, for example, orally or parenterally, such as intravenously, intramuscularly, subcutaneously, intraorbitally, intracapsularly, intraperitoneally, intrarectally, intracisternally, intratumorally, intravasally, intradermally or by passive or facilitated absorption through the skin. The therapeutic agents can be administered locally to the site of a pathologic condition, for example, intravenously or intra-arterially into a blood vessel supplying a tumor. In some embodiments, the glycan composition is administered enterically. This includes oral administration, or by an oral or nasal tube (including nasogastric, nasojejunal, oral gastric, or oral jejunal). In other embodiments, administration includes rectal administration (including enema, suppository, or colonoscopy). Methods of administering to a subject sutiable for use with methods and compositions described herein can be found in WO 2016/122889, WO 2016/172657, and WO 2016/172658.

Active compounds and pharmaceutical agents, e.g., prebiotic substances, probiotic bacteria or drugs, may be administered separately, e.g., prior to, concurrent with or after administration of the glycan compositions and not as a part of the pharmaceutical composition or medical food or dietary supplement (e.g. as a co-formulation) of glycan compositions. In some embodiments, pharmaceutical compositions or medical foods comprising preparations of glycan compositions are administered in combination with a recommended or prescribed diet, e.g. a diet that is rich in probiotic and/or prebiotic-containing foods, such as it may be determined by a physician or other healthcare professional.

### Methods of treating

Provided herein are methods of treating a disease, disorder, condition, or pathological condition comprising administering to a subject in need thereof a glycan composition, e.g., a glycan composition described herein. Also provided herein are exogenous substances (e.g., pharmaceutical agents) for use in any method described herein. The exogenous substance can be included in a composition comprising the glycan composition, or can be administered/formulated separately. In embodiments, the pharmaceutical agent is used to treat a disease, disorder, condition, or pathological condition described herein.

Diseases and disorders that can be treated with methods and compositions described herein can be found in WO 2016/122889, WO 2016/172657, and WO 2016/172658. Exemplary diseases, disorders, conditions, or pathological conditions can include but are not limited to: a proliferative disease (e.g., cancer), a dysbiosis, an infectious disease, a metabolic disease, a neurodegenerative disease, an allergy, and the like.

Diseases, disorders, and conditions that can be treated with methods described herein include: pain, migraines, arthritis, cancer, colon and rectum cancer, bacterial infection, viral infection, HIV, hepatitis, hepatitis C, fungal infection, nematode infection, hookworm infection, osteoporosis, pain related to cancer, diabetes, blood sugar imbalance, seizures, panic disorder, anxiety, heart conditions, heart failure, arrhythmia, high blood pressure, angina, chest pain, thrombocytopenia, aplastic anemia, Parkinson's disease and Parkinson's like symptoms, diarrhea, high cholesterol and triglyceride levels, ADHD, recreational drug use, insomnia, ulcers, gastroesophageal reflux disease (GERD), heart burn, drug toxicity (e.g., 5-fluorouracil-induced gastrointestinal toxicity), schizophrenia, bipolar disorder, irritability caused by autism, constipation, and epilepsy.

In some embodiments, the pharmaceutical composition comprising a glycan composition is administered prior to, concomitant with or after administration of the (e.g. anti-cancer) drug or non-drug (e.g., anti-cancer) treatment, administration of which induces the symptoms.

In one embodiment, a method of lowering toxicity of a drug treatment (e.g., an anti-cancer drug treatment) in a subject is provided. The method includes: a) administering a pharmaceutical composition comprising a glycan composition to a subject who has received the drug treatment; b) administering the drug treatment to a subject who has been treated with a pharmaceutical composition comprising a glycan composition; or c) administering a pharmaceutical composition comprising a glycan composition and administering the drug treatment, to a subject, thereby treating the subject. In some embodiments, the toxicity is dose-limiting toxicity. In some embodiments, the method increases the tolerance of the subject to drug treatment, e.g. an anti-cancer drug treatment.
In some embodiments, dose limiting toxicity prevents subjects from being treated with the maximal efficacious dose of a drug. As one example of dose-limiting toxicity, diarrhea can be caused by the chemotherapy drugs irinotecan and 5-fluoruracil. In some embodiments, glycan composition are administered to treat dose limiting toxicity, e.g., to increase the dose that is tolerated by the subject. In some embodiments, tolerability is increased by limiting one or more digestive abnormalities associated with the respective efficacious drug dose.

### EXAMPLES

The invention is further illustrated by the following examples. The examples are provided for illustrative purposes only, and are not to be construed as limiting the scope or content of the invention in any way. The practice of the present invention will employ, unless otherwise indicated, conventional methods of protein chemistry, biochemistry, recombinant DNA techniques and pharmacology, within the skill of the art. Such techniques are explained fully in the literature. See, e.g., T.E. Creighton, Proteins: Structures and Molecular Properties (W.H. Freeman and Company, 1993); Green & Sambrook et al., Molecular Cloning: A Laboratory Manual, 4th Edition (Cold Spring Harbor Laboratory Press, 2012); Colowick & Kaplan, Methods In Enzymology (Academic Press); Remington: The Science and Practice of Pharmacy, 22nd Edition (Pharmaceutical Press, 2012); Sundberg & Carey, Advanced Organic Chemistry: Parts A and B, 5th Edition (Springer, 2007).

### Example 1: Effect of glycans on microbial populations ex vivo

To determine the desired composition of glycans, bacterial cultures were grown in the presence of candidate glycans and assayed for their growth, community composition (e.g., by 16S rRNA gene sequencing), production of metabolites, and phenotypic or transcriptomic properties. Desired glycans were selected based on their ability to elicit desired properties within the bacterial culture. Bacterial cultures include monocultures, mixed cultures, cultures isolated from humans or laboratory animal models, cultures isolated from a human or laboratory animal model and spiked with an isolate or collection of isolates, or cultures isolated from a human or laboratory animal model and depleted of a collection of species (for example, by application of an antibiotic). This assay can be performed in the presence of antibiotics or other test compounds. The results obtained from the in vitro assays are compared with those obtained after treating humans with glycans or administering the glycans to a laboratory animal establishing the in vitro - in vivo correlation of results.

### Example 2: Growth of beta-glucuronidase-associated strains, non-associated strains, and other gut commensals on several glycans

An *in vitro* assay was performed to assess the ability of various bacterial strains, including commensals of the gastrointestinal tract, to utilize different glycans as growth substrates. This assay was designed to assess the ability of selected glycans to promote the growth of microbiota associated with beta-glucuronidase production, those not associated with beta-glucuronidase production, and other gut commensals. Lactobacillus gasseri was handled aerobically, and all other bacterial strains were handled at all steps in an anaerobic chamber (AS-580, Anaerobe Systems) featuring a palladium catalyst. The chamber was initially made anaerobic by purging with an anaerobic gas mixture of 5% hydrogen, 5% carbon dioxide and 90% nitrogen and subsequently maintained in an anaerobic state using this same anaerobic gas mixture. Anaerobicity of the chamber was confirmed daily using Oxoid anaerobic indicator strips that change color in the presence of oxygen. All culture media, assay plates, other reagents and plastic consumables were pre-reduced in the anaerobic chamber for 24-48 hours prior to contact with bacteria. Glycans glu33gal33fuc33, gal100, glu50gal50, man80glu20, man60glu40, man80gal20, glu100, man100, man52glu29gal19, man66gal33, xyl75ara25, glu80man20 and glu60man40, a commercially available control, FOS (Nutraflora FOS; NOW Foods, Bloomingdale IL), and dextrose were prepared at 5% w/v in water, filter-sterilized and added to Costar 3370 assay plates for a final concentration of 0.5% w/v in the assay, with each glycan assayed in 2-4 non-adjacent wells and dextrose and water supplied as positive and negative controls.

Bacterial isolates were obtained from the American Type Culture Collection (ATCC) and Leibniz Institute DSMZ-German Institute of Microorganisms and Cell Cultures (DSMZ). Cultures of strains *Ruminococcus obeum* ATCC 29714 "ROB.74", *Bacteroides caccae* ATCC 43185 "BCA.26", *Bacteroides thetaiotaomicron* ATCC 29741 "BTH.8", *Bacteroides cellulosilyticus* DSM 14838 "BCE.71", *Parabacteroides distasonis* ATCC 8503 "PDI.6", *Bacteroides vulgatus* ATCC 8482 "BVU.10", *Clostridium scindens* ATCC 35704 "CSC.32", *Dorea formicigenerans* ATCC 27755 "DFO.36", and the Bifidobacteria *Bifidobacterium longum* ATCC 15707 "BLO.16" and *Bifidobacterium longum* DSM 20088 "BLO.83", were grown anaerobically in Chopped Meat Glucose broth (CMG, Anaerobe Systems), a pre-reduced enriched medium including lean ground beef, enzymatic digest of casein, yeast extract, potassium phosphate, dextrose, cysteine, hemin and Vitamin K1, for 18-48 hours at 37° C. Cultures of Lactobacillus gasseri ATCC 33323 "LGA.44" were grown aerobically in CMG for 18-48 hours at 37° C. Inocula were prepared by determining the optical density of each culture at 600 nM (OD600) in a Costar 3370 polystyrene 96-well flat-bottom assay plate using a Biotek Synergy 2 plate reader with Gen5 2.0 All-In-One Microplate Reader Software according to manufacturer's protocol, and diluting the cells to OD600 0.01 final in defined and semi-defined media that were prepared without sugars. *B. vulgatus, D. formicigenerans, P. distasonis,* and *B. longum* isolates were tested in 900 mg/L sodium chloride, 26 mg/L calcium chloride dihydrate, 20 mg/L magnesium chloride hexahydrate, 10 mg/L manganese chloride tetrahydrate, 40 mg/L ammonium sulfate, 4 mg/L iron sulfate heptahydrate, 1 mg/L cobalt chloride hexahydrate, 300 mg/L potassium phosphate dibasic, 1.5 g/L sodium phosphate dibasic, 5 g/L soidum bicarbonate, 0.125 mg/L biotin, 1 mg/L pyridoxine, 1 m/L pantothenate, 75 mg/L histidine, 75 mg/L glycine, 75 mg/L tryptophan, 150 mg/L arginine, 150 mg/L methionine, 150 mg/L threonine, 225 mg/L valine, 225 mg/L isoleucine, 300 mg/L leucine, 400 mg/L cysteine, and 450 mg/L proline (Theriot CM et al. Nat Commun. 2014; 5:3114), supplemented with 0-5% (v/v) CMG. *B. thetaiotaomicron, B. caccae* and *B. cellulosyliticus* were tested in 100 mM potassium phosphate buffer (pH 7.2), 15 mM sodium chloride, 8.5 mM ammonium sulfate, 4 mM L-cysteine, 1.9 µM hematin, 200 µM L-histidine, 100 µM magnesium chloride, 1.4 µM iron sulfate heptahydrate, 50 µM calcium chloride, 1 µg/mL vitamin K3 and 5 ng/mL vitamin B12 (Martens EC et al. Cell Host & Microbe 2008; 4, 447-457). *L. gasseri, C. scindens* and *R. obeum* were tested in 10 g/L tryptone peptone, 5 g/L yeast extract, 0.5 g/L L-cysteine hydrochloride, 0.1 M potassium phosphate buffer pH 7.2, 1 µg/mL vitamin K3, 0.08% w/v calcium chloride, 0.4 µg/mL iron sulfate heptahydrate, 1 µg/mL resazurin, 1.2 µg/mL hematin, 0.2 mM histidine, 0.05% Tween 80, 0.5% meat extract (Sigma), 1% trace mineral supplement (ATCC), 1% vitamin supplement (ATCC), 0.017% v/v acetic acid, 0.001% v/v isovaleric acid, 0.2% v/v propionic acid and 0.2% v/v N-butyric acid (Romano KA et al. mBio 2015; 6(2):e02481-14) supplemented with 0-5% CMG. Bacteria were exposed to glycans ara50gal50, glu33gal33fuc33, glu50gal50, gal100, glu100, xyl100, ara100, ara60xyl40, glu80man20, glu60man40, man52glu29gal19, man100, xyl75ara25, commercial FOS and dextrose at a final concentration of 0.5% w/v in 96-well microplates, 200 µL final volume per well, at 37° C for 18-48 hours, anaerobically. OD600 measurements for each isolate at the end of the incubation period were obtained using a Biotek Synergy2 reader with Gen5 2.0 software according to manufacturer's specifications.

Measurements were normalized by dividing the OD600 readings of the isolate on test glycans by the average OD600 of the isolate in medium supplemented with 0.5% w/v dextrose to facilitate comparison of glycan utilization by strains that grow within significantly different OD600 ranges. Table 8 summarizes the results obtained.

**Table 8: Growth of beta-glucuronidase-associated strains, non-associated strains, and other gut commensals on glycans.**

| | **Gut Commensals, Average Normalized Growth Values** | | | | | | | | | | |
|---|---|---|---|---|---|---|---|---|---|---|---|
| | **β-Glucuronidase-Associated** | | | | **Non-Associated** | | | **Other** | | | |
| **ID** | **LG A.44** | **ROB. 74** | **BLO. 16** | **BLO. 83** | **BTH. 8** | **BVU. 10** | **DFO. 36** | **BCA. 26** | **BCE. 71** | **CSC. 32** | **PDI. 6** |
| 1 | - | - | - | - | ++ | + | + | ++ | + | - | ++ |
| 2 | - | - | - | - | + | - | - | +++ | + | + | ++ |
| 3 | - | - | - | - | ++ | - | - | - | - | - | + |
| 4 | + | - | - | - | +++ | - | - | + | + | + | +++ |
| 5 | + | - | - | - | ++ | + | + | +++ | ++ | - | +++ |
| 6 | + | - | - | - | +++ | + | - | ++ | ++ | - | +++ |
| 7 | + | - | - | - | ++ | - | + | ++ | ++ | - | ++ |
| 8 | + | - | - | - | ++ | - | - | + | + | - | +++ |
| 9 | + | + | - | - | +++ | + | + | +++ | ++ | - | +++ |
| 10 | + | + | - | - | +++ | + | - | +++ | ++ | - | +++ |
| 11 | - | - | - | - | + | - | - | + | + | - | - |
| FOS | - | +++ | +++ | +++ | ++ | +++ | + | +++ | +++ | + | +++ |
| 12 | ++ | ++ | ++ | + | +++ | ++ | +++ | +++ | +++ | + | +++ |
| 13 | ++ | + | + | - | +++ | ++ | ++ | ++ | ++ | + | +++ |

*L. gasseri, R. obeum* and *B. longum* belong to the same species or genera as reported β-glucuronidase producers and thus are associated with β-glucuronidase production (Russell, W.M. and Klaenhammer, T.R. Identification and cloning of gusA, encoding a new β-glucuronidase from Lactobacillus gasseri ADH. Appl Environ. Microbiol. 2001; Beaud et al, Genetic characterization of the β-glucuronidase enzyme from a human intestinal bacterium, Ruminococcus gnavus. Microbiology 2005; Roy, D & Ward, P. Rapid detection of Bifidobacterium dentium by enzymatic hydrolysis of β-glucuronide substrates. J Food Protect. 1992.). *B. thetaiotaomicron* and *B. vulgatus* reportedly have no detectable β-glucuronidase activity *in vitro* (Dabek et al, Distribution of β-glucosidase activity and of β-glucuronidase gene gus in human colonic bacteria. FEMS Microbiol Ecol 2008), and no β-glucuronidase is found in the NCBI protein database for Dorea species, including *D. formicigenerans;* consequently, these 3 isolates are considered to be non-associated with β-glucuronidase production.

In the assay, a number of glycans supported growth of non-β-glucuronidase associated strains and other gut commensals better than β-glucuronidase associated strains, producing relatively low average normalized growth values with the β-glucuronidase associated strains.

Glu33gal33fuc33 and gal100 did not support growth of any of the β-glucuronidase associated strains in the assay but supported growth of 5-6 non- β-glucuronidase associated and other gut commensal strains with average normalized growth values greater than 0.15. In the assay, glu50gal50, man80glu20, man60glu40, man80gal20, glu100, man100, man52glu29gal19 and man66gal33 supported normalized growth values of at least 0.3 with the non-β-glucuronidase associated strain *B. thetaiotaomicron* and with 1-3 other gut commensals, while they supported no relatively weak to no growth of β-glucuronidase producers, with average normalized growth values <0.3 for *L. gasseri* and <0.15 for *R. obeum* and *B. longum.*

Bacterial β-glucuronidases are associated with toxicity of some drugs. For example, microbial β-glucuronidases in the gut convert a non-toxic form of the cancer drug irinotecan to a form that exhibits toxicity toward intestinal epithelial cells (Spanogiannopoulos P. et al. The microbial pharmacists within us: a metagenomics view of xenobiotic metabolism. Nature Reviews Microbiology vol 14, May 2016). Administering a glycan that selectively supports growth of non-β-glucuronidase associated strains to a patient undergoing irinotecan chemotherapy may reduce the relative abundance of β-glucuronidase-producing strains in the gut and thereby reduce irinotecan-associated diarrhea.

**Table 9: Key to Example 2 Glycans**

| **Key to Glycans** | |
|---|---|
| **glycan #** | **composition** |
| 1 | glu33gal33fuc33 |
| 2 | gal100 |
| 3 | glu50gal50 |
| 4 | man80glu20 |
| 5 | man60glu40 |
| 6 | Man80gal20 |
| 7 | glu 100 |
| 8 | man 100 |
| 9 | man52glu29gal19 |
| 10 | Man66gal33 |
| 11 | xyl75ara25 |
| 12 | glu80man20 |
| 13 | glu60man40 |

**Table 10: Key to Example 2 Symbols**

| **Symbol** | **NGV** |
|---|---|
| - | <0.15 |
| + | 0.15-0.3 |
| ++ | 0.3-0.6 |
| +++ | >0.6 |

### Example 3: Glycan-supported growth of bacteria associated with phytoestrogen metabolism

An *in vitro* assay was performed to assess the ability of various bacterial strains, including commensals of the gastrointestinal tract, to utilize different glycans as growth substrates. This assay was designed to assess the ability of selected glycans to promote the growth of microbiota associated with phytoestrogen metabolism, which has been associated with protective effects against breast cancer. Bacterial strains were handled at all steps in an anaerobic chamber (AS-580, Anaerobe Systems) featuring a palladium catalyst. The chamber was initially made anaerobic by purging with an anaerobic gas mixture of 5% hydrogen, 5% carbon dioxide and 90% nitrogen and subsequently maintained in an anaerobic state using this same anaerobic gas mixture. Anaerobicity of the chamber was confirmed daily using Oxoid anaerobic indicator strips that change color in the presence of oxygen. All culture media, assay plates, other reagents and plastic consumables were pre-reduced in the anaerobic chamber for 24-48 hours prior to contact with bacteria. Glycans glu80man20, glu60man40, glu100, gal100, man80glu20, glu33gal33fuc33, man60glu40, man80gal20, man66gal33, man100, man52glu29gal19, xyl75ara25 and xyl100 were prepared at 5% w/v in water, filter-sterilized and added to Costar 3370 assay plates for a final concentration of 0.5% w/v in the assay, with each glycan assayed in two non-adjacent wells and dextrose and water supplied as positive and negative controls.

Bacterial isolates were obtained from the American Type Culture Collection (ATCC) and Leibniz Institute DSMZ-German Institute of Microorganisms and Cell Cultures (DSMZ). Cultures of *Blautia producta* ATCC 27340 "BPR.22", *Clostridium scindens* ATCC 35704 "CSC.32", *Enterococcus faecium* ATCC 700221 "EFM.66" and the Bifidobacteria *Bifidobacterium longum* ATCC 15707 "BLO.16" and *Bifidobacterium longum* DSM 20088 "BLO.83", were grown anaerobically in Chopped Meat Glucose broth (CMG, Anaerobe Systems), a pre-reduced enriched medium including lean ground beef, enzymatic digest of casein, yeast extract, potassium phosphate, dextrose, cysteine, hemin and Vitamin K1, for 18-48 hours at 37 °C. Inocula were prepared by determining the optical density of each culture at 600 nM (OD₆₀₀) in a Costar 3370 polystyrene 96-well flat-bottom assay plate using a Biotek Synergy 2 plate reader with Gen5 2.0 All-In-One Microplate Reader Software according to manufacturer's protocol, and diluting the cells to OD₆₀₀ 0.01 final in defined and semi-defined media that were prepared without sugars. *B. producta, E. faecium* and *B. longum* isolates were tested in 900 mg/L sodium chloride, 26 mg/L calcium chloride dihydrate, 20 mg/L magnesium chloride hexahydrate, 10 mg/L manganese chloride tetrahydrate, 40 mg/L ammonium sulfate, 4 mg/L iron sulfate heptahydrate, 1 mg/L cobalt chloride hexahydrate, 300 mg/L potassium phosphate dibasic, 1.5 g/L sodium phosphate dibasic, 5 g/L soidum bicarbonate, 0.125 mg/L biotin, 1 mg/L pyridoxine, 1 m/L pantothenate, 75 mg/L histidine, 75 mg/L glycine, 75 mg/L tryptophan, 150 mg/L arginine, 150 mg/L methionine, 150 mg/L threonine, 225 mg/L valine, 225 mg/L isoleucine, 300 mg/L leucine, 400 mg/L cysteine, and 450 mg/L proline (Theriot CM et al. Nat Commun. 2014; 5:3114), supplemented with 0-5% (v/v) CMG. *C. scindens* was tested in 10 g/L tryptone peptone, 5 g/L yeast extract, 0.5 g/L L-cysteine hydrochloride, 0.1 M potassium phosphate buffer pH 7.2, 1 µg/mL vitamin K3, 0.08% w/v calcium chloride, 0.4 µg/mL iron sulfate heptahydrate, 1 µg/mL resazurin, 1.2 µg/mL hematin, 0.2 mM histidine, 0.05% Tween 80, 0.5% meat extract (Sigma), 1% trace mineral supplement (ATCC), 1% vitamin supplement (ATCC), 0.017% v/v acetic acid, 0.001% v/v isovaleric acid, 0.2% v/v propionic acid and 0.2% v/v N-butyric acid (Romano KA et al. mBio 2015; 6(2):e02481-14). Bacteria were exposed to glu80man20, glu60man40, glu100, gal100, man80glu20, glu33gal33fuc33, man60glu40, man80gal20, man66gal33, man100, man52glu29gal19, xyl75ara25, xyl100 and dextrose at a final concentration of 0.5% w/v in 96-well microplates, 200 µL final volume per well, at 37 °C for 18-48 hours, anaerobically. OD₆₀₀ measurements for each isolate at the end of the incubation period were obtained using a Biotek Synergy2 reader with Gen5 2.0 software according to manufacturer's specifications. Measurements were normalized by dividing the OD₆₀₀ readings of the isolate on test glycans by the average OD₆₀₀ of the isolate in medium supplemented with 0.5% w/v dextrose to facilitate comparison of glycan utilization by strains that grow within significantly different OD₆₀₀ ranges. Table **11** summarizes the results obtained.

**Table 11. Glycan-supported growth of bacteria associated with phytoestrogen metabolism**

| | **Phytoestrogen Metabolism-Associated Bacteria, Average Normalized Growth** | | | | |
|---|---|---|---|---|---|
| **glycan #** | **BPR.22** | **EFM.66** | **CSC.32** | **BLO.16** | **BLO.83** |
| 1 | +++ | +++ | + | ++ | + |
| 2 | +++ | ++ | + | + | - |
| 3 | +++ | ++ | - | + | + |
| 4 | +++ | + | + | - | - |
| 5 | +++ | + | + | - | - |
| 6 | +++ | + | - | - | - |
| 7 | +++ | + | - | - | - |
| 8 | +++ | + | - | - | - |
| 9 | +++ | + | - | - | - |
| 10 | +++ | + | - | - | - |
| 11 | +++ | + | - | - | - |
| 12 | +++ | - | - | - | - |
| 13 | ++ | - | - | - | - |

**Table 12: Key to Example 3 Symbols**

| **Symbol** | **NGV** |
|---|---|
| - | <0.15 |
| + | 0.15-0.3 |
| ++ | 0.3-0.6 |
| +++ | >0.6 |

Bifidobacterium species and *E. faecium* have been reported to metabolize the phytoestrogen daidzin, an isoflavone, to equol. *B. producta, C. scindens* and *Enterococcus faecalis* have been reported to metabolize plant lignans, a different class of phytoestrogens, to enterodiol and enterolactone (Spanogiannopoulos P. et al. The microbial pharmacists within us: a metagenomics view of xenobiotic metabolism. Nature Reviews Microbiology vol 14, May 2016). *B. longum, B. producta, C. scindens* and *E. faecalis* thus are associated with metabolism of phytoestrogens.

In the assay, different glycans supported different levels of growth of different numbers of these bacterial strains associated with phytoestrogen metabolism. In the assay, glu80man20, glu60man40 and glu100 supported growth of 4-5 of 5 strains, producing average normalized growth values of at least 0.15. Glu100 and gal100 supported growth of 3 strains, with average normalized growth values of at least 0.15 in the assay. Glu33gal33fuc33, man60glu40, man80gal20, man66gal33, man100 and man52glu29gal19 supported growth of 2 strains in the assay, and xyl75ara25 and xyl100 supported growth of 1 strain in the assay.

Metabolism of phytoestrogens such as plant lignans and isoflavones by bacteria in the gut to molecules that bind estrogen receptors may have protective effects against breast cancer (Mabrook, H. B et al. Lignan transformation by gut bacteria lowers tumor burden in a gnotobiotic rat model of breast cancer. Carcinogenesis 33, 203-208 (2012)). Administration of glycans that promote the growth of bacterial species associated with phytoestrogen metabolism thus may elicit protective effects against breast cancer and/or other types of cancer in humans.

**Table 13: Key to Example 3 Glycans**

| **Key to glycans** | |
|---|---|
| **glycan #** | **composition** |
| 1 | glu80man20 |
| 2 | glu60man40 |
| 3 | glu 100 |
| 4 | gal100 |
| 5 | man80glu20 |
| 6 | glu33gal33fuc33 |
| 7 | man60glu40 |
| 8 | man80gal20 |
| 9 | man66gal33 |
| 10 | man 100 |
| 11 | man52glu29gal1 9 |
| 12 | xyl75ara25 |
| 13 | xyl100 |

### Example 4: Modification of exogenous substances by microbiota

Untargeted metabolomics was performed on 30-50 mg of cecal contents from mice fed either a High Fat diet (Research Diets D12492) or High Fat diet + glycan using Metabolon's LC-MS based DiscoveryHD4 platform.

Three exogenous substances, enterolactone, stachydrine, and quinate, were identified that were modified by the microbiota with the addition of man52glu29gal19 at 1% in the drinking water. See Figures 1A, 1B, and 1C.

Enterolactone, a mammalian lignin formed by the action of intestinal bacteria from plant lignin precursors present in the diet, was increased in animals treated with man52glu29gal19. It is believed that enterolactone may possess beneficial health effects in humans. Reduction in enterolactone has been associated with a number of human pathologies. For example, it has been demonstrated that individuals with Type 2 Diabetes have significant reduction of urinary enterolactone when compared to healthy controls (Sun et al., Diabetes Care, 2014). Enterolactone has also been shown to have some anticarcinogenic action as the administration of enterolactone has been shown to inhibit or delay the growth of experimental mammary cancer (Saarinen et al., Molecular Nutrition & Food Research, 2013). In epidemiological studies, lower concentrations of enterolactone have been observed in breast cancer patients compared to healthy controls, which may suggest that enterolactone is anti-carcinogenic. Enterolactone and lignans may also be protective of cardiovascular disease.

In addition, stachydrine was reduced with the addition of man52glu29gal19 at 1% in the drinking water. Stachydrine is also called proline betaine. Proline betaine is a glycine betaine analogue found in many citrus foods. Stachydrine has been shown to be a marker of citrus intake in the diet (Guertin et al., AJCN, 2014). Elevated levels of proline betaine in human urine are found after the consumption of citrus fruits and juices.

Further, quinate was found to increase with the addition of either glu100 or man52glu29gal19 to a high fat diet. Quinate is an abundant plant product that is utilized as a carbon source for a number of microorganisms (Teramoto, et al., Appl. Environ. Microbiol., 2009). These data suggest that microbial turnover of various exogenous substances by microbial taxa (such as those residing in the gastrointestinal tract, e.g., of a human subject) can be modulated by administering a glycan composition (such as described herein) to a subject in an amount effective to modulate (e.g., increase or decrease) the level of the exogenous substance in the subject.

### Comparative Example 5: Modulation of beta-glucuronidase levels in the gastrointestinal tract of human subjects and mouse model by glycans for the reduction/prevention of irinotecan toxicity

Patients with histologically or cytologically confirmed colorectal adenocarcinoma are randomized to receive a glycan treatment or placebo for one week prior to administration of a chemotherapeutic regimen consisting of FOLFIRI [folinic acid (leucovorin) 400 mg/m^2 by vein (IV) Day 1; 5-FU 400 mg/m^2 IV injection Day 1 immediately followed by 2.4 g/m^2 IV over 46 hours over Days 1-3; Irinotecan 180 mg/m^2 IV on Day 1]. Primary treatment outcome will be the proportion of patients on glycan or placebo that experience Grade 2 or greater diarrhea in the week following the FOLFIRI regimen. A first secondary outcome will be the proportion of patients on glycan or placebo that experience Grade 3 or greater diarrhea in the week following the FOLFIRI regimen. A second secondary outcome will be the proportion of patients on glycan or placebo that require an antidiarrheal treatment (such as Loperamide) in the week following the FOLFIRI regimen. The glycan treatment is expected to increase in the gastrointestinal tract of patients the growth of bacteria that do not express β-Glucuronidase relative to bacteria that do express β-Glucuronidase, and thus will proportionally decrease the concentration of β-Glucuronidase enzymes in the gastrointestinal tract that can activate the toxic metabolite of irinotecan (SN38). For example, glycan 1 is expected to increase the growth of gut commensal bacterial strains, BTH. 8, BVU. 10, and DFO. 36, while not increasing the growth of LGA. 44, ROB. 74, BLO. 16, and BLO. 83 (see Example 2 and Table 8 ).

A proof of concept experiment was conducted to elucidate the effects of glycans on chemotherapy-induced toxicity in a mouse model using irinotecan chemotherapeutic.

In this study, 150 male BALB/c mice (Charles River Laboratories) were randomized into 13 mice per group, for 7 groups, 3 groups of 18 mice, and 1 group of 5 mice and were group housed. The mice were allowed to acclimate for five days following arrival. One group of 5 mice received irinotecan. All mice received special diet, with monitoring of daily body weights starting at day -7.

Mice received oral gavage for 14 days with deionized water or glycan composition. Starting on Day -6, animals in glycan-treated groups began treatment with glycan compositions or FOS at 6 g/kg/day by oral gavage; treatment with glycans continued through Day 7. During this same period, animals in the control group received water. All groups (apart from the control group not receiving irinotecan) received a single dose of irinotecan on day 0 at a dose of 250 mg/kg intraperitoneal injection. At the time of irinotecan dosing to prevent transient diarrhea, atropine was given by subcutaneous injection at 0.03mg/kg.

Daily, mice were weighed and monitored for survival. On day -1, 5 mice from 4 groups were euthanized with cecal and colonic contents collected for 16S sequencing and short-chain fatty acid assessment. Feces collected on days -6, -1, 1, 3, 5 and 7 and stored at - 80°C. On day 7 remaining animals were euthanized for blood and tissue collection. Figures 18A and 18B show that mice fed FOS, Glu50Gal50, or Gal100 lost less weight over the time period of the experiment than mice that were not fed a glycan composition. The reduction in weight loss is a sign that the animals were healthier and encountered less toxicity from irinotecan metabolism. These data suggest that the glycans may shift the microbial community in the animals in a way that limits the production of toxic irinotecan products/intermediates by the microbial processing of the exogenous substance (as described above) in the animal, thereby limiting the overall toxicity of the drug.

### Comparative Example 6: Modulation of phytoestrogen metabolism-associated bacteria by glycans for the treatment of breast cancer

Metabolism of phytoestrogens such as plant lignans and isoflavones by bacteria in the gut to molecules that bind estrogen receptors may have protective effects against breast cancer (Mabrook, H. B et al. Lignin transformation by gut bacteria lowers tumor burden in a gnotobiotic rat model of breast cancer. Carcinogenesis 33, 203-208 (2012)). Administration of glycans increases the relative growth of phytoestrogen metabolism-associated bacteria, such as BPR.22 and EFM.66 (see Example 3 and Table 11), and thus will increase the concentration of phytoestrogens and elicit protective effects against breast cancer and/or other types of cancer in humans. Women who are at high risk for breast cancer are randomized to receive glycan treatment or placebo for 12 months. Inclusion criteria are any of: i) Five year Gail risk > 1.7%, ii) Known BRCA1/BRCA2 mutation carrier, iii) Family history consistent with hereditary breast cancer, iv) Prior biopsy exhibiting atypical hyperplasia or lobular carcinoma in situ (LCIS), or v) History of invasive breast cancer or ductal carcinoma in situ (DCIS) and have completed standard therapy including tamoxifen/aromatase inhibitor or will not be treated with tamoxifen/aromatase inhibitor. After 1 year of glycan or placebo treatment, patients are assessed for: i) reduced magnetic resonance imaging (MRI) volume (equivalent to 3-dimensional mammographic density) in high-risk women or those with invasive breast cancer or DCIS who are supplemented daily with glycan compared to placebo (microcrystalline cellulose), ii) decreased cell proliferation and apoptosis, as measured by Ki67 and caspase 3 staining, respectively, of breast epithelial cells with glycan treatment compared to placebo, iii) decreased intermediate molecular marker expression of estrogen receptor alpha (ER alpha) and ER beta in women treated with glycans compared to the placebo.

### Comparative Example 7: Modulation of phytoestrogen metabolism-associated bacteria by glycans for the treatment of type 2 diabetes

The microbiome affects the development and progression of type 2 diabetes. Patients with type 2 diabetes have been found to have altered levels of microbial metabolites circulation as measured by urine. For example, the concentration of the microbially-produced lignan enterolactone is inversely correlated with the risk of type 2 diabetes (Sun et al. Diabetes Care 37.5(2014):1287-95). Increasing the concentration of molecules such as enterolactone may improve the outcome of patients with type 2 diabetes. Glycans can increase the production of enterolactone from the diet of mice as shown in Figure 6.

Patients with type 2 diabetes can be treated with glycans to increase the concentration of enterolactone in circulation compared to a placebo control treatment. Patients are included based on at least one of the following criteria: *1*) an elevated glucose concentration (fasting plasma glucose ≥7.0 mmol/L, random plasma glucose ≥11.1 mmol/L, or plasma glucose ≥11.1 mmol/L after an oral glucose load) and at least one symptom related to diabetes; 2) no symptoms, but elevated glucose concentrations on two separate occasions; or 3) treatment with insulin or oral hypoglycemic medication. Patients are treated with a glycan or a placebo control for 6 months. At the end of 6 months, glycan treatment is expected to increase the concentration of enterolactone in the patient's urine as assessed via electrospray ionization orbitrap liquid chromatography-mass spectrometry compared to the placebo. Furthermore, at the end of 6 months, glycan treatment is expected to decrease fasting plasma glucose or glucose after an oral load compared to patients treated with placebo.

### Example 8: Protocol for Examples 9-21

Examples 9-21 were carried out according to the following protocol:
An *ex vivo* assay was designed to determine if glycans can be used to modulate a complex community of microorganisms and if the glycans produced consistent effects across communities obtained from multiple (twelve) subjects. Fecal samples and slurries were handled in an anaerobic chamber (AS-580, Anaerobe Systems) featuring a palladium catalyst. Glycans ara100, gal100, glu60man40, glu100, glu50gal50, gal33man33ara33, man75gal25, glu33gal33man34, glu33gal33Ara33, man100, man52glu29gal19 and commercially available glycans lactulose (Alfa Aesar) and FOS (Nutraflora FOS; NOW Foods, Bloomingdale IL) were included in the study. The fecal sample donations obtained from 12 subjects were stored at - 80°C. To prepare working stocks the fecal samples were transferred into the anaerobic chamber and allowed to thaw. The fecal samples were prepared to 20% w/v in phosphate buffered saline (PBS) pH 7.4 (P0261, Teknova Inc., Hollister, CA), 15% glycerol and stored at -80°C. The 20% w/v fecal slurry + 15% glycerol was centrifuged at 2,000 x g, supernatant was removed, and the pellet was suspended in PBS pH 7.4 to 1% w/v fecal slurry. Prepared 1% w/v fecal slurry were exposed to the studied glycans at a final concentration of 0.5% w/v in 96-well deep well microplates, 500 µL final volume per well, at 37° C for 18 hours, anaerobically. Genomic DNA was extracted from the fecal samples and variable region 4 of the 16S rRNA gene was amplified and sequenced (Earth Microbiome Project protocol www.earthmicrobiome.org/emp-standard-protocols/16s/ and Caporaso JG et al. 2012. Ultra-high-throughput microbial community analysis on the Illumina HiSeq and MiSeq platforms. ISME J.). Operational Taxonomic Units (OTUs) were generated by aligning 16S rRNA sequences at 97% identity. Microbial communities were compared to each other using UniFrac distance metric (Lozupone C. et al., Appl. Environ. Microbiol. December 2005 vol. 71 no. 12 8228-8235).

### Comparative Example 9: Modulation of cardiac glycoside (e.g., digoxin) metabolism-associated bacteria by glycans

The gut actinobacterium, *Eggerthella lenta,* has been previously shown to inactivate cardiac drug digoxin (see, e.g., Haiser et al. 2014. Gut Microbes: 5(2):233-238). The association with digoxin inactivation is limited to strains of *Eggerthella lenta* possessing the cardiac glycoyl reductase(cgr) operon, but suppressing the levels of *Eggerthella lenta* in patients under digoxin treatment might prevent or decrease inactivation of the digoxin drug.

*Ex vivo* assays showed that several glycans significantly reduced the levels of *Eggerthella lenta* (Figure 9). All the samples with glycan preparations showed statistically significant (wilcoxon rank sum test - p-value < 0.05) reduction in the relative composition of *Eggerthella lenta* when compared to the controls at 45 hours post inoculation. Four glycans also showed higher reduction than the naturally occurring fructose oligosaccharide (FOS).

### Example 10: Modulation of Sulfonamide (e.g., sulfasalazine) metabolism-associated bacteria by glycans

Microbial azoreductase, found in Bacteroides sp., Enteroccocus faecalis, and Lactobacillus sp., is known to transform sulfasalazine, which is used to treat rheumatoid arthritis, into its active form. Increasing the levels or activity of microbial azoreductase expressing microbes may increase the effectiveness of sulfasalazine treatment.

*Ex vivo* assays showed that several glycans significantly increase the levels of Bacteroides sp. and Enteroccaceae/Enterococcus (Figs. 2 and 3).

In this assay, man100, glu100, man75gal25, man52glu29gal19, glu50gal50, ara100, FOS, gal100, and glu60man40 appeared to increase the levels of Bacteroides sp. and/or Enteroccaceae/Enterococcus. In addition, man100 increased the levels of Lactobacillus sp.

### Comparative Example 11: Modulation of SN-38 glucuronide metabolism-associated bacteria by glycans

Microbial beta-glucuronidase, found in Proteobacteria, Firmicutes, and Actinobacteria, is known to modify SN-38 glucuronide, which is used to treat cancer (e.g., colorectal cancer) and has the side-effect of diarrhea, by removing a sugar moiety, creating a compound that is toxic to intestinal epithelial cells. Decreasing the levels or activity of microbial beta-glucuronidase expressing microbes may increase the effectiveness of SN-38 glucuronidine treatment and/or decrease/prevent side effects.

*Ex vivo* assays showed that several glycans significantly decrease the levels of Firmicutes, Proteobacteria, and Actinobacteria (Figs. 4-6).

In this assay, man100, gal100, man52glu29gal19, man75gal25, glu33gal33man34, glu100, glu50gal50, lactulose, glu33gal33ara33, FOS, glu60man40, and ara100 appeared to decrease the levels of Firmicutes, Proteobacteria, and/or Actinobacteria.

### Example 12: Modulation of NSAID (e.g., tyrosine and phenylalanine) metabolism-associated bacteria by glycans

A microbial enzyme, found in Firmicutes (e.g., Clostridium difficile), Bacteroidetes, Actinobacteria, and/or Fusobacteria, is known to modify tyrosine and/or phenylalanine into p-cresol, which competes with acetaminophen for SULT1A1. Decreasing the levels or activity of microbes expressing said microbial enzyme may increase the effectiveness of acetaminophen and/or reduce drug-induced toxicity.

*Ex vivo* assays showed that several glycans significantly decrease the levels of Firmicutes and Actinobacteria (Figs. 4 and 6).

In this assay, man100, gal100, man52glu29gal19, man75gal25, glu33gal33man34, glu33gal33ara33, and gal33man33ara33 appeared to decrease the levels of Firmicutes and/or Actinobacteria.

### Comparative Example 13: Modulation of polyphenol (e.g., ellagitannin) metabolism-associated bacteria by glycans

A microbial enzyme found in Actinobacteria and Coriobacteriaceae/Gordonibacter is known to hydrolyze ellagitannin to ellagic acid.

*Ex vivo* assays showed that several glycans significantly decrease the levels of Actinobacteria and Coriobacteriaceae/Gordonibacter (Figs. 6 and 8). In this assay, man100, gal100, man75gal25, man52glu29gal19, glu33gal33man34, glu33gal33ara33, glu50gal50, glu100, lactulose, glu60man40, FOS, and ara100 appeared to decrease the levels of Actinobacteria and/or Coriobacteriaceae/Gordonibacter.

### Comparative Example 14: Modulation of phytoestrogen metabolism-associated bacteria by glycans

A microbial enzyme, found in Actinobacteria, Bacteroidetes, and Firmicutes is known to metabolize phytoestrogen to molecules that bind estrogen receptors and may have protective effects against breast cancer. Increasing the levels or activity of microbes expressing said microbial enzyme may have a preventative effect on cancer, e.g., breast cancer, or treat cancer, e.g., breast cancer.

*Ex vivo* assays showed that several glycans significantly increase the levels of Bacteroidetes (Fig. 9).

In this assay, gal100, man75gal25, man100, glu33gal33man34, man52glu29gal19, glu100, glu50gal50, glu33gal33ara33, glu60man40, and gal33man33ara33 appeared to increase the levels of Bacteroidetes.

### Comparative Example 15: Modulation of polyphenol/isoflavone (e.g., daidzein) metabolism-associated bacteria by glycans

A microbial enzyme, found in Enterococcus faecium, Lactobacillus mucosae, Bifidobacterium sp., and Eggerthella sp., is known to metabolize daidzin into equol, which binds estrogen receptor-beta and may be associated with lower risk/incidence of breast cancer. Increasing the levels or activity of microbes expressing said microbial enzyme may increase the have a preventative effect on cancer, e.g., breast cancer, or treat cancer, e.g., breast cancer.

*Ex vivo* assays showed that several glycans significantly increase the levels of Enterococcaceae/Enterococcus and Bifidobacteriaceae/Bifidobacterium (Figs. 3 and 10).

In this assay, glu60man40, gal33man33ara33, man75gal25, glu50gal50, man100, FOS, and lactulose appeared to increase the levels Enterococcaceae/Enterococcus and/or Bifidobacteriaceae/Bifidobacterium.

### Comparative Example 16: Modulation of phytoestrogen/polyphenol (e.g., lignan metabolism-associated bacteria by glycans

A microbial enzyme, found in E. faecalis, E. lenta, Blautia product, Eubacterium limosum, Clostridium scindens, Lactonifactor longoviformis, Clostridium saccharogumia, and P. product, is known to metabolize lignan, e.g., pinoresinol and secoisolariciresinol, to enterodiol and enterolactone, which may be protective against breast cancer. Increasing the levels or activity of microbes expressing said microbial enzyme may increase the have a preventative effect on cancer, e.g., breast cancer, or treat cancer, e.g., breast cancer.

*Ex vivo* assays showed that several glycans significantly increase the levels of Lachnospiraceae/Blautia, Eryspelotrichaceae/Clostridium_XVIII, and/or Lactonifactor/longoviformis (Figs. 11-13).

In this assay, glu100, glu60man40, ara100, gal33man33ara33, glu33gal33ara33, glu50gal50, man75gal25, man52glu29gal19, and glu33gal33man34 appear to increase the levels of Lachnospiraceae/Blautia, Eryspelotrichaceae/Clostridium_XVIII, and/or Lactonifactor/longoviformis.

### Comparative Example 17: Modulation of Heterocyclic amine (HCA)/polycyclic aromatic hydrocarbon (PAH) metabolism-associated bacteria by glycans

Microbial beta-glucuronidase, found in bacteria that carry the uidA gene, e.g., Escherichia coli, is known to reactivate glucuronidated substrate, e.g., heterocyclic amines, (detoxified by hepatic glucuronidation), by removing the conjugate, generating a toxic compound. Decreasing the levels or activity of microbial beta-glucuronidase expressing microbes may decrease the generation of toxic, e.g., carcinogenic, compounds, which may have a preventative effect on cancer.

*Ex vivo* assays showed that several glycans significantly decrease the levels of Enterobacteriaceae/Escherichia/Shigella microbes (Fig. 14).

In this assay, ara100, gal33man33ara33, glu33gal33ara33, glu60man40, man75gal25, man100, FOS, glu33gal33man34, man52glu29gal19, gal100, lactulose, glu100, and glu50gal50 appeared to decrease the levels of Enterobacteriaceae/Escherichia/Shigella microbes.

### Comparative Example 18: Modulation of non-caloric artificial sweetener metabolism-associated bacteria by glycans

A microbial enzyme, found in Enterococcus, Clostridium, Corynebacterium, Campylobacter, and Escherichia, is known to convert sweetener to a compound that can be toxic, e.g., converts cyclamate to cyclohexylamine, which can be toxic. Decreasing the levels or activity of microbes expressing said microbial enzyme may decrease the generation of toxic compounds.

*Ex vivo* assays showed that several glycans significantly decrease the levels of Enterococcaceae/Enterococcus and Enterobacteriaceae/Escherichia/Shigella microbes (Figs. 3 and 14).

In this assay, ara100, gal33man33ara33, glu33gal33ara33, glu60man40, man75gal25, man100, FOS, glu33gal33man34, man52glu29gal19, gal100, lactulose, glu100, and glu50gal50 appeared to decrease the levels of Enterococcaceae/Enterococcus and/or Enterobacteriaceae/Escherichia/Shigella microbes.

### Comparative Example 19: Modulation of nucleoside analog (e.g., sorivudine) metabolism-associated bacteria by glycans

Microbial phosphorylase, e.g., thymidine phosphorylase or uridine phosphorylase, found in Enterobacteria, e.g., K. pneumoniae, is known to deglycosylate sorivudine, inactivating it. Sorivudine is used to treat viral infection (e.g. herpes simplex virus 1 and varicella-zoster virus). Decreasing the levels or activity of microbes expressing said microbial phosphorylase may decrease the inactivation of sorivudine and increase the effectiveness of sorivudine treatment against viral infections.

*Ex vivo* assays showed that several glycans significantly decrease the levels of Enterobacteriales/enterobacteriaceae microbes (Fig. 15).

In this assay, ara100, gal33man33ara33, glu60man40, FOS, man75gal25, glu33gal33man34, glu33gal33ara33, man52glu29gal19, man100, gal100, glu100, lactulose, and glu50gal50 appeared to decrease the levels of Enterobacteriales/Enterobacteriaceae microbes.

### Comparative Example 20: Modulation of immunotherapeutic antigen (e.g., CTLA4 inhibitor) metabolism-associated bacteria by glycans

A microbial enzyme found in Bacteroides, e.g., Bacteroides thetaiotaomicron and/or Bacteroides fragilis, is known to modify immunotherapeutic antigens. Increasing the levels or activity of microbes expressing said microbial enzyme may increase the effectiveness of CTLA4 inhibitor, e.g., to treat cancer.

*Ex vivo* assays showed that several glycans significantly increase the levels of Bacteroides/dorei/fragilis and BacteroidaceaeBacteroides microbes (Figs. 16 and 2).

In this assay, man72gal25, glu33gal33man34, glu50gal50, man100, glu100, man52glu29gal19, ara100, FOS, gal100, and glu60man40 appeared to increase the levels of Bacteroides/dorei/fragilis and BacteroidaceaeBacteroides microbes.

### Comparative Example 21: Modulation of drug additive (e.g., emulsifying agent) metabolism-associated bacteria by glycans

A microbial enzyme found in Bacteroidetes (e.g., Bacteroidales) and mucolytic bacteria such as Ruminococcus gnavus, is known to cause metabolic and inflammatory response to emulsifying agents. Decreasing the levels or activity of microbes expressing said microbial enzyme may increase the effectiveness of emulsifying agents, e.g, carboxymethylcellulose or polysorbate-80, e.g., to decrease risk/incidence of metabolic syndrome and/or inflammation.

*Ex vivo* assays showed that several glycans significantly decrease the levels of Ruminococcaceae/Ruminococcus microbes (Fig. 17).

In this assay, ara100, lactulose, gal33man33ara33, glu60man40, man100, glu33gal33ara33, glu33gal33man34, man75gal25, glu50gal50, man52glu29gal19, gal100, and glu100 appeared to decrease the levels of Ruminococcaceae/Ruminococcus microbes.

The scope of the present embodiments described herein is not intended to be limited to the above Description, Figures, or Examples but rather is as set forth in the appended claims.

## Claims

1. A glycan composition for use in increasing drug activity in a human subject comprising:
a) administering a glycan composition in an amount effective and for a time sufficient to increase the drug activity in the subject;
b) administering a glycan composition in an amount effective and for a time sufficient to increase drug activity in the subject, and wherein at the time of administration of the glycan composition, the subject comprises a level of the drug that, in the presence of the administered glycan composition, provides a therapeutic effect;
c) administering the drug, wherein at the time of administration of the drug, the subject has already been administered the glycan composition in an amount effective and for a time sufficient to increase the drug activity in the subj ect;
d) administering the drug in an amount effective and for a time sufficient to increase the drug activity in the subject, wherein subject has been determined to be in need of the glycan composition; or
e) administering the drug and the glycan composition to the subject, in amounts effective and for times sufficient to increase the drug activity in the subject, wherein administration of the drug and the glycan composition overlap;
wherein the glycan composition comprises any combination of two, three, four, five, six, seven, eight, or nine of
i) the glycan composition comprises glycan polymers that comprise glucose, galactose, arabinose, mannose, fructose, xylose, fucose, or rhamnose glycan units;
ii) the average degree of branching (DB) of the glycan polymers in the glycan preparation is between 0.01 and 0.6;
iii) at least 50% of the glycan polymers in the glycan composition have a degree of polymerization (DP) of at least 3 and less than 30 glycan units;
iv) the average DP or mean DP of the glycan composition is between 5-15;
v) the ratio of alpha- to beta-glycosidic bonds present in the glycan polymers of the glycan preparation between 0.8:1 to 5: 1;
vi) the glycan composition comprises between 15 mol % and 75 mol % 1,6 glycosidic bonds;
vii) the glycan composition comprises between 1 mol % and 40 mol % of each at least one of 1,2; 1,3; and 1,4 glycosidic bonds;
viii) the glycan composition has a final solubility limit in water of at least 50 Brix at 23 °C; or
ix) the glycan composition has a dietary fiber content of at least 50%;
wherein the drug comprises 4-aminosalicylic acid, 5-aminosalicylic acid, balsalazide, olsalazine, or sulfasalazine.

2. The glycan composition for use according to claim 1, wherein the drug activity is increased relative to a reference level, wherein the reference level is a preselected level, the baseline level at the time of administration of the glycan composition, or the level that would be seen in the absence of administration of the glycan composition.

3. The glycan composition for use according to any of claims 1-2, wherein the drug is administered at a higher dose compared to a reference dose, wherein the reference dose is the dose of drug administered to a subject not administered the glycan composition or a dose that was approved by a regulatory agency.

4. The glycan composition for use according to any of any of claims 1-3, wherein the use comprises modifying the prevalence of a microbial taxa in the gastrointestinal tract of a subject as compared to a reference level, wherein the reference level is a baseline level, a level prior to administration, or a level in the absence of administration of the glycan composition.

5. The glycan composition for use according to any of claims 1-4, wherein the use comprises modulating enzyme activity in the gastrointestinal tract of the human subj ect.

6. The glycan composition for use according to any of claims 1-5, wherein the glycan composition comprises glycan polymers that comprise at least two distinct glycan units of glucose, galactose, arabinose, mannose, fructose, xylose, fucose, and rhamnose.

7. The glycan composition for use according to any of claims 1-6, wherein the glycan composition comprises glycan polymers that comprise at least three distinct glycan units of glucose, galactose, arabinose, mannose, fructose, xylose, fucose, and rhamnose.

8. The glycan composition for use according to claims 1-7, wherein at least three of the glycosidic bonds independently comprise a 1->2 glycosidic bond, a 1->3 glycosidic bond, a 1->4 glycosidic bond, or a 1->6 glycosidic bond.

## Patentansprüche

1. Eine Glykan-Zusammensetzung zur Verwendung bei der Erhöhung der Arzneimittelaktivität in einem menschlichen Subjekt, umfassend:
a) Verabreichung einer Glykan-Zusammensetzung in einer wirksamen Menge und über einen ausreichenden Zeitraum, um die Arzneimittelaktivität in dem Subjekt zu erhöhen;
b) Verabreichung einer Glykan-Zusammensetzung in einer wirksamen Menge und über einen ausreichenden Zeitraum, um die Arzneimittelaktivität in dem Subjekt zu erhöhen, und wobei das Subjekt zum Zeitpunkt der Verabreichung der Glykan-Zusammensetzung ein Level des Arzneimittels aufweist, das in Gegenwart der verabreichten Glykan-Zusammensetzung eine therapeutische Wirkung ergibt;
c) Verabreichung des Arzneimittels, wobei zum Zeitpunkt der Verabreichung des Arzneimittels dem Subjekt bereits die Glykan-Zusammensetzung in einer wirksamen Menge und über einen ausreichenden Zeitraum verabreicht worden ist, um die Arzneimittelaktivität in dem Subjekt zu erhöhen;
d) Verabreichung des Arzneimittels in einer wirksamen Menge und über einen Zeitraum, der ausreicht, um die Arzneimittelaktivität in dem Subjekt zu erhöhen, wobei festgestellt wurde, dass das Subjekt die Glykan-Zusammensetzung benötigt; oder
e) Verabreichung des Arzneimittels und der Glykan-Zusammensetzung an das Subjekt in Mengen, die wirksam sind, und für Zeiten, die ausreichen, um die Arzneimittelaktivität in dem Subjekt zu erhöhen, wobei sich die Verabreichung des Arzneimittels und der Glykan-Zusammensetzung überlappen;
wobei die Glykan-Zusammensetzung eine beliebige Kombination von zwei, drei, vier, fünf, sechs, sieben, acht oder neun von umfasst:
i) die Glykan-Zusammensetzung umfasst Glykan-Polymere, die Glucose-, Galactose-, Arabinose-, Mannose-, Fructose-, Xylose-, Fucose- oder Rhamnose-Glykan-Einheiten umfassen;
ii) der durchschnittliche Verzweigungsgrad (DB) der Glykanpolymere in der Glykanzubereitung liegt zwischen 0,01 und 0,6;
iii) mindestens 50 % der Glykanpolymere in der Glykanzusammensetzung haben einen Polymerisationsgrad (DP) von mindestens 3 und weniger als 30 Glykaneinheiten;
iv) der durchschnittliche DP oder mittlere DP der Glykanzusammensetzung liegt zwischen 5-15;
v) das Verhältnis der in den Glykanpolymeren der Glykanzubereitung vorhandenen alpha- zu beta-glykosidischen Bindungen zwischen 0,8:1 und 5:1;
vi) die Glykan-Zusammensetzung umfasst zwischen 15 Mol-% und 75 Mol-% 1,6-glykosidische Bindungen;
vii) die Glykan-Zusammensetzung umfasst zwischen 1 Mol-% und 40 Mol-% von jeweils mindestens einer der glykosidischen Bindungen 1,2; 1,3 und 1,4;
viii) die Glykan-Zusammensetzung hat eine finale Löslichkeitsgrenze in Wasser von mindestens 50 Brix bei 23 °C; oder
ix) die Glykan-Zusammensetzung hat einen Ballaststoffgehalt von mindestens 50 %;
wobei das Arzneimittel 4-Aminosalicylsäure, 5-Aminosalicylsäure, Balsalazid, Olsalazin oder Sulfasalazin umfasst.

2. Glykan-Zusammensetzung zur Verwendung gemäß Anspruch 1, wobei die Arzneimittelaktivität relativ zu einem Referenzwert erhöht ist, wobei der Referenzwert ein vorgewählter Wert, der Basiswert zum Zeitpunkt der Verabreichung der Glykan-Zusammensetzung oder der Wert ist, der bei Abwesenheit der Verabreichung der Glykan-Zusammensetzung zu beobachten wäre.

3. Glykan-Zusammensetzung zur Verwendung nach einem der Ansprüche 1-2, wobei das Arzneimittel in einer höheren Dosis im Vergleich zu einer Referenzdosis verabreicht wird, wobei die Referenzdosis die Dosis des Arzneimittels ist, die einem Subjekt verabreicht wird, dem die Glykan-Zusammensetzung nicht verabreicht wurde, oder eine Dosis, die von einer Aufsichtsbehörde genehmigt wurde.

4. Glykan-Zusammensetzung zur Verwendung nach einem der Ansprüche 1-3, wobei die Verwendung die Modifizierung der Prävalenz einer mikrobiellen Taxa im Gastrointestinaltrakt eines Subjekts im Vergleich zu einem Referenzwert umfasst, wobei der Referenzwert ein Ausgangswert, ein Wert vor der Verabreichung oder ein Wert bei Abwesenheit der Verabreichung der Glykan-Zusammensetzung ist.

5. Glykan-Zusammensetzung zur Verwendung nach einem der Ansprüche 1-4, wobei die Verwendung die Modulation der Enzymaktivität im Gastrointestinaltrakt des menschlichen Subjekts umfasst.

6. Glykan-Zusammensetzung zur Verwendung nach einem der Ansprüche 1-5, wobei die Glykan-Zusammensetzung Glykan-Polymere umfasst, die mindestens zwei verschiedene Glykan-Einheiten von Glucose, Galactose, Arabinose, Mannose, Fructose, Xylose, Fucose und Rhamnose umfassen.

7. Glykanzusammensetzung zur Verwendung nach einem der Ansprüche 1-6, wobei die Glykanzusammensetzung Glykanpolymere umfasst, die mindestens drei verschiedene Glykaneinheiten von Glucose, Galactose, Arabinose, Mannose, Fructose, Xylose, Fucose und Rhamnose umfassen.

8. Glykanzusammensetzung zur Verwendung gemäß den Ansprüchen 1-7, wobei mindestens drei der glycosidischen Bindungen unabhängig voneinander eine 1->2 glycosidische Bindung, eine 1->3 glycosidische Bindung, eine 1->4 glycosidische Bindung oder eine 1->6 glycosidische Bindung umfassen.

## Revendications

1. Composition de glycanes destinée à être utilisée dans l'activité médicamenteuse chez un sujet humain comprenant :
a) l'administration d'une composition de glycanes en une quantité efficace et durant un temps suffisant pour augmenter l'activité médicamenteuse chez le sujet ;
b) l'administration d'une composition de glycanes en une quantité efficace et durant un temps suffisant pour augmenter l'activité médicamenteuse chez le sujet, et au moment de l'administration de la composition de glycanes, le sujet comprenant un niveau du médicament qui, en présence de la composition de glycanes administrée, fournit un effet thérapeutique ;
c) l'administration du médicament, au moment de l'administration du médicament, le sujet ayant déjà été administré avec la composition de glycanes en une quantité efficace et durant un temps suffisant pour augmenter l'activité médicamenteuse chez le sujet ;
d) l'administration du médicament en une quantité efficace et durant un temps suffisant pour augmenter l'activité médicamenteuse chez le sujet, le sujet ayant été déterminé en besoin de la composition de glycanes ; ou
e) l'administration du médicament et de la composition de glycanes au sujet, en quantités efficaces et durant des temps suffisants pour augmenter l'activité médicamenteuse chez le sujet, l'administration du médicament et de la composition de glycanes se chevauchant ;
la composition de glycanes comprenant toute combinaison de deux, trois, quatre, cinq, six, sept, huit ou neuf de :
i) la composition de glycanes comprend des polymères de glycanes qui comprennent des unités de glycane de glucose, de galactose, d'arabinose, de mannose, de fructose, de xylose, de fucose ou de rhamnose ;
ii) le degré moyen de branchement (DB) des polymères de glycanes dans la préparation de glycanes est compris entre 0,01 et 0,6 ;
iii) au moins 50 % des polymères de glycanes dans la composition de glycanes ont un degré de polymérisation (DP) d'au moins 3 et de moins de 30 unités de glycane ;
iv) le DP moyen ou DP moyen de la composition de glycanes est compris entre 5 et 15 ;
iv) le rapport de liaisons alpha- à béta-glycosidiques présentes dans les polymères de glycanes de la préparation de glycanes compris entre 0,8:1 à 5:1 ;
v) la composition de glycanes comprend entre 15 % en moles et 75 % en moles de liaisons 1,6-glycosidiques ;
vi) la composition de glycanes comprend entre 1 % en moles et 40 % en moles de chacune d'au moins une des liaisons 1,2 ; 1,3 ; et 1,4 glycosidiques ;
viii)la composition de glycanes a une limite de solubilité finale dans l'eau d'au moins 50 Brix à 23°C ; ou
ix) la composition de glycanes a une teneur en fibres diététiques d'au moins 50 % ;
le médicament comprenant l'acide 4-aminosalicylique, l'acide 5-aminosalicylique, le balsalazide, l'olsalazine ou la sulfasalazine.

2. Composition de glycanes destinée à être utilisée selon la revendication 1, dans laquelle l'activité médicamenteuse est augmentée par rapport à un niveau de référence, le niveau de référence étant un niveau présélectionné, le niveau de ligne de base au moment de l'administration de la composition de glycanes ou le niveau qui serait vu en l'absence d'administration de la composition de glycanes.

3. Composition de glycanes destinée à être utilisée selon l'une quelconque des revendications 1 à 2, dans laquelle le médicament est administré à une dose supérieure par rapport à une dose de référence, la dose de référence étant la dose de médicament administrée à un sujet non administré avec la composition de glycanes ou une dose qui a été approuvée par une agence réglementaire.

4. Composition de glycanes destinée à être utilisée selon l'une quelconque des revendications 1 à 3, dans laquelle l'utilisation comprend la modification de la prévalence d'un taxon microbien dans le système digestif d'un sujet par rapport à un niveau de référence, le niveau de référence étant un niveau de ligne de base, un niveau avant l'administration ou un niveau en l'absence d'administration de la composition de glycanes.

5. Composition de glycanes destinée à être utilisée selon l'une quelconque des revendications 1 à 4, dans laquelle l'utilisation comprend la modulation de l'activité enzymatique dans le système digestif du sujet humain.

6. Composition de glycanes destinée à être utilisée selon l'une quelconque des revendications 1 à 5, dans laquelle la composition de glycanes comprend des polymères de glycanes qui comprennent au moins deux unités de glycane distinctes de glucose, de galactose, d'arabinose, de mannose, de fructose, de xylose, de fucose et de rhamnose.

7. Composition de glycanes destinée à être utilisée selon l'une quelconque des revendications 1 à 6, dans laquelle la composition de glycanes comprend des polymères de glycanes qui comprennent au moins trois unités de glycane distinctes de glucose, de galactose, d'arabinose, de mannose, de fructose, de xylose, de fucose et de rhamnose.

8. Composition de glycanes destinée à être utilisée selon l'une quelconque des revendications 1 à 7, dans laquelle au moins trois des liaisons glycosidiques comprennent indépendamment une liaison 1 -> 2 glycosidique, une liaison 1 -> 3 glycosidique, une liaison 1 -> 4 glycosidique ou une liaison 1 -> 6 glycosidique.
